# EUROPEAN PATENT SPECIFICATION

(11) **EP 2 205 736 B1**
(45) Date of publication and mention of the grant of the patent: **02.04.2014**
(21) Application number: 08802850.1
(22) Date of filing: 09.10.2008
(51) Int. Cl.: C12N 15/10, A61K 39/21

(54) **A method for producing an HIV immunogenic composition based on HIV-specific antibodes**
Methode zur Herstellung einer gegen HIV gerichteten immunogenischen Zusammensetzung auf Basis von HIV-spezifischen Antikörper.
Méthode de préparation d'un composé immunogénique contre le VIH à base d'anticorps spécifiques pour HIV

(30) Priority: 09.10.2007 US 978536 P
(43) Date of publication of application: 14.07.2010
(73) Proprietor: Technologie Integrale Ltd., Ramsey Isle of Man IM8 1RD (GB)
(72) Inventor: FILINOVA, Elena, Yu., 103045 Moscow (RU)
(74) Representative: Lahrtz, Fritz
(86) International application number: PCT/EP2008/008544
(87) International publication number: WO 2009/046984

(56) References cited:
- WANG X. ET AL.: "Construction of human combinatorial antibody library and screening of monoclonal antibody Fabs to human immunodeficiency virus type I." CHINESE SCIENCE BUL., vol. 44, no. 4, February 1999 (1999-02), pages 352-356, XP002515243
- ZHANG M-Y ET AL: "Broadly cross-reactive HIV neutralizing human monoclonal antibody Fab selected by sequential antigen panning of a phage display library" JOURNAL OF IMMUNOLOGICAL METHODS, ELSEVIER SCIENCE PUBLISHERS B.V.,AMSTERDAM, NL, vol. 283, no. 1-2, 1 December 2003 (2003-12-01), pages 17-25, XP004476975 ISSN: 0022-1759
- ZHANG ET AL: "Selection of a novel gp41-specific HIV-1 neutralizing human antibody by competitive antigen panning" JOURNAL OF IMMUNOLOGICAL METHODS, ELSEVIER SCIENCE PUBLISHERS B.V.,AMSTERDAM, NL, vol. 317, no. 1-2, 28 November 2006 (2006-11-28), pages 21-30, XP005782716 ISSN: 0022-1759
- CHOUDHRY ET AL: "Cross-reactive HIV-1 neutralizing monoclonal antibodies selected by screening of an immune human phage library against an envelope glycoprotein (gp140) isolated from a patient (R2) with broadly HIV-1 neutralizing antibodies" VIROLOGY, ACADEMIC PRESS,ORLANDO, US, vol. 363, no. 1, 5 May 2007 (2007-05-05), pages 79-90, XP022065040 ISSN: 0042-6822

## Description

The present invention relates to a method for producing a HIV immunogenic composition. In particular, the present invention provides formation of HIV specific antibodies as an immune response in an individual, which bind to existing HIV-subtypes and mutants selected after antiretroviral therapy. The present invention also discloses HIV specific antibodies capable to recognize and bind to substantially all HIV-isoforms.

### BACKGROUND.

Human immunodeficiency virus type 1 (HIV-1) is characterized by a striking genetic variability caused by accumulation of mutations, arising during viral replication, and also by the recombination events [1, 18, 24]. Failures of chemotherapeutic methods of HIV-treatment are caused by this high mutagenic activity of HIV-1 viral strains [8]. It was shown earlier that resistant viral variants quickly have been arisen in patients after different courses of antiretroviral therapy and even after complex therapy (HAART). These resistant viruses have specific alterations in their protein's conformation and structure. Usually such mutations responsible for HIV-1 escape from current treatment are saved and accumulated as a result of selection under the treatment conditions.

The treatment with anti-HIV-1 medicines does not stop the virus replication completely, that makes possible the selection and accumulation of pre-existing resistance mutations, and arising and accumulation of the new ones, thus, bringing new gates for virus surviving. Thus, all of the existing antiretroviral preparations (NRTI, NNRTI, protease inhibitors, fusion inhibitors and even mixtures of different drugs, like HAART) can only slow down the HIV-1 replication for more or less prolonged period of time [7], until the arising and propagation of resistant viral strains. The wide spreading of HIV-1 resistant variants, tolerant to common anti-HIV treatments, became the serious problem, especially for the economically developed countries, where HIV-infected patients regularly receive antiretroviral therapies [8].

For 25-years history of HIV researches several types of approaches of HIV immunotherapeutic vaccine development were proposed and their practical outcome studied. These approaches can be classified according to vaccine active components, mechanism of their action and method to produce the vaccine as follows:
Type 1: Monoclonal HIV-specific antibodies-based HIV/AIDS vaccines,
Type 2: HIV particles disruption-based vaccines,
Type 3: HIV-peptides based vaccines and
Type 4: DNA plasmid or viral (adeno-, adeno-associated, fowlpox, vaccinia etc.) vector vaccines encoding genes of HIV peptides.

Type 1: Monoclonal HIV-specific antibodies-based HIV/AIDS therapeutic vaccines, among them neutralizing antibodies as mAb or cocktail of 2-3 HIV-neutralizing mAbs [5, 14, 28].

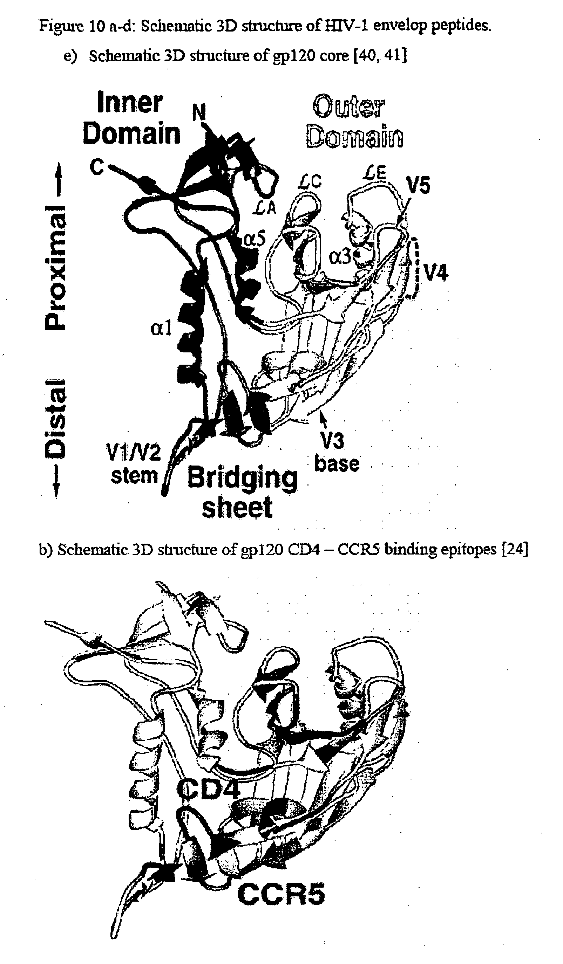

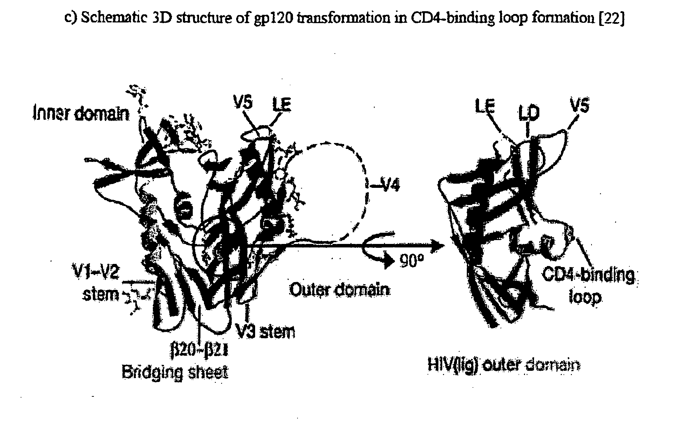

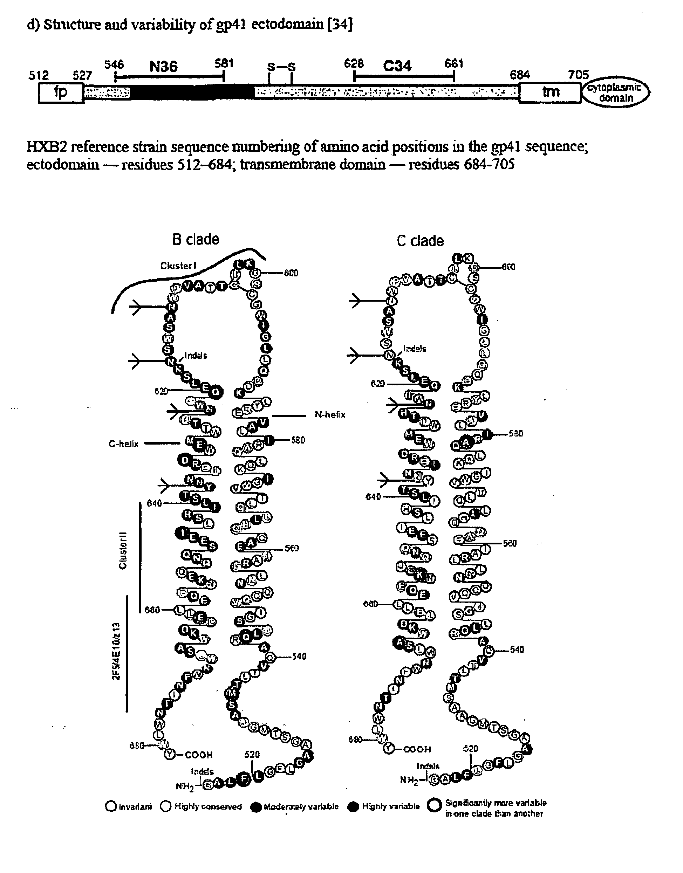

The first thing discovered about HIV infection mechanism was its way of entrance into lymphocytes or other host cells through CD4 receptor and CCR5 and CXCR4 co-receptors. Then HIV envelop proteins structure was studied (Fig. 10 a-b), variability of gp120 loops 3D and crucial role of gp120-gp41 complex formation for distinguishing and adhering CD4 and co-receptors were settled as a doctrine. Monoclonal antibodies able to find virus env proteins, to bind their epitops responsible for HIV cells entrance, or to bind respective domains or epitops on CD4 receptors and co-receptor and therefore comprehensible to block HIV infection process in stage or cell binding were called HIV neutralizing antibodies.

The major problem in antibody-based vaccine development, also caused by HIV genetic variability, is that recombinant antibodies elicited for some HIV antigen are not capable of neutralizing different isolates of HIV-1. The vast majority of anti-HIV-1 monoclonal antibodies elicited with immunization has poor or no cross-neutralizing activity and typically bind to determinants that either vary from virus to virus because of mutation or are poorly exposed on the surface of infectious virions. Several variations of neutralizing mAbs were created, but then clinical trials demonstrated that vaccines based on neutralizing antibodies against envelop proteins gp120 and gp41 stop to work within 1-2 months (in rare cases when they worked from the beginning) due to the same reason - variability and changes in the surface epitops of target HIV proteins.

The backward of described approach to vaccine development is monoclonal selection of either antibodies of viral antigens for animal's immunization. Even in case the panel of neutralizing antibodies specific for different variants of viral target-proteins is created every mAb is produced as recombinant monoclone in bacterial system. Moreover procariotic recombinant antibodies have at least ten times lower affinity to their antigens compared to native Abs in animal's or human blood serum. Polyclonal HIV-specific immunoglobulines elicited in animals are normally immunotoxic for different organisms such as humans. It is possible to use them for diagnostic purpose, but high chances for development of anaphylactic reactions are the natural limit for their immunotherapeutic application. Technology of hybridoma mAbs production does not solve the problem of biological specimen's differences in immunoglobulines. Humanized or chimeric mAbs production technology is highly laborious, relatively long and cost-consumptive. Therefore with this technology it is not possible to produce decades or hundreds of mAbs variations for anti-HIV immunotherapy.

Type 2: HIV particles disruption-based vaccines [9, 20]. The idea to use natural HIV virions and HIV peptides appeared more than 15 years ago and reincarnated in several forms. Among them it was the conservation of HIV particles infectious activity with β-propiolactone, psoralen or similar agent well-known as lethal for small viruses but with relatively low destroying effect for peptide bounds and protein's conformation. Quickly it became evident that concentration of native virus from patients bloodstream with ultracentrifugation method cannot bring the amount of virus applicable for some immunization, it can hardly deliver some material for research analysis. So the practical variations of this type of vaccine are either in vitro infection-cultivation of laboratory strains, or infection of primary isolates and their cultivation with donor lymphocytes. In both cases large-scale production in hundred liters fermenters is being described in order to provide the mass of viral particles necessary for HIV proteins immune response formation after immunization.

The idea itself was not bad completely, it even has advantages before the other three vaccine types. First, the safety to use inactivated viral particles for immunization is getting more obvious if one tried to make real time quantification of HIV RNA copies after ultracentrifugation in sucrose pillow gradient. Viral RNA is mostly being disrupted into little pieces and destroyed to the level with 10⁴-10⁵ lower numbers than real concentration of HIV virions or their proteins obtained after concentration in sucrose gradient. Second, the obtaining of native viral proteins seems more likely to have chances to cover existing variety of HIV env proteins epitopes. However this last statement is the real reason why this type of vaccine never worked.

HIV particles disruption-based vaccine development is the best example how much in vitro conditions of genetic mutations selection is different from bounds of the same process in animal or human organisms. Analysis of viral peptides revealed the high variability of antigen epitops specific not only for different viral subtypes but even for viral variants isolated from the same patient. However all laboratory strains, among them highly infective BIII, A455, have constant and more homogenous composition of env peptide's sequences. The variety of env peptide libraries analyzed with mass spectrometry or 3D structural methods for laboratory HIV strains is up to 5 percent from the equivalent taken from one single patient. The same tendency is being observed for primary HIV isolates co-cultivated in vitro with donor's blood lymphocytes or CD4, CCR5 or CXCR4-bearing human cell cultures. It means that selection conditions for in vitro infection of virus are very much different from natural virus replication and virion's formation processes in the organism, and gates for virus survivorship in human organism are 95 percent wider than during in vitro cultivation. Therefore all attempts to prepare anti-HIV vaccine using virus particles inactivation after large-scale in vitro production failed, as well as peptide-based vaccines sourced from laboratory HIV strains.

Type 3: HIV-peptides based vaccines [3, 6, 13, 15, 27, 33, 36]. This modem type of vaccines includes small HIV peptides, multiple little 15-20-aminoacid fragments of larger HIV proteins mimic epitopes of viral proteins responsible for receptors recognition and infectious activity, panels of these small peptides. As a member of small lentiviruses family HIV consists of a little number of peptides (totally 18) the majority of HIV peptide vaccines comprise fragments of gp120 (gp140, gp160) or both gp120 and gp41 env proteins, the others include little easy-to-maintain matrix peptides and p24 fragments. The other part of this class is full-length env peptides or their large fragments produced in yeasts with provided glycosylation natural for HIV lifecycle, or so called carbohydrate-based HIV vaccines. Some of HIV peptide vaccines are intended for therapeutic immunization, some are declared to possess preventive activity.

However neither cocktails of recombinant HIV peptides nor cocktails of synthesized 15-20-aminoacid peptides up to now were able to provide defense from virus infection and replication. The main reason for that can be revealed from analysis of principle how these peptides were obtained. Recombinant peptides sequences are made with technique of automated DNA sequencing of samples obtained with RT-PCR from viral material from patient including the stage of HIV genome fragments amplified with long-length Taq-polymerase PCR (usually 1000-3000 b.p.), or sequence of DNA from patient's lymphocytes after HIV-specific primers PCR, then the selection of transformed E.coli strain colonies. The existing technique is based on monoclonal selection of HIV genotypes in random regime with a frequency of one sequence case from the variety of variants 10⁵ - 10⁶ if not higher in average, from that the average infectious viral titr is 1%, so it is 10³-10⁴ copies of infectively active virus. It is well known for researchers who made HIV genome sequences and their analysis themselves that two sequences made with this technique from the same sample of one individual patient blood the data of complete HIV genome will be dramatically different. Therefore immunization with these recombinant peptides or the cocktail of 3-4 recombinant peptides even properly glycosylated (carbohydrated) in eucariotic expression system cannot provide the formation of immune response specific for inactivation of virus variants which it currently has to deal with. Therefore HIV vaccine development approach should get free of these standards, information of recombinant peptides sequence should be created with other method.

Synthetic aminoacid small HIV peptides [27] are produced with controversial approach - hundreds of variants are being made as a mixture in automated peptide synthesizer when for each cycle of peptide bound formation a mixture of possible aminoacid variants in known HIV sequences is being added. Many variants of variable regions of env proteins can be obtained using peptide synthesizer. However the size of these peptides is restricted with 15-20, maximum 30 aminoacids, longer peptide versions is possible to produce only in recombinant systems. In practice immunization with small synthetic peptides and their cocktails boosts high enough but low- or non-specific for HIV immune reaction. Respectively, even attempts of synthetic HIV peptides immunization of animals (macaque-resus) deliver unsatisfactory results of absence of HIV-specific antibodies in their blood tested with standard ELISPOT methods. Maybe as a composition for therapeutic purpose in combination with HAART the existing peptide-based HIV vaccines might have some chances. However no one of peptides vaccine composition up to now demonstrated HIV infection-preventive effect after immunization.

Type 4: DNA plasmid or viral (adeno-associated, fowlpox, vaccinia, retroviral, etc.) vector vaccines encoding genes of HIV peptides [11, 12, 16, 21, 26, 29, 30]. Among 55 anti-HIV vaccines obtained permissions for 99 clinical trials in the world most belong to DNA-based class. But only one candidate passed Phase IIb clinical trial and have some chances to pass the Phase III [37, 42]. The idea to use this type of vaccine has a healthy background that DNA immunization does not cause immediate bystander effects such as autoimmune complications and anaphylactic reactions, so its clinical application is safe and easy. In spite this advantage all viral and non-viral DNA vaccines comprise a number of backwards that give a weak hope for their real anti-HIV effectiveness possibility.

As DNA does not cause any immune reaction itself the vaccine effectiveness is a magnification of three conditions, each of them of equal importance:
1) the transfection/infection efficiency or how many cells can be supplied with genetic material from once applied certain amount of DNA;
2) the expression level or how much protein is being expressed in cells which got a copy of gene/genes;
3) the continuation of immune response or how long MHC will continue to elicit mAbs recognizing targeting pathogen.

The measurement for in vitro transfection/infection efficiency is a percentage of cells expressing a current protein counted 24 hours after gene transfer until cells could pass the next cycle of division, percentage is being counted for cells expressing a fluorescent protein or LacZ transferred simultaneously in the same conditions. For non-viral plasmid vectors in vitro efficiency can achieve 40-90% but for the same vectors intravenous administration in vivo brings 1-5% in the best case. From these 40-90% (1-5% in vivo) 98-99% is a transient or episomal expression which disappears after 2 weeks, and only 1-2% of transfected genetic material inserts into cell genome and provides long-time expression. The amount of plasmid DNA vaccine [16] is limited with maximal tolerated dose for its delivery agents - cationic lipids and liposomes made of them, cationic polymers (polyethyleneimine, polylysine), pluronic and their different combinations. Practically all cationic substances that are able to bind and carry negatively charged DNA are highly toxic in concentrations 10⁵ - 10⁴ M and more. The expression level for non-viral vectors is relatively high compared to viral vectors expession.

The infection efficiency for viral DNA vectors is variable but normally does not exceed 10-20% for in vitro experiments. But viral vectors became attractive for their ability to provide delivery of genetic material directly to genome. So in spite infection efficiency of viral vectors for in vivo administration is 2-5% in average the expression of target protein is mainly a long-term, not transient one. Therefore viral DNA vectors supposed to possess the sufficient continuation of immune response and anti-HIV activity for therapeutic or preventive purpose.

However is to study viral DNA vaccines components and how they work in step-by-step manner limitations of their prospective activity can be observed. The first class of DNA vectors that went into clinical trials was adenoviral constructions. Though their modem versions already demonstrate infection efficiency different from zero, and titres of elicited after immunization mAbs are detectable with all immunochemistry method they are never being used in mono regime. The point is the adenoviral - ADV [11] or adeno-associated viral vectors - AAV [29] cause only relatively low expression of delivering protein normally recognized with ELISA, INF-γ ELISPOT or Western-blot assays two weeks later the scheduled immunization. If to compare these data for ADV and AAV with antibodies titres two weeks after standard immunization with any recombinant protein or a mixture of proteins it becomes clear that absolute numbers are 5-10 times lower for ADV and AAV vaccinations. Looking at these numbers the researcher can make some conclusions about possible period of immune response.

The only vaccine composition reached the Phase III clinical trial and applied to 16000 non-infected individuals in Thailand since October 2003 is based on lined-up immunization with plasmid DNA-gag-pol-env vaccine (AIDSVAX B/E) following with two poxvirus (vaccinia virus)-HIV vaccinations (ALVAC-HIV) [12]. The examination of data of this patent shows that titres of elicited antibodies from blood samples of vaccinated Rhesus macaques are increasing one-three weeks after each immunization and the rest of one year period of vaccinations are modestly deviating to plus plot from control numbers [12]. The continuation of immune response is a matter of question how to evaluate it in this case. One should also remember that adenoviruses and poxviruses are among the biggest in viral families, they expose hundreds of their own proteins on the surface and in viral matrix. It means that immune response boosted in short (one-two weeks) period after the administration is high but mostly non-specific, and besides non-specifity cause immunotoxic reactions as bystander effects.

The only exclusion in viral vaccines effectiveness is retro- (lenti-) viral vectors-based approaches [26]. HIV itself is a good representative from a family of lentiviruses. Retroviral vectors provide high enough (up to 5%) infection efficiency in vivo, the expression of delivered genes proteins is sufficient and long-term if not stable due to infection of cell's genome. Retroviral vectors demonstrated significantly better antitumor responses in clinical trials as cancer therapeutic vaccines than any other genetic constructions. Only all retroviruses including HIV have one feature that makes doubtful even their therapeutic application and not considerable the preventive vaccination - it is their ability to enter human genome as the mobile genetic elements and to drive multiple genetic mutations which cascade becomes uncontrollable after some period of time and causes multiple cancer transformations in different cells and tissues.

The general backward of DNA-based HIV vaccines is the original nucleotide sequence obtained with the same method as it was described above for recombinant HIV peptides compositions, such as standard DNA sequencing after PCR and monocloning. It is close to the truth to apprehend the average number of HIV genetic variations in one patient bloodstream equal to 10⁵ - 10⁶ variants. The genetic construction made of one or several sequences data obtained this way in random regime cannot work in principle for majority of HIV variants even for the same patient. And all plasmid DNA and any viral vectors-based DNA HIV vaccines are based on sequence of HIV genome for single env, pol, gag and their combinations regions. Until these constructions will consist of monoclonal nucleotide HIV genome region's sequences it is a blind alley for HIV vaccine development. To combat HIV genetic variability and mutability it is necessary to maintain quantitative analysis of its existing variations and to formulate prospective vaccine for the more frequently existing variants.

As was described above the other main limitation for DNA-based HIV vaccines effectiveness is the poor immune response which is due to imperfect known methods for in vivo delivery of viral and non-viral gene therapy vectors. The right comparison for academic scientist to understand the low chances of DNA-based vaccines type for providing any kind of anti-infection immunization is as follows. Please imagine the hypothetic monoclonal antibodies (mAbs) for any protein or antigen and their recombinant linked L-H IgG chains version produced in procariotic E.coli system. Now we will try to make a comparison of the affinity to bind antigen for these two mAbs types with all possible laboratory immune reactions assays - ELISA, ELISPOT, immuno dot-blot, Western-blot, flow cytometry, fluorescent microscopy, etc. What we will see in every picture where these types are in one assay - the affinity of recombinant mAbs is always at least 10 times lower than the affinity of natural animal monoclonal antibodies, moreover the difference in minimal binding activity during titration can achieve 100-200 times. The same situation for in vivo assessment of vaccine immunogeniety is being observed if the researcher analyzes the activities of DNA-based and protein-based compositions used for animal immunization. The effectiveness of specific immune response measured as a titre of mAbs for current antigen in blood of immunized animals will be many times lower for the antigen delivered as genetic vector than for original protein-antigen. The strength of specific antigen immune response for DNA variant is always 5-20 times lower than for its "positive control" - protein variant.

There is one more a small category of compositions being described as a potential HIV vaccine candidate - it is so called dendritic vaccines. Their development was based on stem cells science, and dendritic vaccines are applied for the treatment of several types of tumors in combination with chemotherapy or irradiation with modest enough therapeutic results in spite relatively high cost (45-60 thousand US dollars for one patient treatment in average). However as dendritic cells-macrophage predecessors in situ taught to distinguish and kill some certain pathology or microorganism can be applied only autologously into the same patient bloodstream their potency for HIV treatment and moreover, infection prevention, is rather doubtful. The question about where to obtain viral peptides for macrophage's "teaching" is the same, recombinant ones have fixed for years sequences, and native ones should be provided in huge concentrations nowhere to isolate. Therefore dendritic cells application cannot be supposed as the serious anti-HIV vaccine's candidate.

The only possible way of the HIV-1 pandemia control is the creation of a vaccine which is able to prevent HIV-1 infection and/or to stop its development through immunization of non-infected individuals, especially representatives of high risk groups. Such vaccine must comprise the mixture of individual natural HIV-1 peptide's epitopes, precisely major HIV-1 envelope protein gp120 which is only outer one on viral surface, it's fragment's epitopes, and also gp41 peptide as the material for env gp120-gp41 tetramer with appropriate outer parts and/or epitopes recognizable for immune system of vaccinated individual. These peptides cannot be native ones from virus for the reasons mentioned above (pp. 3-4 lines 13-30, 1-20). And for recombinant peptides the correct sequence information should be delivered. We developed an alternative way of HIV vaccine development specified in details in this patent application including the env sequence study based on:
1) collecting and affine purification of native viral peptides with phage display reverse panning technique;
2) following quantitative and sequencing analysis of native viral peptides using a group of LC-MS methods delivering information about sequences of gp120 and it's fragments represented in major number of variants in the current cohort of HIV-infected individuals;
3) reconstruction of natural env peptides epitopes using leishmania system for recombinant env peptides production with identical to HIV and eukaryote's glycosylation;
4) composition of HIV immunogenic composition using approach of either sterically stabilized liposomal packaging or virosomes for immunogenic env peptides providing a) necessary immune boost period prolongation b) immunotoxicity control

The present invention relates to a method for producing an HIV immunogenic composition, comprising the steps of:
a) creation of a human recombinant IgG phagemid library containing HIV-1 specific scFv antibody fragments created from RNA isolated from B-lymphocytes of infected individuals,
b) enrichment for HIV-specific scFv antibody fragments in the phagemid library by panning with native or recombinant HIV-1 peptides,
c) multiplying HIV-1 material comprising HIV-1 peptides, polypeptides or proteins by virus cultivation in PBMC,
d) collecting HIV-1 peptides by reverse panning of the multiplied HIV-1 material using the enriched HIV-1 phagemid library of step b) bound to a support,
e) identification and characterization of the HIV-peptides obtained in step d),
f) expressing glycosylated env HIV-1 peptides using the results of step e) in an expression system, wherein the expression system is a suitable host with eukaryotic glycosylation, such as a *Leishmania tarentolae* expression system,
g) purification of the glycosylated env HIV-1 peptides, and
h) production of an immunogenic composition.

Proteomics analysis of gp120 done up to now was rare and incomplete due to lack of native peptide's variants purified from cocktail of other viral peptides and cellular proteins. Reverse panning technique with affine sorption of viral *env* peptides at columns with sufficient absorption capacity can solve this problem. Before vaccine composition for immunization against HIV infection is created it is necessary to select isoforms of *env* peptides that are presented in majority in current cohort of HIV infected individuals.

In spite of great variability of genetic variants 10⁵ for a single patient in average the selection of most adopted and having higher infective survivorship variants takes place in each infected person. Data of epidemiologic variability prove that spreading of HIV variants has territory bounds, sexual- or IDU- transmission personal contacts dependence as genetic sequences present. The number of dominating viral peptides variants is definitely much smaller than genetic variants though can alter to different number of dominations quickly enough. And nucleotide sequence cannot give information which ones are dominating and infectively dangerous variants, only proteomic quantitative and sequence analysis can. This method that we have tried is liquid chromatography ion electrospray mass spectrometry.

Native gp120 HIV peptides have high immunogenicity but to keep the same level for recombinant variants without loss of epitopes identity requires recombinant system with the similar glycosylation. It is possible to use cell cultures, yeast cultures and leishmania systems to solve this problem. Eukaryotic cell culture production brings very little amount of recombinant peptides due to a large number of own cellular proteins - decades of million in average compared to 1000 in E.coli. Yeast cultures provide sufficient production but carbohydration in yeasts is not so very much similar to eukaryotes and HIV as it was supposed earlier. Therefore we have chosen leishmania system with inducible and high expression and way of glycosylation typical for eukaryotes. Gp 120 recombinant variants produced in leishmania provide high and 100% HIV-specific immune response, the next stage was to make this response elongated for infection development prevention.

There are two possible ways how sterically stabilized liposomes can be used as peptide vaccine carries: either peptides are being encapsulated in water content of liposomal vesicles or bound to activated distal PEG ends and presented on liposome's surface. In both cases *env* peptides are protected from rapid protease cleavage and degradation, therefore immune boost period is elongated. Sterically stabilized liposomes are non-toxic and harmless themselves. These visicles can keep enloaded immunogenic peptides inside for several weeks or months and are able to lease their content gradually within this long enough period and not at once. This makes it possible to use more protein amount for one vaccination. The stronger and longer immune responses are being formed when longer permanent contact with foreign proteins for HCC is provided. It might be crucial for preventive HIV infection catching and development vaccine success.

The last what should be kept in mind about HIV vaccines candidates analysis is that there are no existing adequate in vivo models for their effectiveness preclinical assessment. All attempts to use chimpanzees for modeling HIV infection with further treatment with antiretroviral chemotherapeutic developments were persuading and valid but it is not possible to evaluate anti-HIV immune response in chimps or macaques-resus. Immunogenic reactions that could be elicited in apes and monkeys are quite different in spectrum from those that are being produced in humans with the same antigen immunization. Moreover, chimpanzees, for example, can be infected with any HIV subtype and live happily with lethal for humans levels of viral load for many years without any slightest sign of disease development symptoms as well as it happens with their own simian virus infection. So for testing any anti-HIV immunogenic compositions normal laboratory mice are not worse than apes but are available in statistically significant number and more frequent blood immunoassays. Clinical trials only can certify whether immunoprotective effect is provided by the disclosed HIV immunogenic composition.

### FIGURES

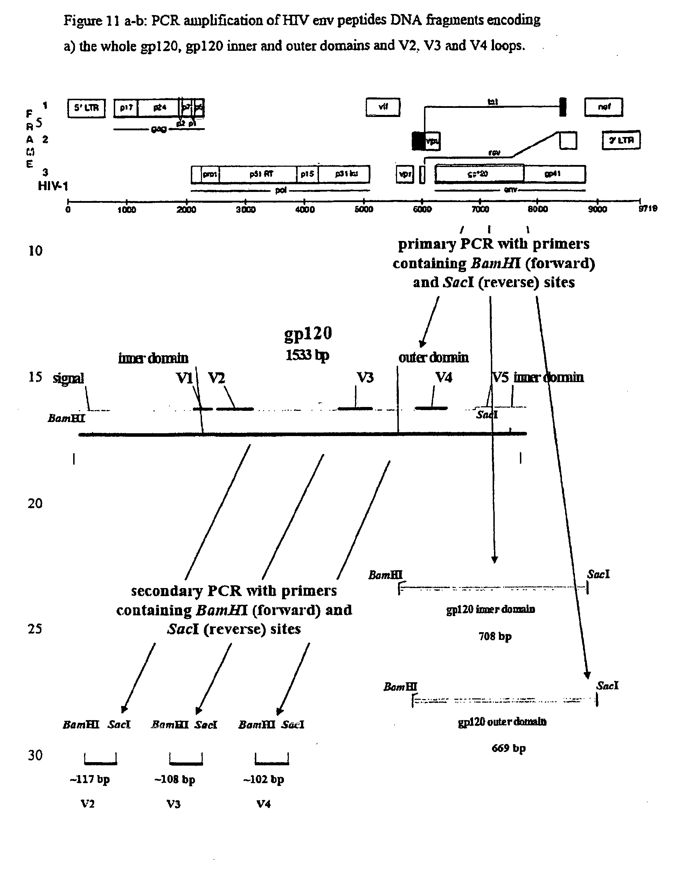

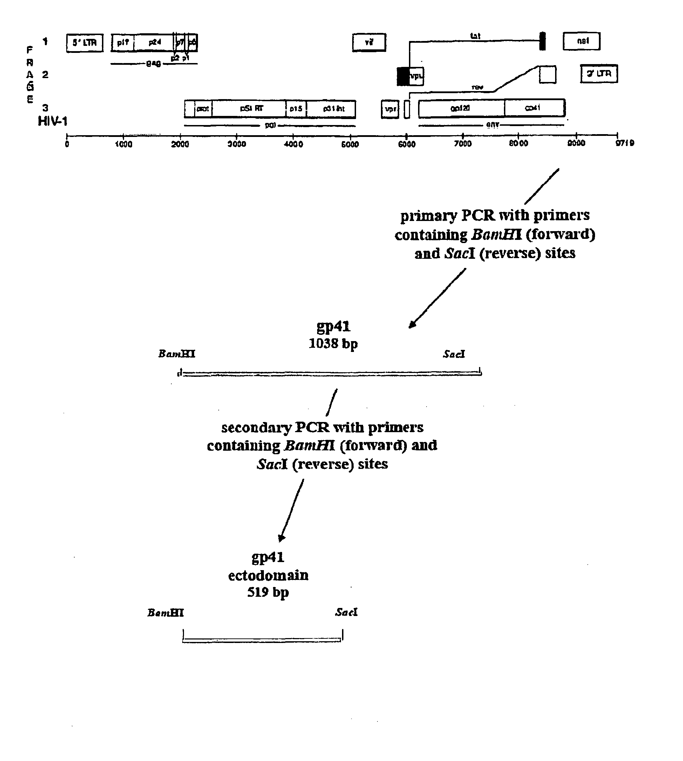

Fig. 1: HIV infected person's B lymphocytes analysis done with CD-45 monoclonal antibodies, confocal microscopy:
   a,b) "good" source for HIV-specific mAbs RNA isolation;
   c,d) rather "poor" source from patient with advanced disease stage progression (AIDS);
   e) T- and B-lymphocytes from infected person's blood, transparency scanning;

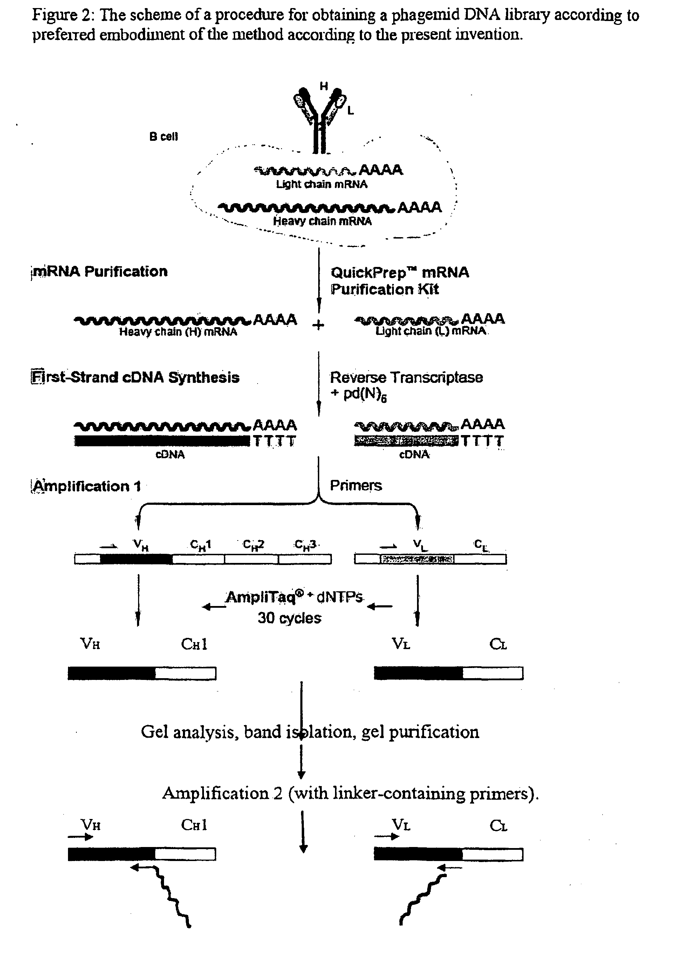

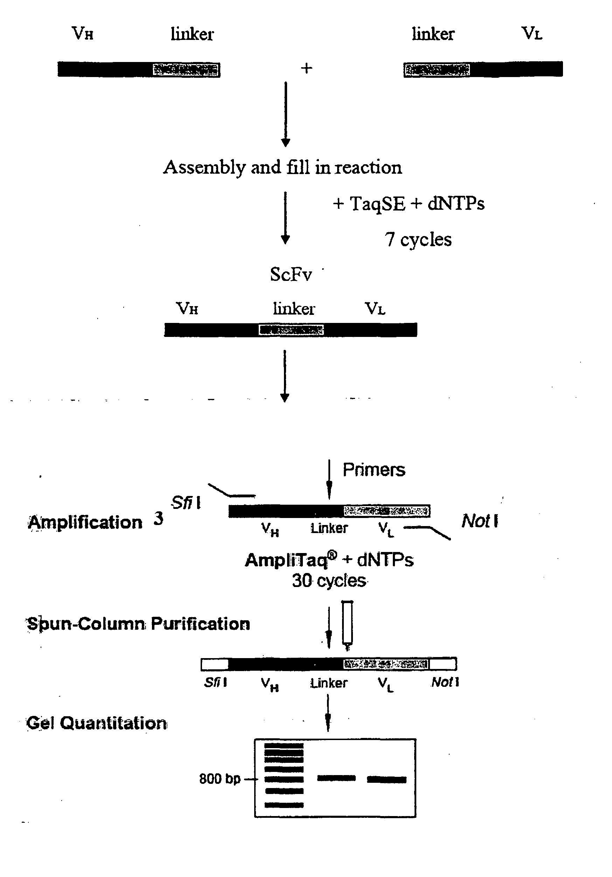

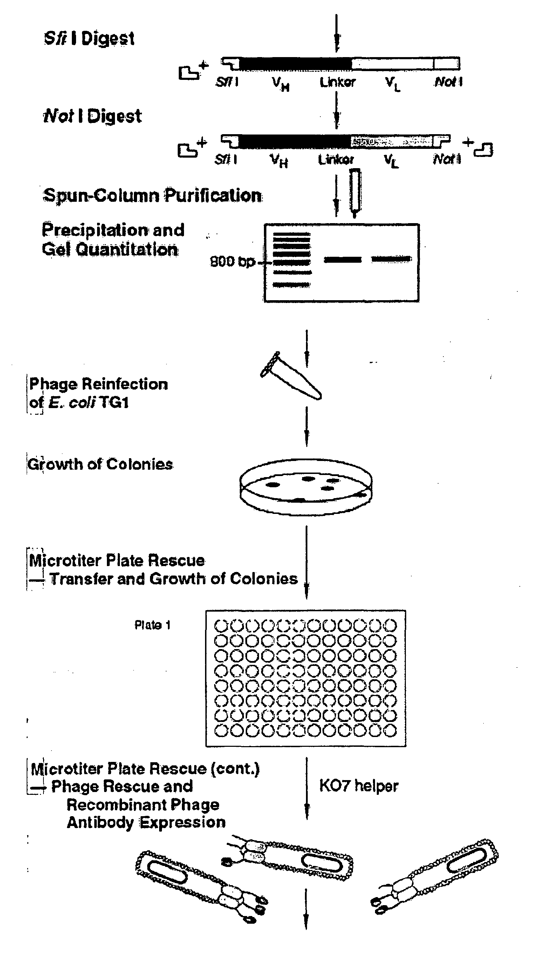

Fig. 2: The scheme of a procedure for obtaining a phagemid DNA library according to a preferred embodiment of the method according to the present invention;
Fig. 3: A diagram indicating selection of a positive antibody producing clone by ELISA technology according to a preferred embodiment of the present invention;

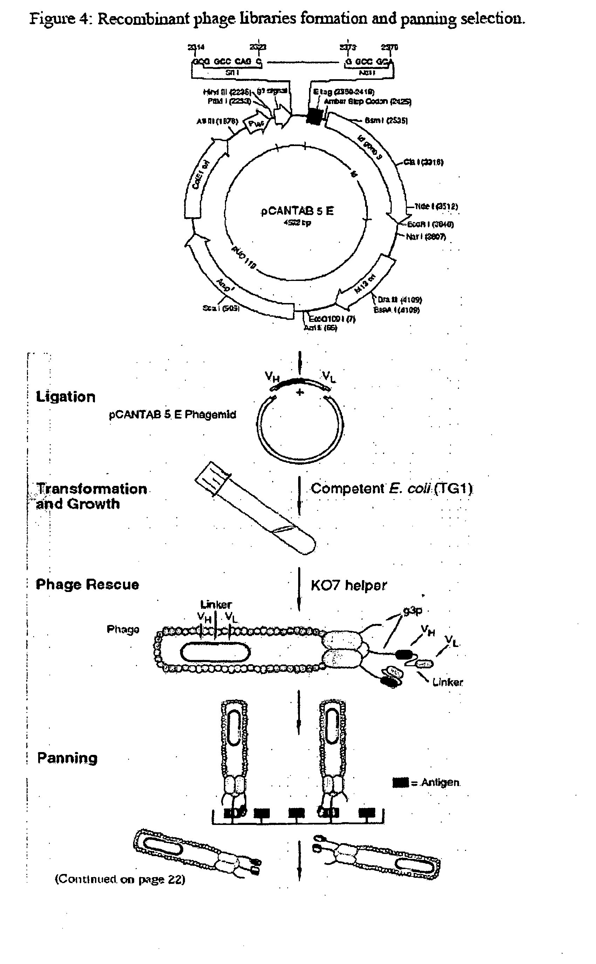

Fig. 4: Recombinant phage libraries formation and panning selection;
Fig. 5 a-b: The structure of recombinant helper M13 phage with presented on "heads" enriched HIV env peptides-specific antibodies library. Scanning Probe Microscopy (SPM or AFM) contact mode is performed using NanoWizard (JPK Instruments, Germany) on base of Nikon Eclipse 2000U, with sting cantilever CSC17/noAl, resonant frequency 12 kHz (MicroMash, Estonia).
   Phage length is 800 nm in average, thickness 40-50 nm, the presentation of HIV-specific ScFv library is 2-10 antibodies molecules for one phage particle; the measured size of this "head" is 200-250 nm in average.
   a) recombinant M13 phage and its "head" with presented HIV-specific antibodies library
   b) control M13Ko7 helper phage;

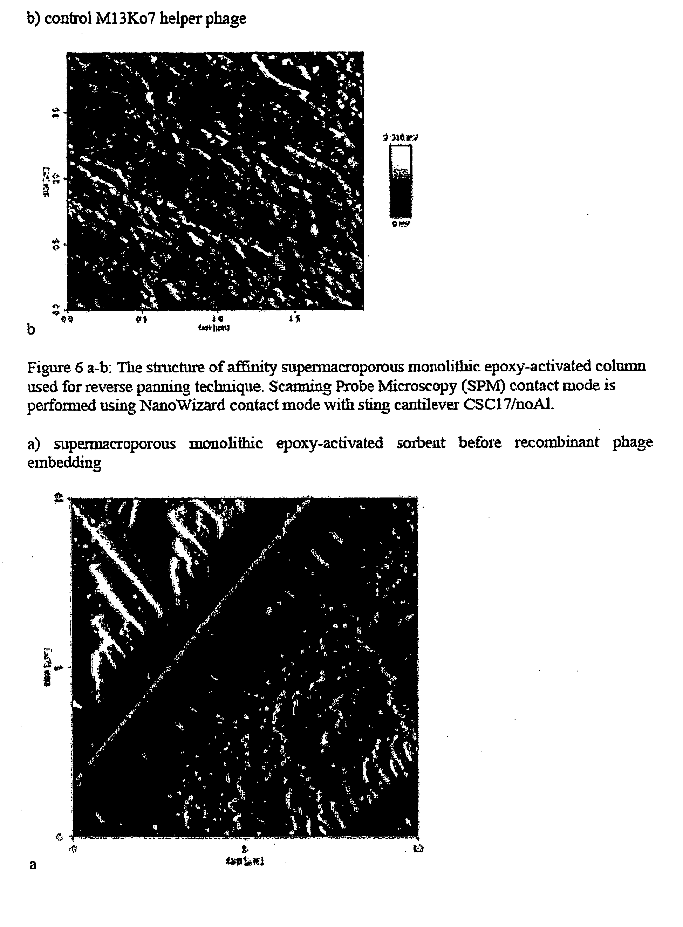

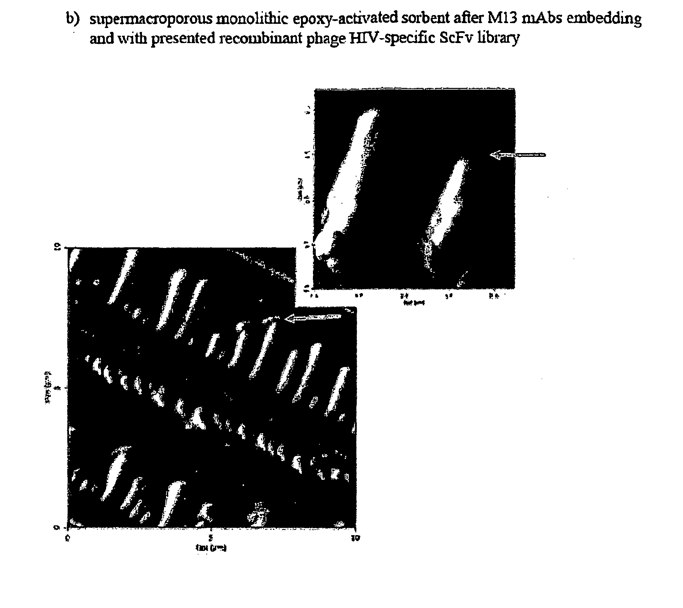

Fig. 6 a-b: The structure of affinity supermacroporous monolithic epoxy-activated column used for reverse panning technique. Scanning Probe Microscopy (SPM) contact mode is performed using NanoWizard contact mode with sting cantilever CSC17/noAl.
   a) supermacroporous monolithic epoxy-activated sorbent before recombinant phage embedding
   b) supermacroporous monolithic epoxy-activated sorbent after M13 mAbs embedding and with presented recombinant phage HIV-specific ScFv library;

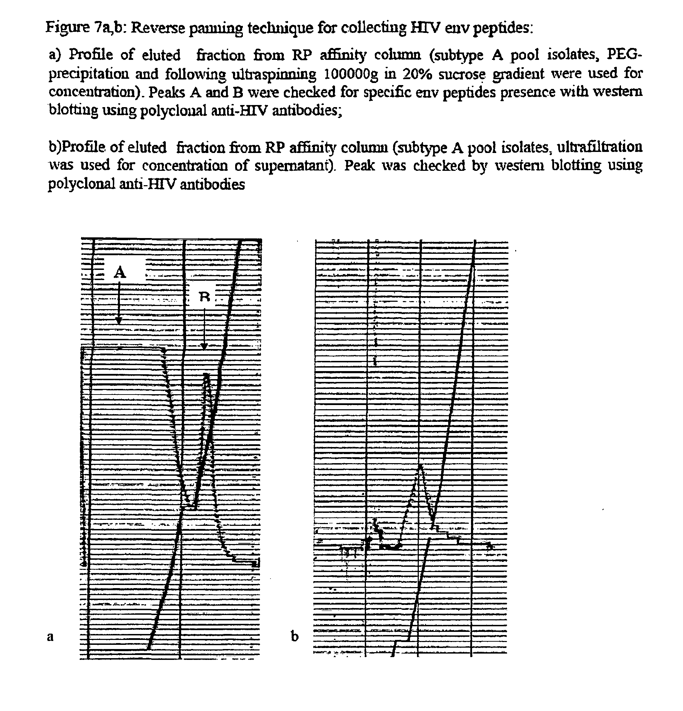

Fig. 7a,b: Reverse panning technique for collecting HIV env peptides:
   a) Profile of eluted fraction from RP affinity column (subtype A pool isolates, PEG-precipitation and following ultraspinning 100000g in 20% sucrose gradient were used for concentration). Peaks A and B were checked for specific env peptides presence with western blotting using polyclonal anti-HIV antibodies;
   b)Profile of eluted fraction from RP affinity column (subtype A pool isolates, ultrafiltration was used for concentration of supernatant). Peak was checked by western blotting using polyclonal anti-HIV antibodies;

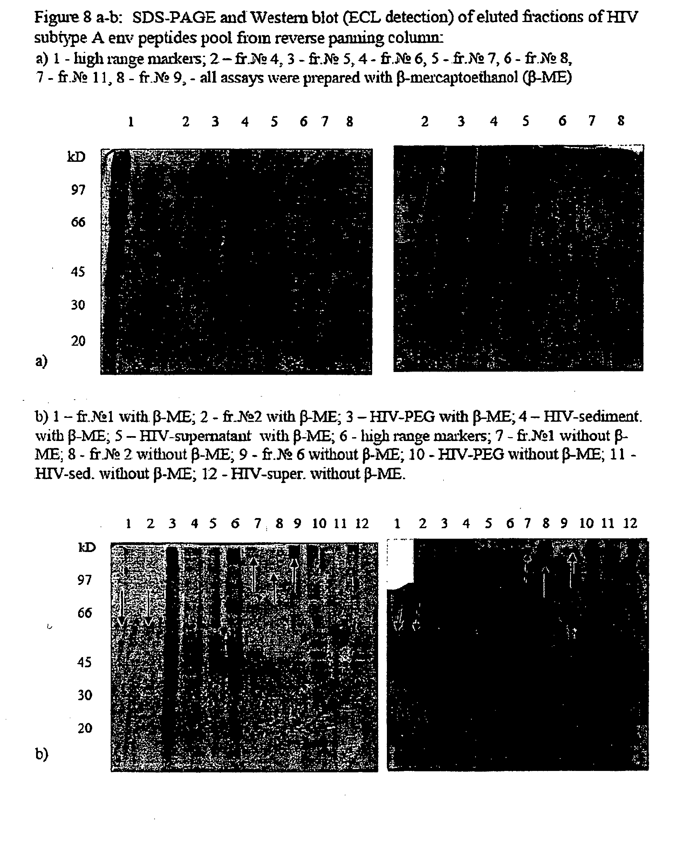

Fig. 8 a-b: SDS-PAGE and Western blot (ECL detection) of eluted fractions of HIV subtype A env peptides pool from reverse panning column:
   a) 1 - high range markers; 2 - fr.Nº 4, 3 - fr.Nº 5, 4 - fr.Nº 6, 5 - fr.Nº 7, 6 - fr.Nº 8, 7 - fr.Nº 11, 8 - fr.Nº 9, - all assays were prepared with β-mercaptoethanol (β-ME)
   b) 1 - fr.Nº1 with β-ME; 2 - fr.Nº2 with β-ME; 3 - HIV-PEG with β-ME; 4 - HIV-sediment. with β-ME; 5 - HIV-supernatant with β-ME; 6 - high range markers; 7 - fr.Nº1 without β-ME; 8 - fr.Nº 2 without β-ME; 9 - fr.Nº 6 without -ME; 10 - HIV-PEG without β-ME; 11 - HIV-sed. without β-ME; 12 - HIV-super. without β-ME.

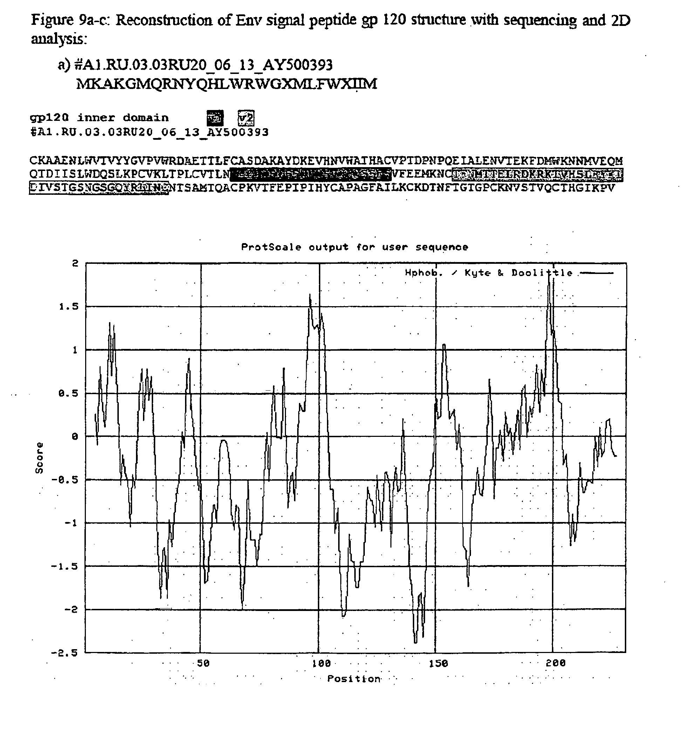

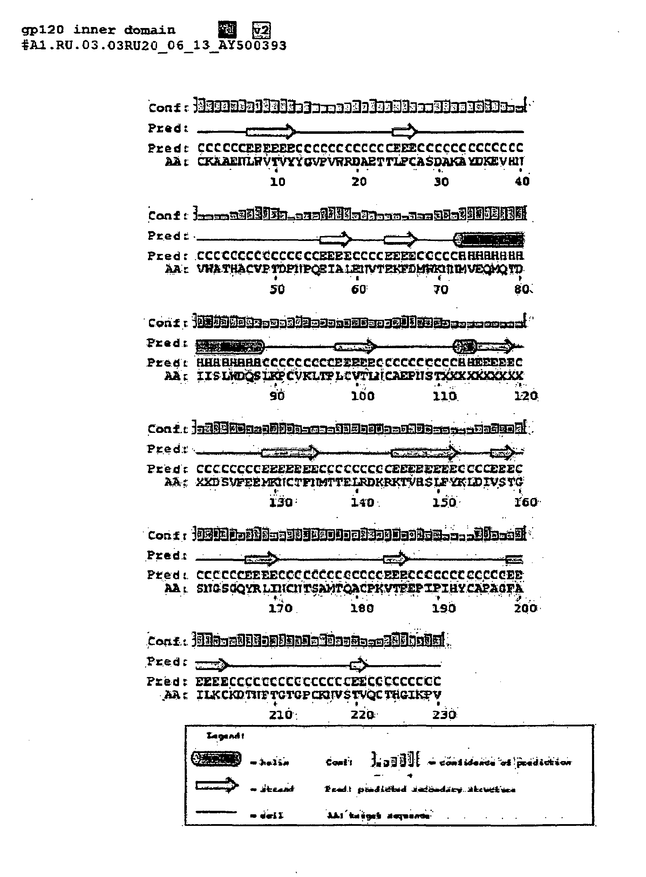

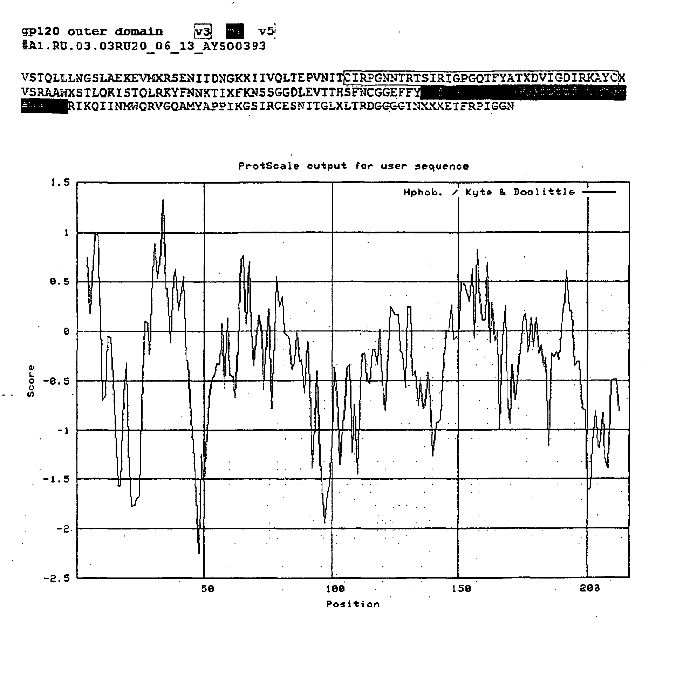

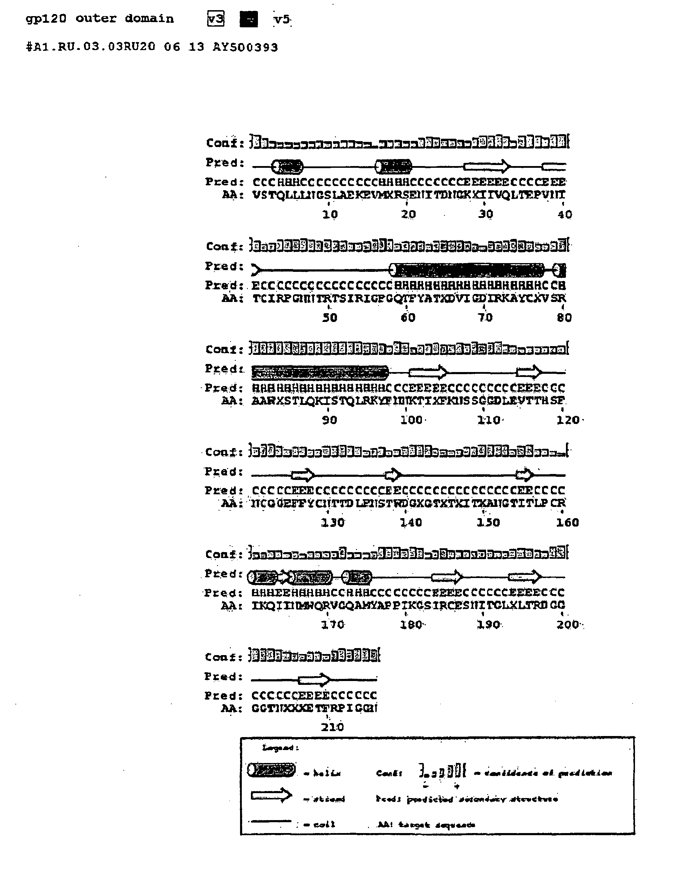

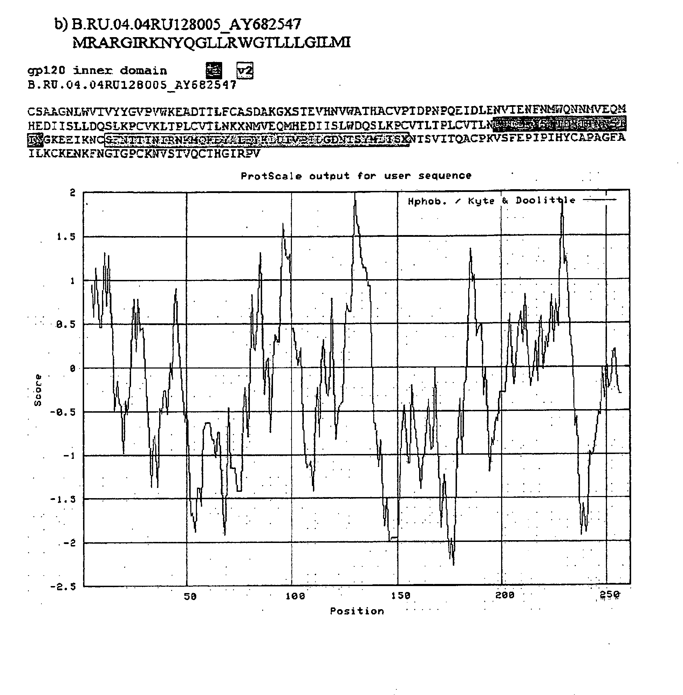

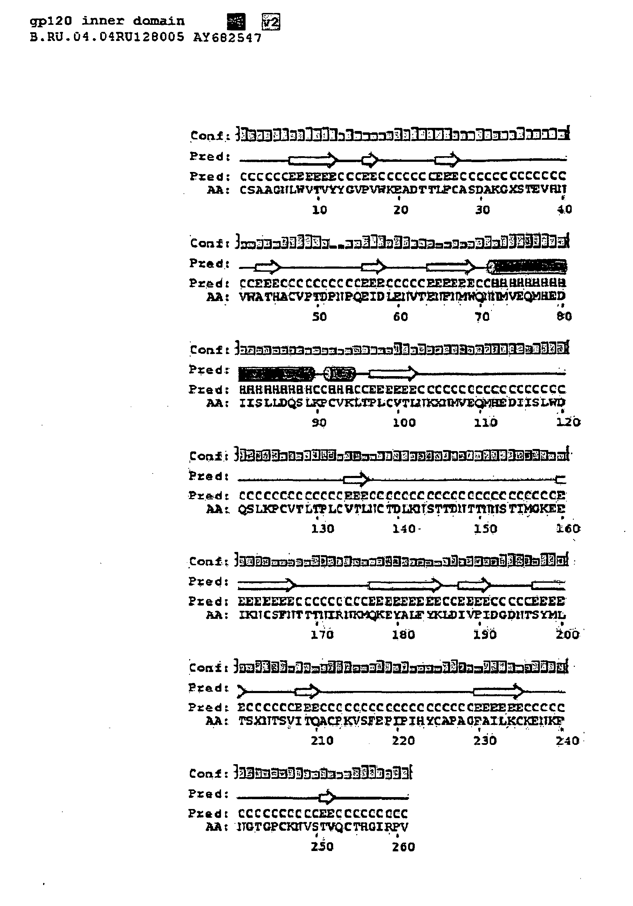

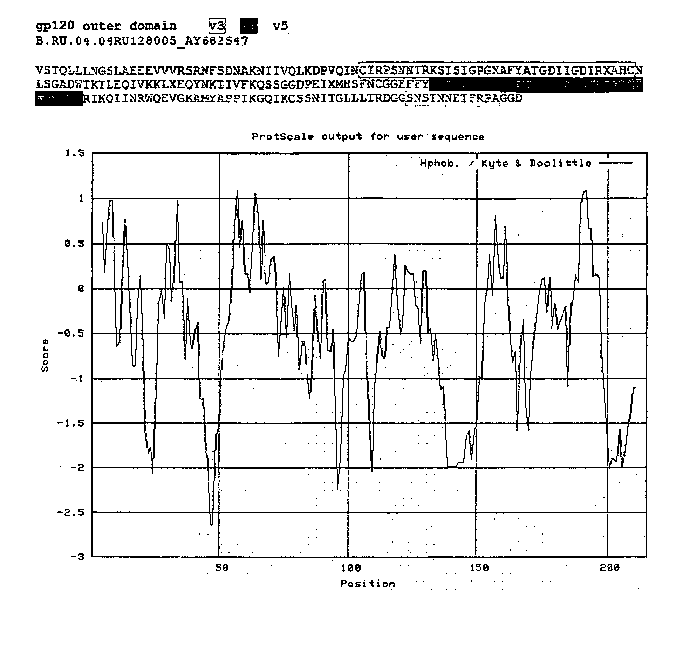

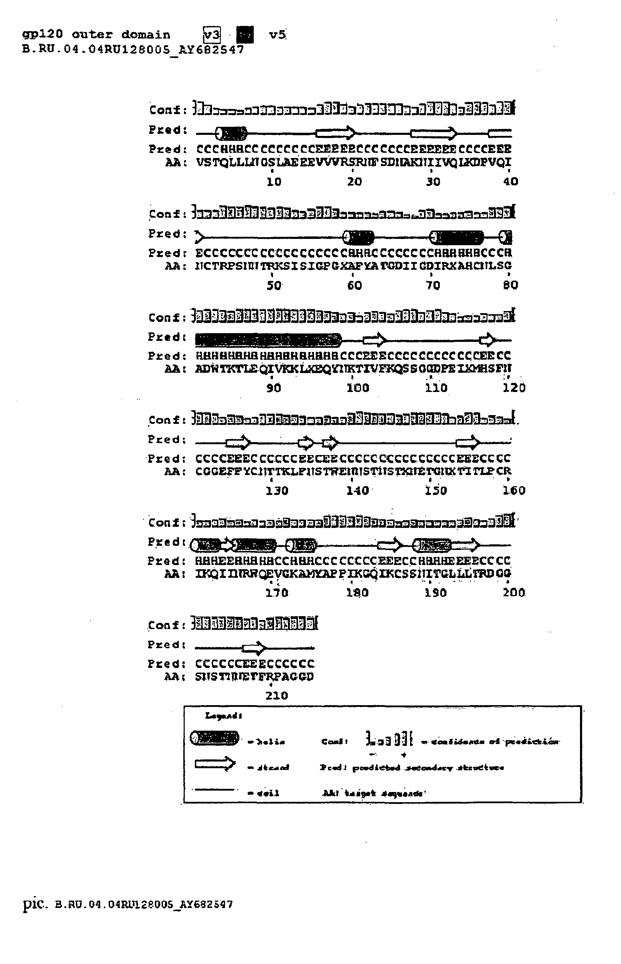

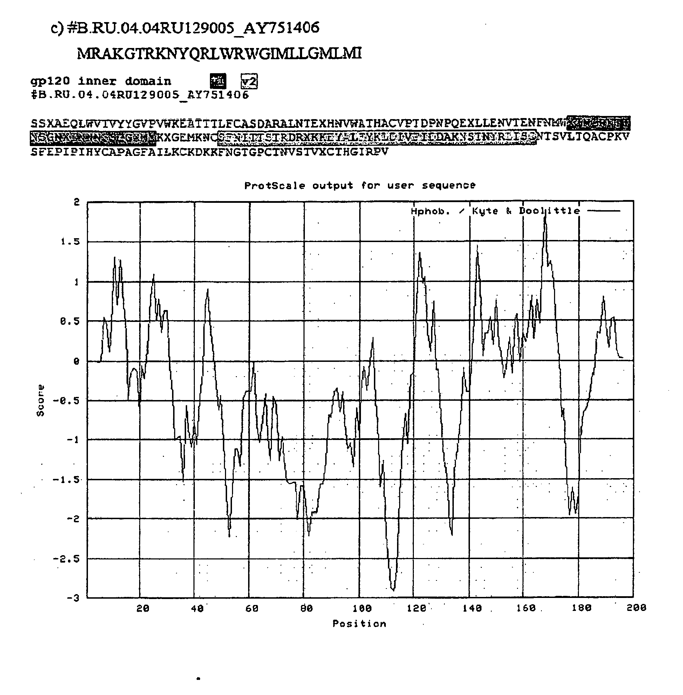

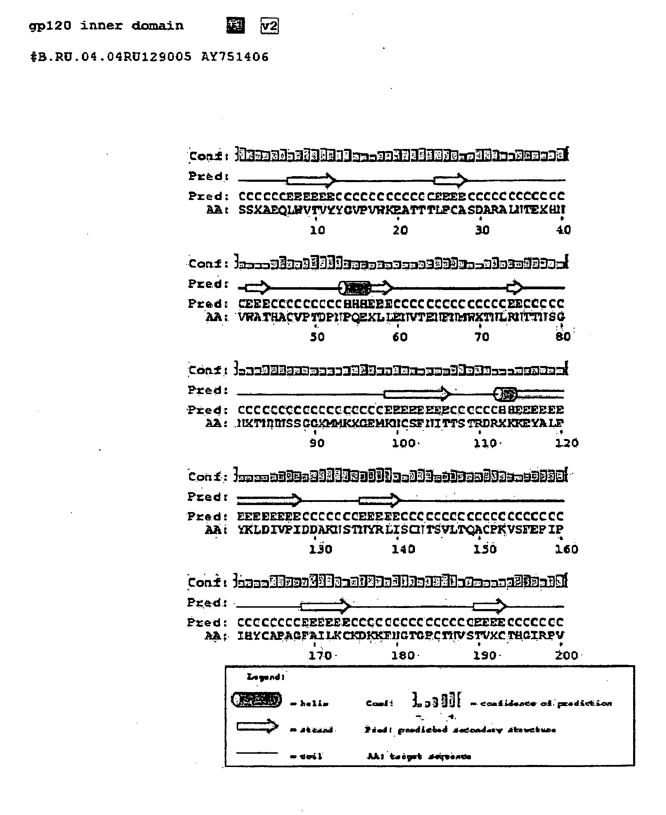

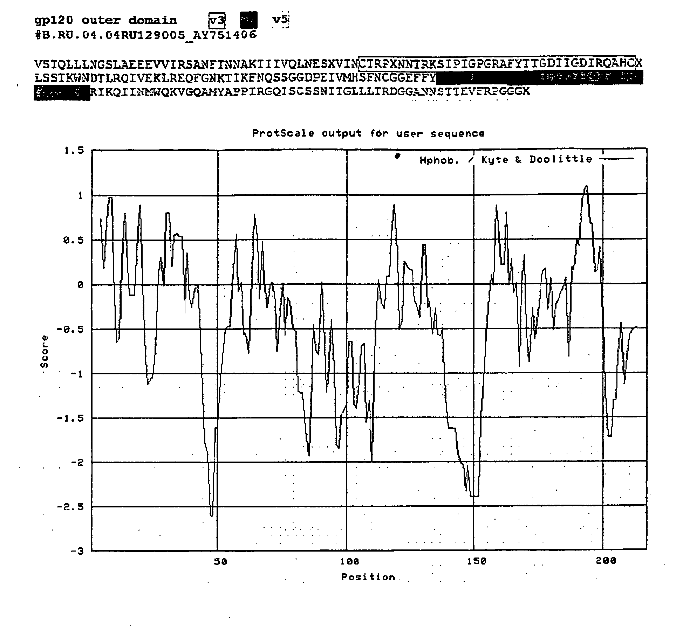

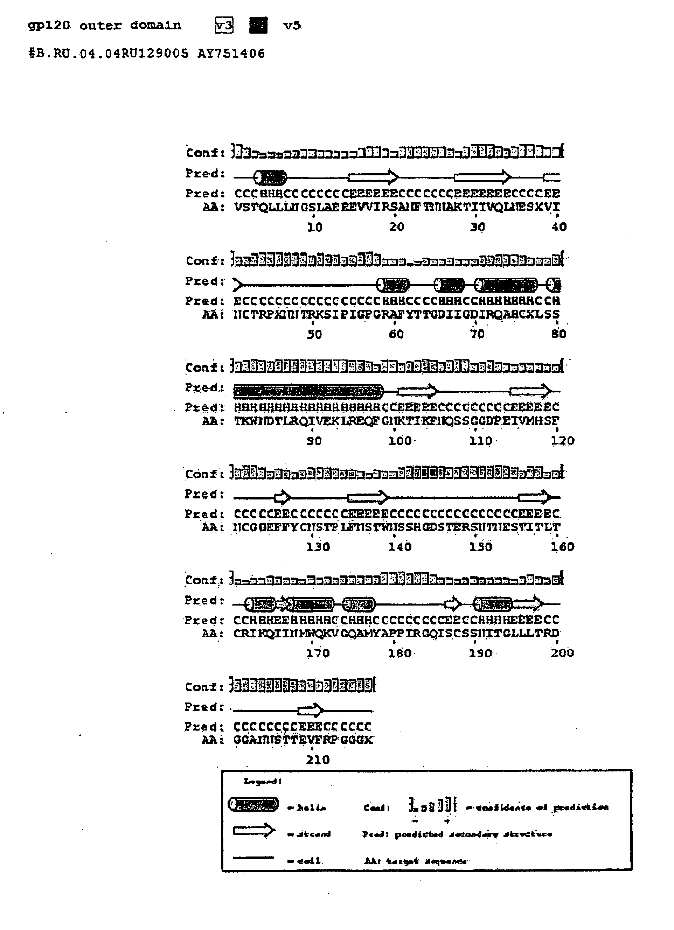

Fig. 9 a-c: Reconstruction of Env signal peptide gp 120 structure with sequencing and 2D analysis:
   a) #A1.RU.03.03RU20_06_13_AY500393
      MKAKGMQRNYQHLWRWGXMLFWXIIM
   b) B.RU.04.04RU128005_AY682547
      MRARGIRKNYQGLLRWGTLLLGILMI
   c) #B.RU.04.04RU129005_AY751406
      MRAKGTRKNYQRLWRWGIMLLGMLMI
Fig. 10 a-d: Schematic 3D structure of HIV-1 envelop peptides.
   a) Schematic 3D structure of gp120 core [40, 41]
   b) Schematic 3D structure of gp120 CD4 - CCR5 binding epitopes [24]
   c) Schematic 3D structure of gp120 transformation in CD4-binding loop formation [22]
   d) Structure and variability of gp41 ectodomain [34]
Fig. 11 a-b: PCR amplification of HIV env peptides DNA fragments encoding
   a) the whole gp120, gp120 inner and outer domains and V2, V3 and V4 loops
   b) the whole gp41 and gp41 ectodomain;

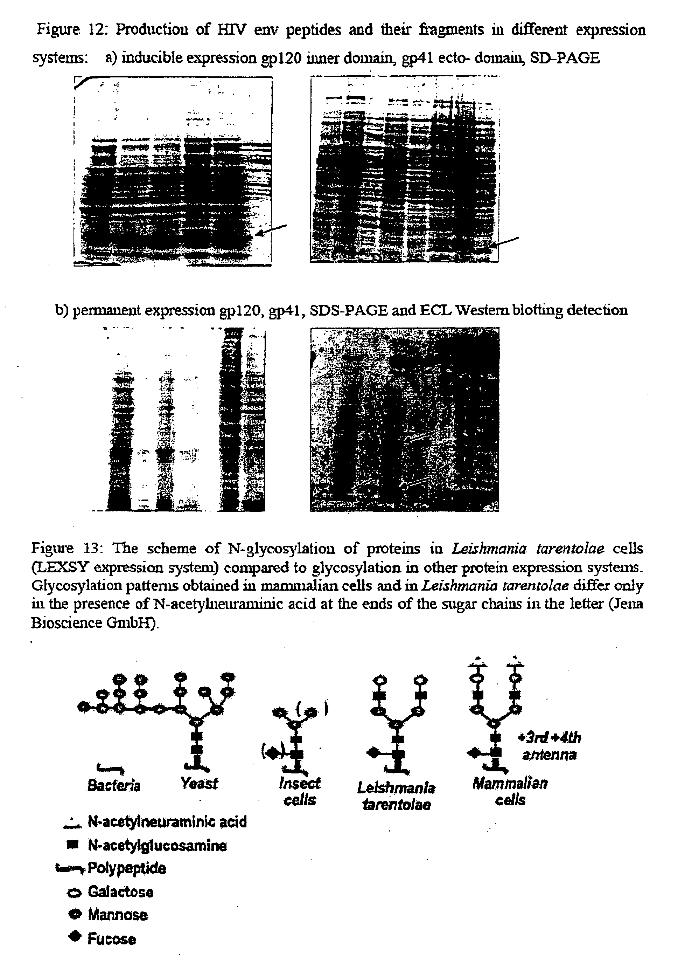

Figure 12: Production of HIV env peptides and their fragments in different expression systems:
   a) inducible expression gp120 inner domain, gp41 ecto- domain, SD-PAGE
   b) permanent expression gp120, gp41, SDS-PAGE and ECL Western blotting detection
Fig. 13: The scheme of N-glycosylation of proteins in *Leishmania tarentolae* cells (LEXSY expression system) compared to glycosylation in other protein expression systems. Glycosylation patterns obtained in mammalian cells and in *Leishmania tarentolae* differ only in the presence of N-acetylneuraminic acid at the ends of the sugar chains in the letter (Jena Bioscience GmbH);

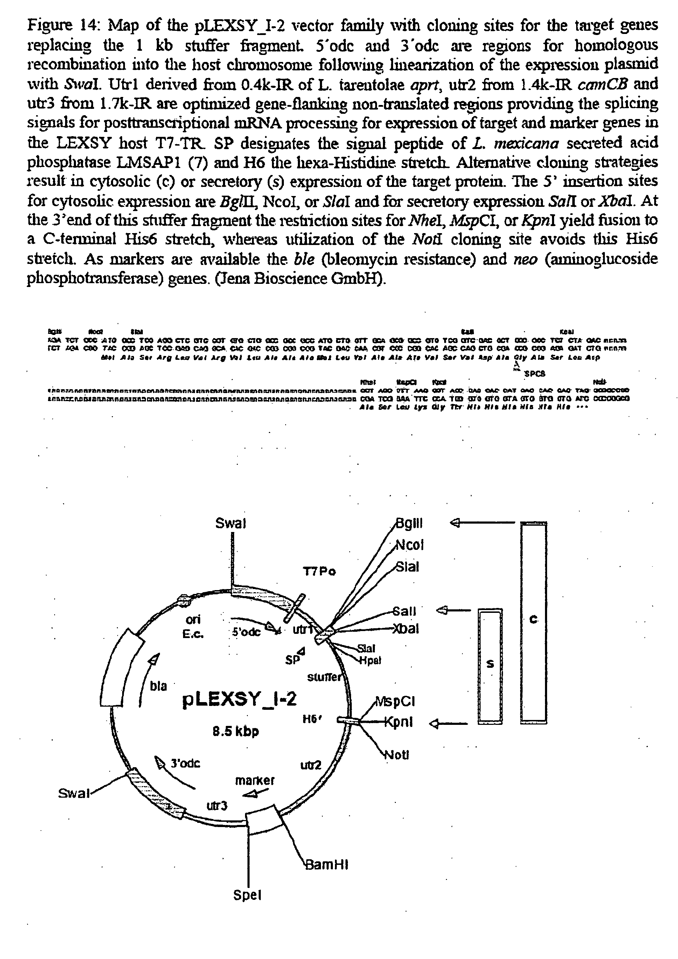

Fig. 14: Map of the pLEXSY_I-2 vector family with cloning sites for the target genes replacing the 1 kb stuffer fragment. 5'odc and 3'odc are regions for homologous recombination into the host chromosome following linearization of the expression plasmid with *Swa*I. Utr1 derived from 0.4k-IR of L. tarentolae *aprt,* utr2 from 1.4k-IR *camCB* and utr3 from 1.7k-IR are optimized gene-flanking non-translated regions providing the splicing signals for posttranscriptional mRNA processing for expression of target and marker genes in the LEXSY host T7-TR. SP designates the signal peptide of *L. mexicana* secreted acid phosphatase LMSAP1 (7) and H6 the hexa-Histidine stretch. Alternative cloning strategies result in cytosolic (c) or secretory (s) expression of the target protein. The 5' insertion sites for cytosolic expression are *Bgl*II, NcoI, or *Sla*I and for secretory expression *Sal*I or *Xba*I*.* At the 3'end of this stuffer fragment the restriction sites for *Nhe*I, *Msp*CI*,* or *Kpn*I yield fusion to a C-terminal His6 stretch, whereas utilization of the *Not*I cloning site avoids this His6 stretch. As markers are available the *ble* (bleomycin resistance) and *neo* (aminoglucoside phosphotransferase) genes. (Jena Bioscience GmbH);

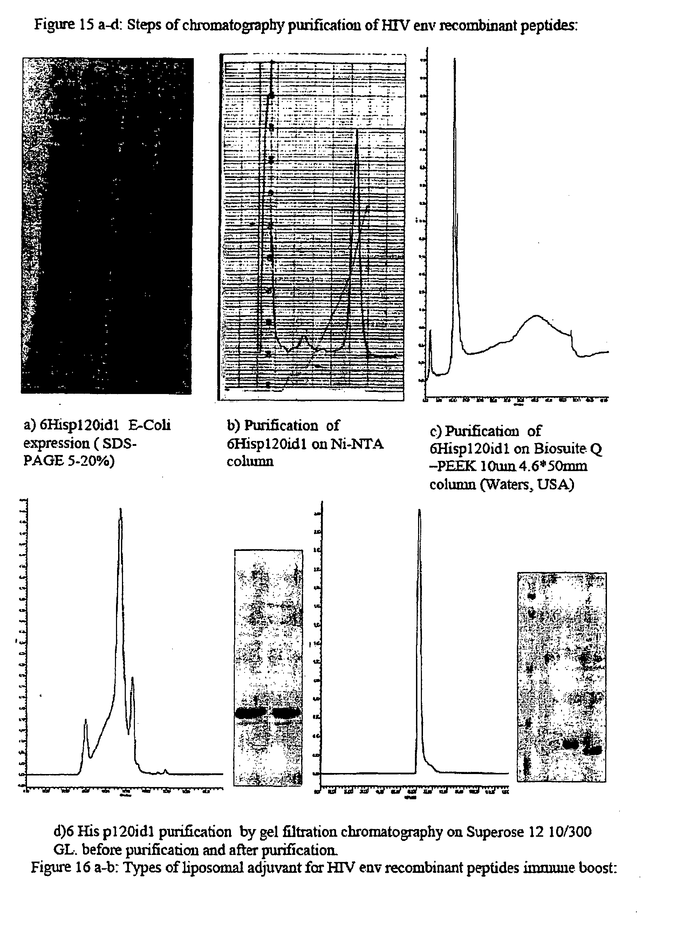

Figure 15 a-d: Steps of chromatography purification of HIV env recombinant peptides:
   a) 6Hisp120id1 E-Coli expression (SDS-PAGE 5-20%)
   b) Purification of 6Hisp120id1 on Ni-NTA column
   c) Purification of 6Hisp120id1 on Biosuite Q -PEEK 10um 4.6*50mm column (Waters, USA)
   d) 6 His p120id1 purification by gel filtration chromatography on Superose 12 10/300 GL. before purification and after purification;

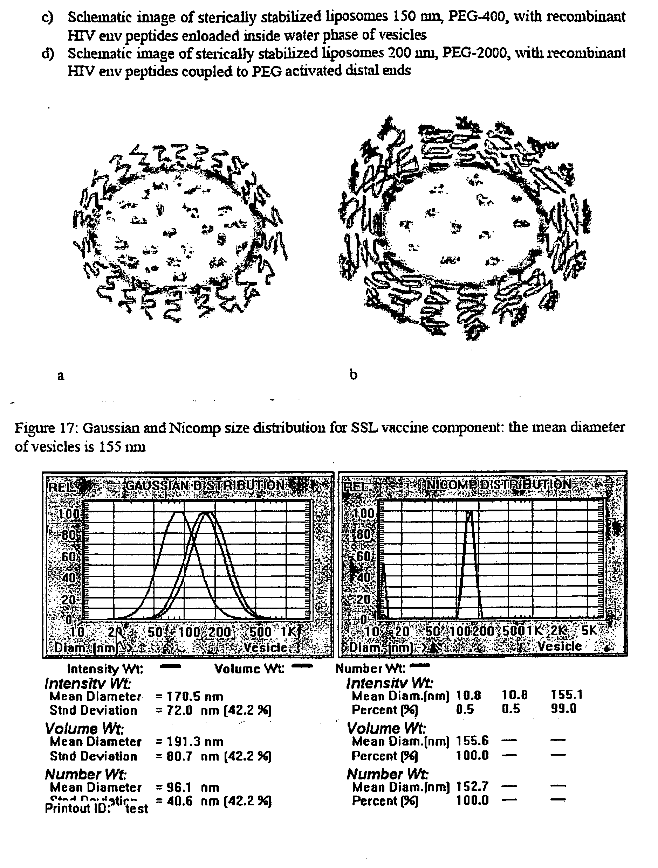

Figure 16 a-b: Types of liposomal adjuvant for HIV env recombinant peptides immune boost:
   a) Schematic image of sterically stabilized liposomes 150 nm, PEG-400, with recombinant HIV env peptides enloaded inside water phase of vesicles
   b) Schematic image of sterically stabilized liposomes 200 nm, PEG-2000, with recombinant HIV env peptides coupled to PEG activated distal ends;
Figure 17: Gaussian and Nicomp size distribution for SSL vaccine component: the mean diameter of vesicles is 155 nm.

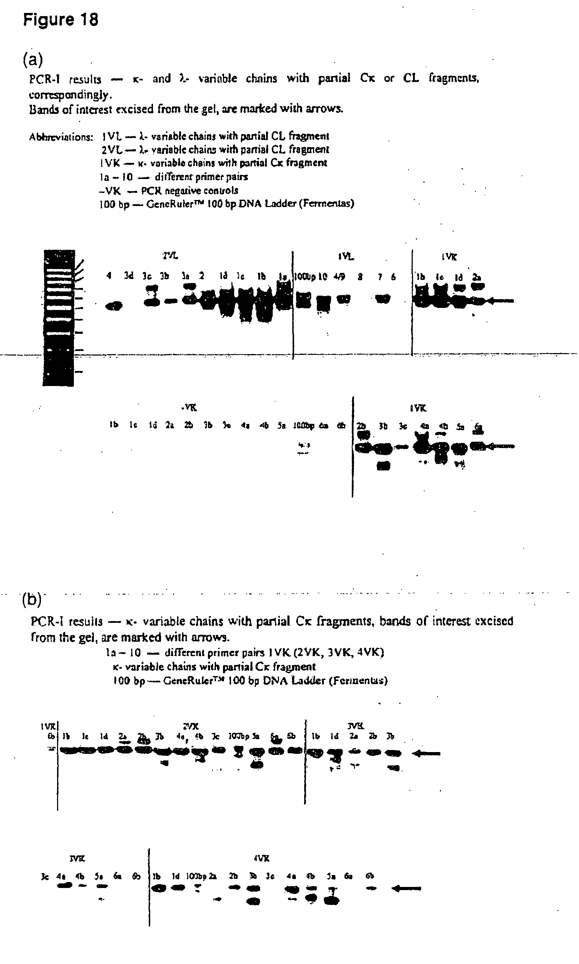

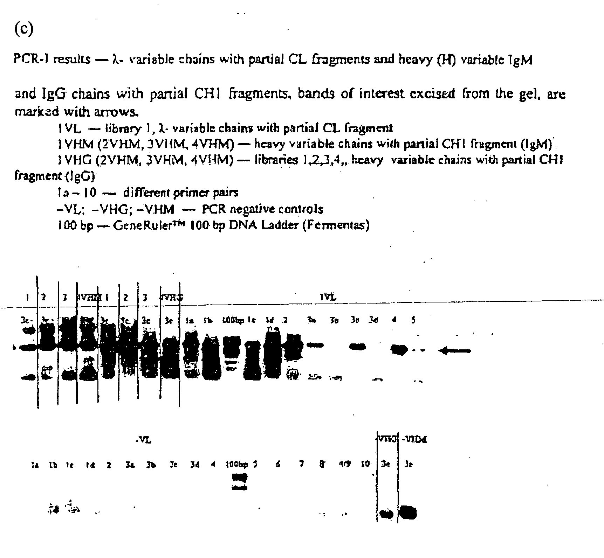

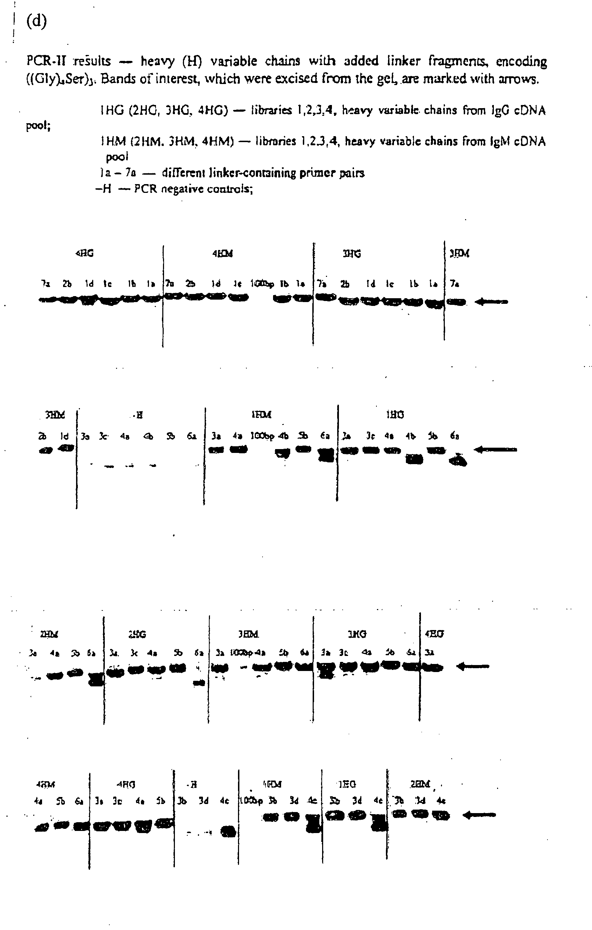

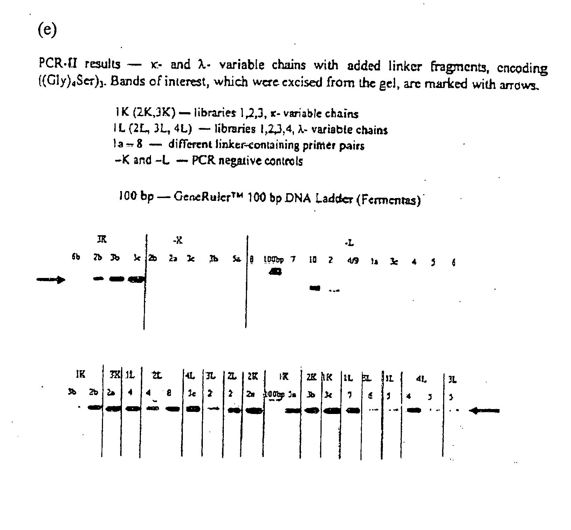

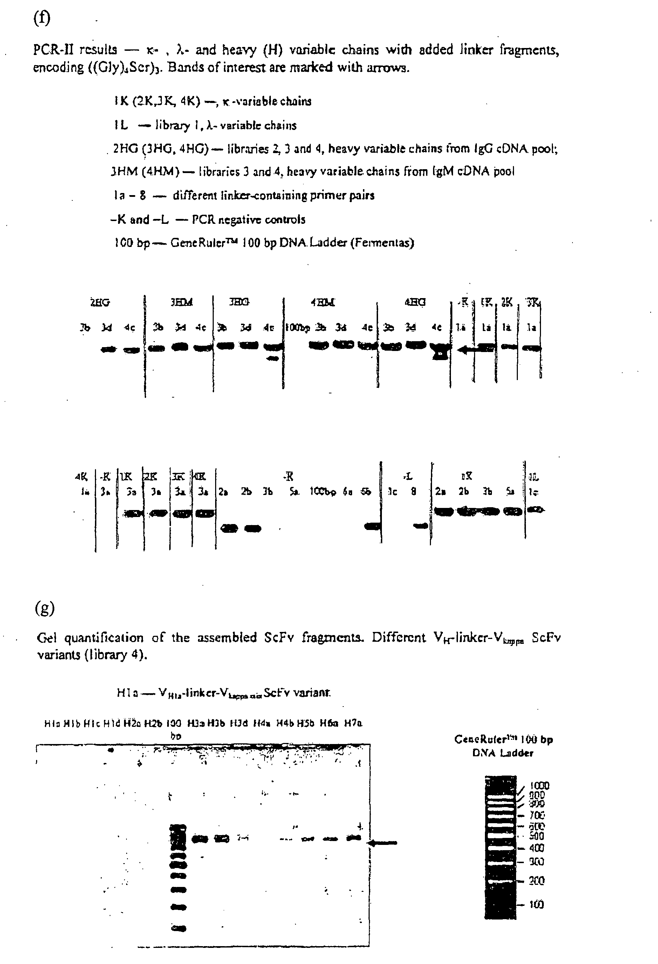

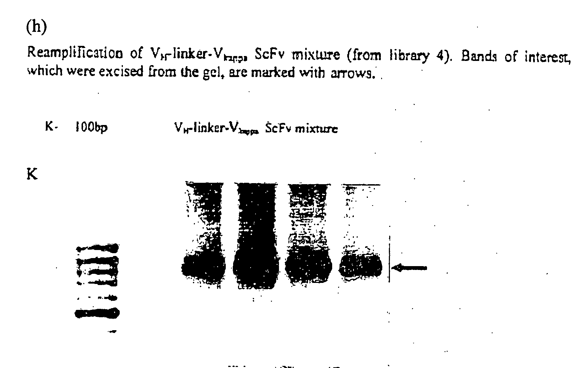

Figure 18: The electrophoresis data illustrate the stages of human recombinant IgG phagemid library containing HIV-specific scFv antibody's fragments creation (phage display technology).

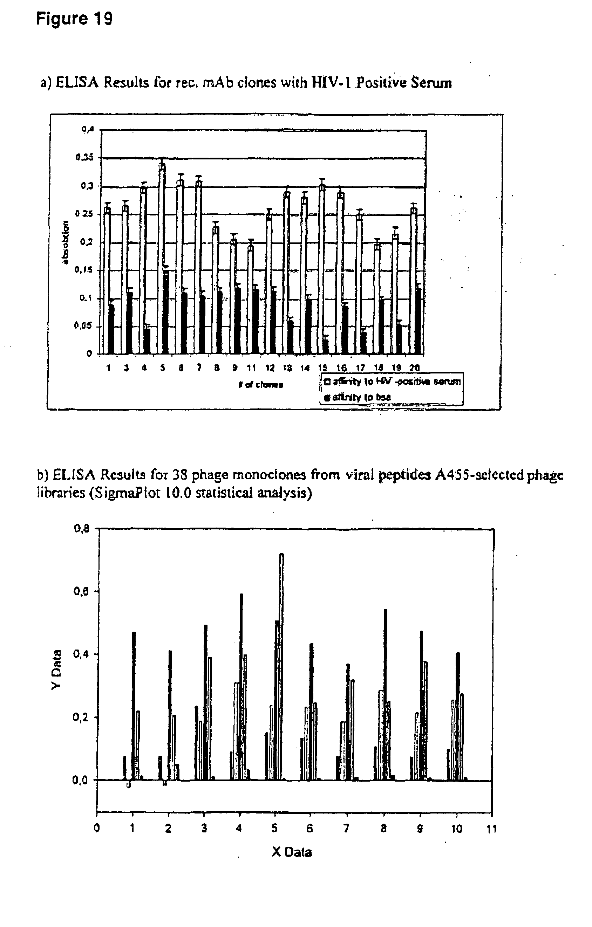

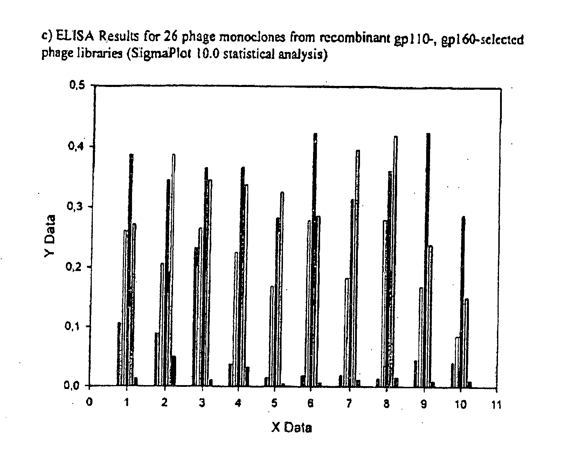

Figure 19: shows specificity quantification results for recombinant HIV-specific libraries

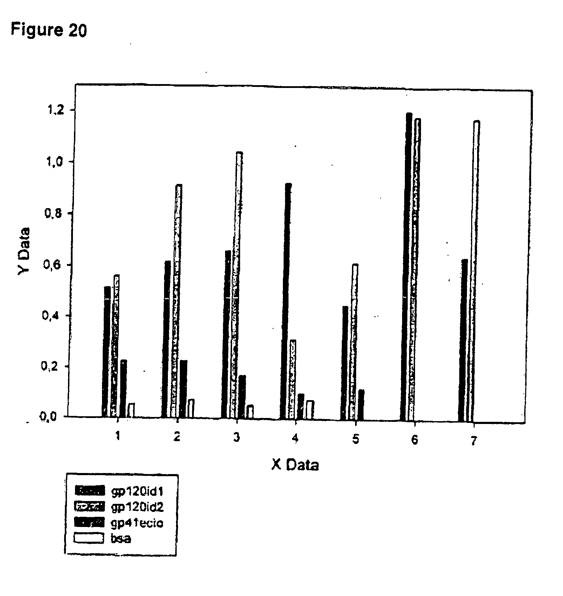

Figure 20: shows the preliminary results of the immunization of animals (SigmaPlot 10.0 statistical analysis) of Example 4

### DETAILED DESCRIPTION OF THE INVENTION

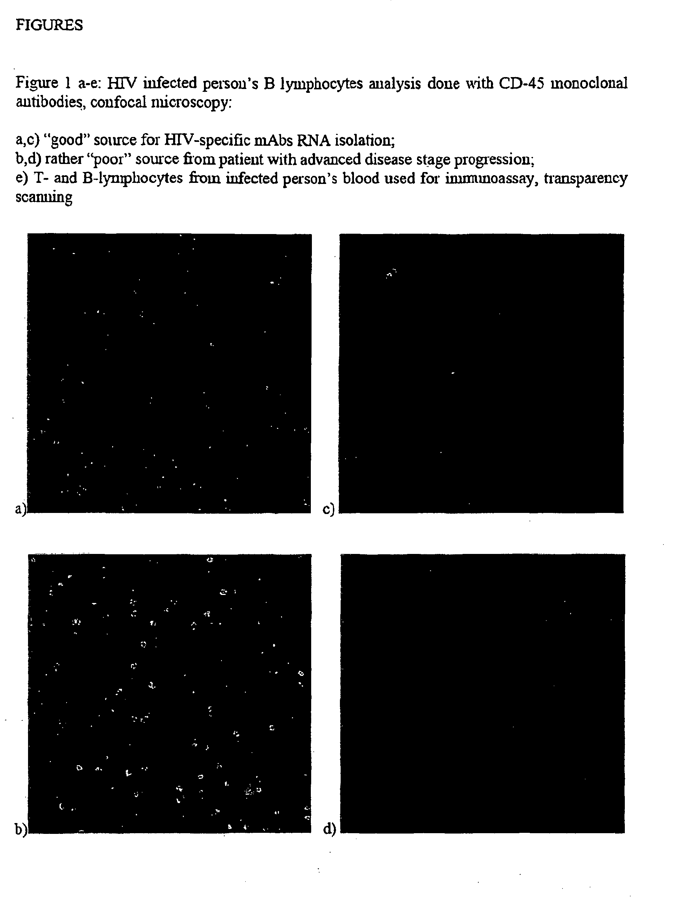

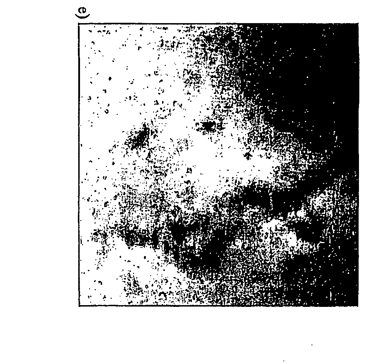

Disclosed herein is a HIV, preferably HIV-1 subtypes A and B, immunogenic composition eliciting a specified immune response in an individual challenged with the immunogenic composition. Hence, the active substance is a recombinant polypeptide/peptide mixture prepared and selected according complex technology described in detail below. Basic vaccine components are represented by viral surface and envelope proteins and their fragments that comprise according to a preferred embodiment HIV envelope proteins gp120, gp140, gp160 (Fig.1) and gp41 in different states of glycosylation, conservative domains in V1-V3 loops of gp120, antibodies to resistance-associated variable parts of gp120 V1-V5 loops, glycosylated variants of gyp41; CD4 binding epitopes to virus envelop proteins gp120, gp140, gp160 with proximal V1/V2 and V3 loops to undergo conformational change upon CD4 receptor engagement by the HIV-1 envelope spike and the external part of gp41 protein; CXCR5 and CCR4 co-receptors binding sites of virus envelope proteins; p24 viral peptide different epitopes.

These recombinant polypeptides and their mixtures are collected, identified and cloned using recombinant phage displayed library of antibodies created from different donors B-lymphocytes mRNA. Each created phage antibody library is specific to bind different epitopes of recombinant gp120-, gp41 and native HIV-polypeptides, and preferably also to epitopes present on recombinant gp 140-, gp160- and p24 HIV-1 subtype A proteins.

These recombinant phage antibodies libraries may be used in different applications as detecting, analyzing and/or purification means [23]. Applications using the above antibody libraries comprise, but are not limited to, immunoassays, immunoblots, chromatography, etc..
The antibodies are also useful for the development of new medicaments for HIV treatment and/or prevention.

In a preferred embodiment of the present invention recombinant antibodies presented on M13KO7 phage are used for the development of a HIV immunogenic composition.

Since the antibody fragments displayed by the phagemid library bind to essentially conserved conformational epitopes of HIV proteins, the targets of said antibodies qualify as a vaccine against HIV infection, since upon challenge the individuals immune system will develop a specific immune response against these epitopes, i.e. mature B- cells and T-cells, which will eventually end as memory cells, present in the individual to convey immunity.

A HIV immunogenic composition disclosed comprises recombinant gp41 and p24 HIV-1 subtype A proteins and fragments of gp120, gp140 and gp 160, which fragments (Table 9 from Example 3) bind to an antibody as prepared by the method according to the present invention, and in addition conventional carriers and excipients and optionally immune stimulans.

Said recombinant proteins and/or fragments are based on sequence information acquired by binding and analyzing native HIV-1 envelop proteins which are selected with HIV-specific antibodies obtained by the method disclosed herein.

In detail, proteins, such as envelope proteins are obtained from disrupted viral particles by appropriate methods such as ultracentrifugation and lysis of viral particles.

The selection of suitable proteins may be performed by reverse panning using the phagemid library bound to a support. In a preferred embodiment the selection may be performed either by (i) phage panning with usage of recombinant phage with presented antibodies for collecting viral envelope proteins, and/or (ii) affine sorption on HIV-specific antibodies adhered to a surface of plastic for cultivation, and/or (iii) affine chromatography selection of viral envelope proteins with column embedded HIV-specific antibodies.

In a next step a sequence of the obtained and selected native viral proteins and/or 3D conformation of isoforms may be identified. Proceeding accordingly provides a mixture of several variants highly specific variable and/or constant fragments of viral proteins, such as gp120, gp41 and p24 circulating in bloodstream of HIV-1 infected individuals and also those of them who received antiretroviral therapy in different regimes such as variants of NRTI, NNRTI and HAART.

Based on said sequences recombinant polypeptides and/or fragments of viral proteins are produced. These sequences may be obtained by using any method suitable to produce polypeptides which can be recognized by the immune system to induce an appropriate immune response thereto.

Said recombinant polypeptides are obtained in any suitable expression system, wherein the expression is a host with eukaryotic glycosylation, such as an eukaryotic expression system, such as leishmania inducible expression system and yeasts with an eukaryotic-like glycosylation.

An exemplary technique for the preparation of different variants of HIV-1 A and B subtypes immunogenic composition includes steps 1-9, which will be illustrated in more detail below:
1. Creation of human recombinant IgG phagemid library containing HIV-specific ScFv antibody's fragments (phage display technology);
2. The enrichment of recombinant phagemid library presenting HIV-specific antibody's ScFv fragments (biopanning);
3. Optionally, multiplication of antiretroviral therapy naïve viral material in situ with PBMC-MT infection method;
4. Concentration of HIV particles and peptides; virus inactivation and disruption;
5. Collecting the native HIV *env* peptides with HIV-specific recombinant ScFv presenting phagemid library with reverse panning technique; and
6. Optionally quantitative and sequence analysis of *env* peptides variability and frequency with Liquid Chromatography Mass Spectrometry (LC-MS) method;
7. Optionally cloning of major HIV env peptides and production of recombinant peptides for vaccine development in Leishmania tarentolae;
8. Optionally recombinant HIV env peptides chromatography purification and 3D structure analysis; and
9. Preparation of HIV preventive vaccine immune boost composition preferably using sterically stabilized liposomes or virosomes as vehicles for vaccine delivery.

### 1. Creation of human recombinant IgG phagemid library containing HIV-specific ScFv antibody's fragments (Phage display technology)

Hence in the method of the present invention, a phagemid library may be created in step 1) according to stages i) to iii), including:
i) An amplification of DNA-fragments derived from RNA encoding the variable region of a light chain and a heavy chain, respectively, of IgG expressed in B-lymphocytes obtained from a number of individuals infected with HIV are prepared;
ii) An assembly of two DNA-fragments of light and heavy chains obtained in i) into one construct comprising a nucleic acid encoding the variable region of an IgG light chain which is associated to a nucleic acid encoding a variable region of an IgG heavy chain;
iii) A transformation into pCANTAB phagemid vector.

In detail, first B- lymphocytes are isolated from a number of individuals, which are known to be infected by HIV, and in which HIV specific antibodies are expected to be present. Also individuals harboring resistant HIV-variants may be included. The isolation of the B-cells may be carried out by any known technique, e.g. leukapherese with a subsequent isolation of B cells from the lymphocyte population [19]. Subsequently RNA is isolated from the B lymphocytes by techniques well known in the art, such as e.g. illustrated in [23].

For a ScFv libraries creation mRNA containing HIV-specific immunoglobuline's sequences are isolated. In this respect the number of B-lymphocytes are evaluated, e.g. with CD45 mAb immunoassay with confocal microscopy analysis in blood of HIV-infected persons before RNA isolation. Data presented in Fig.1 show that some patients with advanced stage of disease and symptoms of AIDS have very low ratio of B-lymphocytes to total isolated lymphocytes (Fig.1 c,d), and usually low CD45 immunostaining correlates with high viral load and very low CD4 : CD8 status. Unlike the others (Fig.1 a,b) these patients are a rather poor source for HIV-specific phagemid libraries creation cohorts. High viral load along or courses of previous antiretroviral treatment and their frequency do not limit the chances to obtain HIV-specific ScFv libraries.

The total RNA such obtained may be transcribed to cDNA by e.g. using oligo dT, or, according to a preferred embodiment by using oligonucleotides as primers, specific for a constant region of the immunoglobuline heavy and light chains. The sequence of the different constant regions of the immunoglobuline heavy and light chains are well known in the art, so that appropriate primers for the transcription into cDNA may easily be designed. Proceeding accordingly allows a first selection for immunoglobuline transcripts in the RNA pool(s) and an easier handling of the different RNA-samples from the different donors, since material of no interest may be excluded in said first step. Also combining the RNA-pools from the different donors prior to transcribing the mRNA into cDNA is envisaged and preferred, since a greater variety may be obtained (cf. below). The complement of the cDNA thus prepared is synthesized according to techniques well known in the art.

In order to prepare a sufficient amount of the DNA fragments the regions of interest may be amplified using directly mRNA obtained from the B-/T-lymphocytes, cDNA or the double stranded DNA prepared from the cDNA as a template.

For said PCR reactions appropriate primers annealing to the 5'- and 3'-end of the nucleic acid sequences to be amplified may be used, which generally are oligonucleotides in a length of from about 10 - 40, preferably 15 - 30, more preferred 20 - 30 nucleotides.

As reverse primers oligonucleotides may be used, the sequence of which is derived from the constant region of the immunoglobulines. Preferably said reverse oligonucleotide primers hybridize to the CH1 region of heavy chains or Cλ or Cκ regions of the light λ and κ chains, correspondingly. The forward primers to be used hybridize to the opposite ends of the variable regions of heavy and light chains.

In a preferred embodiment forward and reverse primers for the primary PCR amplification are selected from the group consisting of nucleic acid sequences as shown in tables 1 to 3, which were taken from V BASE database (http://vbase.mrc-cpe.cam.ac.uk). The PCR reactions in general yield fragments about 750 in length.

**Table 1: List of oligonucleotide primers for PCR amplification of human immunoglobuline light κ chains.**

| # | **Name of primer, direction** | **Nucleotide sequence (5'-3')** |
|---|---|---|
| 1 | Vκ1a forward | RAC ATC CAG ATG ACC CAG |
| 2 | Vκ1b forward | GMC ATC CAG TTG ACC CAG |
| 3 | Vκ1c forward | GCC ATC CRG ATG ACC CAG |
| 4 | Vκ1d forward | GTC ATC TGG ATG ACC CAG |
| 5 | Vκ2a forward | GAT ATT GTG ATG ACC CAG |
| 6 | Vκ2b forward | GAT RTT GTG ATG ACT CAG |
| 7 | Vκ3a forward | GAA ATT GTG TTG ACR CAG |
| 8 | Vκ3b forward | GAA ATA GTG ATG ACG CAG |
| 9 | Vκ3c forward | GAA ATT GTA ATG ACA CAG |
| 10 | Vκ4a forward | GAC ATC GTG ATG ACC CAG |
| 11 | Vκ4b'forward | GAT ATT GTG ATG ACC CAC ACT CC |
| 12 | Vκ5a forward | GAA ACG ACA CTC ACG CAG |
| 13 | Vκ6a forward | GAA ATT GTG CTG ACT CAG |
| 14 | Vκ6b forward | GAT GTT GTG ATG ACA CAG |
| 15 | Cκ1' reverse | ACA CTC TCC CCT GTT GAA GCT C |

**Table 2: List of oligonucleotide primers for PCR amplification of human immuno-globuline light λ chains.**

| # | **Name of primer, direction** | **Nucleotide sequence (5'-3')** |
|---|---|---|
| 1 | Vλ1a' forward | CAG TCT GTG CTG ACT CAG CCA CC |
| 2 | Vλ1b' forward | CAG TCT GTG YTG ACG CAG CCG CC |
| 3 | Vλ1c' forward | CAG TCT GTC GTG ACG CAG CCG CC |
| 4 | Vλ2 forward | CAG TCT GCC CTG ACT CAG |
| 5 | Vλ3a forward | TCC TAT GWG CTG ACT CAG |
| 6 | Vλ3b forward | TCC TAT GAG CTG ACA CAG |
| 7 | Vλ3c forward | TCT TCT GAG CTG ACT CAG |
| 8 | Vλ3d forward | TCC TAT GAG CTG ATG CAG |
| 9 | Vλ4 forward | CAG CYT GTG CTG ACT CAA |
| 10 | Vλ5 forward | CAG SCT GTG CTG ACT CAG |
| 11 | Vλ6 forward | AAT TTT ATG CTG ACT CAG |
| 12 | Vλ7 forward | CAG RCT GTG GTG ACT CAG |
| 13 | Vλ8 forward | CAG ACT GTG GTG ACC CAG |
| 14 | Vλ4/9 forward | CWG CCT GTG CTG ACT CAG |
| 15 | Vλ10 forward | CAG GCA GGG CTG ACT CAG |
| 16 | Cλ2' reverse | TGA ACA TTC TGT AGG GGC CAC TG |
| 17 | Cλ7' reverse | AGA GCA TTC TGC AGG GGC CAC TG |

**Table 3: List of oligonucleotide primers for PCR amplification of human immunoglobuline heavy chains (IgM, IgG, IgA).**

| # | **Name of primer, direction** | **Nucleotide sequence (5'-3')** |
|---|---|---|
| 1 | VH1aM forward | CAG GTK CAG CTG GTG CAG TCT GG |
| 2 | VH1bM forward | CAG GTC CAG CTT GTG CAG TCT GG |
| 3 | VH1cM forward | SAG GTC CAG CTG GTA CAG TCT GG |
| 4 | VH1dM forward | CAR ATG CAG CTG GTG CAG TCT GG |
| 5 | VH2aM forward | CAG ATC ACC TTG AAG GAG TCT GGT C |
| 6 | VH2bM forward | CAG GTC ACC TTG ARG GAG TCT GG |
| 7 | VH3aM forward | GAR GTG CAG CTG GTG GAG TCT G |
| 8 | VH3bM forward | CAG GTG CAG CTG GTG GAG TCT G |
| 9 | VH3cM forward | GAG GTG CAG CTG TTG GAG TCT G |
| 10 | VH3dM forward | GAG GTG CAG CTG GTG GAG WCY G |
| 11 | VH4aM forward | CAG STG CAG CTG CAG GAG TCS G |
| 12 | VH4bM forward | CAG GTG CAG CTA CAG CAG TGG G |
| 13 | VH5b' forward | GAR GTG CAG CTG GTG CAG TCT GG |
| 14 | VH6a'M forward | CAG GTA CAG CTG CAG CAG TCA GG |
| 15 | VH7aM forward | CAG GTG CAG CTG GTG CAA TCT GG |
| 16 | IgM'M reverse | TGG AAG AGG CAC GTT CTT TTC TTT GTT G |
| 17 | IgG1'M1 reverse | CTT GTC CAC CTT GGT GTT GCT GG |
| 18 | IgA reverse | GCA GGG CAC AGT CAC ATC CTG G |

In a next step a linking of two DNA-fragments of light and heavy chains obtained in i) into one construct comprising a nucleic acid encoding the variable region of an IgG light chain which is associated to a nucleic acid encoding a variable region of an IgG heavy chain, to allow expression of a polypeptide comprising the variable regions ScFv of a light and heavy IgG chains, respectively.

According to a preferred embodiment, to obtain a specific linkage between a DNA fragments encoding a variable light and heavy chain an amount of sample obtained in step i) may be aliquoted, e.g. in two parts, and optionally diluted. The said DNA fragments, either prepared of cDNA via amplification from mRNA, cDNA or double stranded DNA derived from the cDNA, may then separately be contacted with a linker specific for the light chain or the heavy chain, such that the linker binds to the respective DNA fragments in each of the sample parts only. That is one part will have linkers for the light chain only, while the other parts will have linkers for the heavy chain only. The linkers to be used will allow hybridization under appropriate conditions to each other to result in a DNA fragment comprising a variable region of a light chain and a variable region of a heavy chain. Again, the association of the two DNA fragments will be effected such that the linkage of the two DNA fragments is in frame, so that a polypeptide will result that harbors the amino acid sequence of a variable region of the light chain and a variable region of the heavy chain. The same may be effected to obtain specifically two heavy chains and two lights chains, as desired.
In tables 4 and 5 preferred primers are listed

**Table 4: List of reverse oligonucleotide primers for secondary PCR amplification of human λ and κ light chain variable fragments.**

| **#** | **Name of primer, direction** | **Nucleotide sequence (5'-3')** |
|---|---|---|
| 1 | Jλ235 reverse | TAG GAC GGT CAG CTY GGT CCC |
| 2 | Jλ7 reverse | GAG GRC GGT CAG CTG GGT GCC |
| 3 | Jλ1 reverse | TAG GAC GGT GAC CTT GGT CCC |
| 4 | Jλ6 reverse | GAG GAC GGT CAC CTT GGT GCC |
| 5 | Jλ4 reverse | ACC TAA AAT GAT CAG CTG GGT TCC |
| 6 | Jκ2 reverse | TCG TTT GAT CTC CAG CTT GGT CCC |
| 7 | Jκ3 reverse | TCG TTT GAT ATC CAC TTT GGT CCC |
| 8 | Jκ14 reverse | TCG TTT GAT YTC CAC CTT GGT CCC |
| 9 | Jκ5 reverse | TCG TTT AAT CTC CAG TCG TGT CCC |

**Table 5: List of oligonucleotide primers for PCR amplification and assembly of human immunoglobuline light and heavy chains.**

| | **Name of primer, direction** | **Nucleotide sequence (5'-3')** |
|---|---|---|
| 1 | linkM-JH6 reverse | GCT ACC GCC ACC GCC GCT GCC ACC GCC ACC AGA ACC ACC GCC GCC TGA GGA GAC GGT GAC CGT GGT C |
| 2 | linkM-JH3 reverse | GCT ACC GCC ACC GCC GCT GCC ACC GCC ACC AGA ACC ACC GCC GCC TGA AGA GAC GGT GAC CAT TGT CC |
| 3 | linkM-JH1245 reverse | GCT ACC GCC ACC GCC GCT GCC ACC GCC ACC AGA ACC ACC GCC GCC TGA GGA GAC RGT GAC CAG GG |
| 4 | linkM-VL1a' forward | GGC GGC GGT GGT TCT GGT GGC GGT GGC AGC GGC GGT GGC GGT AGC CAG TCT GTG CTG ACT CAG CC |
| 5 | linkM-VL1b' forward | GGC GGC GGT GGT TCT GGT GGC GGT GGC AGC GGC GGT GGC GGT AGC CAG TCT GTG YTG ACG CAG CCG |
| 6 | linkM-VL1c' forward | GGC GGC GGT GGT TCT GGT GGC GGT GGC AGC GGC GGT GGC GGT AGC CAG TCT GTC GTG ACG CAG CCG |
| 7 | linkM-VL2 forward | GGC GGC GGT GGT TCT GGT GGC GGT GGC AGC GGC GGT GGC GGT AGC CAG TCT GCC CTG ACT CAG CC |
| 8 | linkM-VL3a forward | GGC GGC GGT GGT TCT GGT GGC GGT GGC AGC GGC GGT GGC GGT AGC TCC TAT GWG CTG ACT CAG C |
| 9 | linkM-VL3b forward | GGC GGC GGT GGT TCT GGT GGC GGT GGC AGC GGC GGT GGC GGT AGC TCC TAT GAG CTG ACA CAG C |
| 10 | linkM-VL3c forward | GGC GGC GGT GGT TCT GGT GGC GGT GGC AGC GGC GGT GGC GGT AGC TCT TCT GAG CTG ACT CAG GAC |
| 11 | linkM-VL3d forward | GGC GGC GGT GGT TCT GGT GGC GGT GGC AGC GGC GGT GGC GGT AGC TCC TAT GAG CTG ATG CAG |
| 12 | linkM-VL4 forward | GGC GGC GGT GGT TCT GGT GGC GGT GGC AGC GGC GGT GGC GGT AGC CAG CYT GTG CTG ACT CAA TC |
| 13 | linkM-VL5 forward | GGC GGC GGT GGT TCT GGT GGC GGT GGC AGC GGC GGT GGC GGT AGC CAG SCT GTG CTG ACT CAG CC |
| 14 | linkM-VL6 forward | GGC GGC GGT GGT TCT GGT GGC GGT GGC AGC GGC GGT GGC GGT AGC AAT TTT ATG CTG ACT CAG |
| 15 | linkM-VL7 forward | GGC GGC GGT GGT TCT GGT GGC GGT GGC AGC GGC GGT GGC GGT AGC CAG RCT GTG GTG ACT CAG GAG |
| 16 | linkM-VL8 forward | GGC GGC GGT GGT TCT GGT GGC GGT GGC AGC GGC GGT GGC GGT AGC CAG ACT GTG GTG ACC CAG GAG |
| 17 | linkM-VL4/9 forward | GGC GGC GGT GGT TCT GGT GGC GGT GGC AGC GGC GGT GGC GGT AGC CWG CCT GTG CTG ACT CAG CC |
| 18 | linkM-VL10 forward | GGC GGC GGT GGT TCT GGT GGC GGT GGC AGC GGC GGT GGC GGT AGC CAG GCA GGG CTG ACT CAG CC |
| 19 | linkM-Vk1a forward | GGC GGC GGT GGT TCT GGT GGC GGT GGC AGC GGC GGT GGC GGT AGC RAC ATC CAG ATG ACC CAG TCT C |
| 20 | linkM-Vk1b forward | GGC GGC GGT GGT TCT GGT GGC GGT GGC AGC GGC GGT GGC GGT AGC GMC ATC CAG TTG ACC CAG TCT C |
| 21 | linkM-Vk1c forward | GGC GGC GGT GGT TCT GGT GGC GGT GGC AGC GGC GGT GGC GGT AGC GCC ATC CRG ATG ACC CAG TCT C |
| 22 | linkM-Vk1d forward | GGC GGC GGT GGT TCT GGT GGC GGT GGC AGC GGC GGT GGC GGT AGC GTC ATC TGG ATG ACC CAG TCT C |
| 23 | linkM-Vk2a forward | GGC GGC GGT GGT TCT GGT GGC GGT GGC AGC GGC GGT GGC GGT AGC GAT ATT GTG ATG ACC CAG ACT C |
| 24 | linkM-Vk2b forward | GGC GGC GGT GGT TCT GGT GGC GGT GGC AGC GGC GGT GGC GGT AGC GAT RTT GTG ATG ACT CAG TCT C |
| 25 | linkM-Vk3a forward | GGC GGC GGT GGT TCT GGT GGC GGT GGC AGC GGC GGT GGC GGT AGC GAA ATT GTG TTG ACR CAG TCT C |
| 26 | linkM-Vk3b forward | GGC GGC GGT GGT TCT GGT GGC GGT GGC AGC GGC GGT GGC GGT AGC GAA ATA GTG ATG ACG CAG TCT C |
| 27 | linkM-Vk3c forward | GGC GGC GGT GGT TCT GGT GGC GGT GGC AGC GGC GGT GGC GGT AGC GAA ATT GTA ATG ACA CAG TCT C |
| 28 | linkM-Vk4a forward | GGC GGC GGT GGT TCT GGT GGC GGT GGC AGC GGC GGT GGC GGT AGC GAC ATC GTG ATG ACC CAG TCT C |
| 29 | linkM-Vk4b' forward | GGC GGC GGT GGT TCT GGT GGC GGT GGC AGC GGC GGT GGC GGT AGC GAT ATT GTG ATG ACC CAC ACT CC |
| 30 | linkM-Vk5a forward | GGC GGC GGT GGT TCT GGT GGC GGT GGC AGC GGC GGT GGC GGT AGC GAA ACG ACA CTC ACG CAG TCT C |
| 31 | linkM-Vk6a forward | GGC GGC GGT GGT TCT GGT GGC GGT GGC AGC GGC GGT GGC GGT AGC GAA ATT GTG CTG ACT CAG TCT C |
| 32 | linkM-Vk6b forward | GGC GGC GGT GGT TCT GGT GGC GGT GGC AGC GGC GGT GGC GGT AGC GAT GTT GTG ATG ACA CAG TCT C |

In a more preferred embodiment of the present invention linker fragments, encoding ((Gly)₄Ser)₃ polypeptide linker, are added to the nucleic acid sequences encoding variable heavy and light chains of immunoglobulines. The linker parts of heavy and light chains anneal to each other and prime a fill-in reaction in the presence of a TaqSE DNA Polymerase, such as for example TaqSE DNA Polymerase. Finally the heavy and light chains are assembled into a single gene using their DNA linker fragment parts.

Proceeding accordingly enables to obtain a vast number of antibodies artificially created by a randomly linking of nucleic acids encoding a variable region of an immunoglobuline light or heavy chain with a nucleic acid encoding a variable region of another immunoglobuline light or heavy chain, respectively, also comprising combinations of light and heavy chains for building up antigen binding sites not being present in the originally obtained RNA-pool. As could be shown, using already naturally pre-formed parts of antigen binding sites on the variable region of the immunglobulines and combining those in a random manner also antibodies may be produced showing an enhanced and constant binding affinity to HIV proteins as compared to antibodies naturally produced in individuals infected with HIV.

Additionally restriction sites may be introduced into the DNA-fragments thus obtained, which are useful in subsequent applications, such as e.g. cloning steps. In principle any suitable restriction site may be used according to the requirements, while it is within the knowledge of the skilled person to CHO-Klose appropriate ones. Restriction sites may be introduced by any suitable method known in the art, such as e.g. using oligonucleotide primers comprising a nucleic acid sequence for a restriction site or using adapter molecules comprising a nucleic acid sequence for a restriction site combined with the 5'-and/or 3'- end, respectively.

In a preferred embodiment of the present invention *Sfi* I and *Not* I restriction sites are introduced to the ends of the linked nucleic acid fragments, which according to a preferred embodiment may comprise a light and heavy chain nucleic acid sequence, wherein the restriction sites are used for further cloning steps into cloning vectors. *Sfi* I and *Not* I restriction sites are added to the 5'- and 3'-ends of said linked fragments (ScFv gene), respectively. These particular restriction sites occur with very low frequency in antibody genes and allow most of the obtained linked fragments, e.g. comprising the light and heavy chain nucleic acid sequence, to be cloned as a single *Sfi* I/*Not* I fragment. In a more preferred embodiment of the present invention *Sfi* I and *Not* I restriction sites are introduced via oligonucleotide primers. Preferred *Sfi* I-site- and *Not* I-site- comprising oligonucleotide primers used are designed on basis of primer sequences from the article [21]. Primers useful for introducing *Sfi* I and *Not* I restriction sites at the ends of the obtained linked fragment comprising of the light and heavy chain nucleic acid sequence are shown in table 6.

**Table 6: List of oligonucleotides primers for introduction of Sfi I and Not I restriction sites into the ends of the assembled scFv gene.**

| # | **Name of primer, direction** | **Nucleotide sequence (5'-3')** |
|---|---|---|
| 1 | JK2-NotI | TTC TCG ACT TGC GGC CGC TCG TTT GAT CTC CAG CTT GGT CCC |
| 2 | JK3-NotI | TTC TCG ACT TGC GGC CGC TCG TTT GAT ATC CAC TTT GGT CCC |
| 3 | JK14-NotI | TTC TCG ACT TGC GGC CGC TCG TTT GAT YTC CAC CTT GGT CCC |
| 4 | JK5-NotI | TTC TCG ACT TGC GGC CGC TCG TTT AAT CTC CAG TCG TGT CCC |
| 5 | JL235-NotI | TTC TCG ACT TGC GGC CGC TAG GAC GGT CAG CTY GGT CCC |
| 6 | JL1-NotI | TTC TCG ACT TGC GGC CGC TAG GAC GGT GAC CTT GGT CCC |
| 7. | JL7-NotI | TTC TCG ACT TGC GGC CGC GAG GRC GGT CAG CTG GGT GCC |
| 8 | JL6-NotI | TTC TCG ACT TGC GGC CGC GAG GAC GGT CAC CTT GGT GCC |
| 9 | JL4-NotI | TTC TCG ACT TGC GGC CGC ACC TAA AAT GAT CAG CTG GGT TCC |
| 10 | VH1aM-SfiI | CTC GCA ACT GCG GCC CAG CCG GCC CAG GTK CAG CTG GTG CAG TCT GG |
| 11 | VH1bM-SfiI | CTC GCA ACT GCG GCC CAG CCG GCC CAG GTC CAG CTT GTG CAG TCT GG |
| 12 | VH1cM-SfiI | CTC GCA ACT GCG GCC CAG CCG GCC SAG GTC CAG CTG GTA CAG TCT GG |
| 13 | VH1dM-SfiI | CTC GCA ACT GCG GCC CAG CCG GCC CAR ATG CAG CTG GTG CAG TCT GG |
| 14 | VH2aM-SfiI | CTC GCA ACT GCG GCC CAG CCG GCC CAG ATC ACC TTG AAG GAG TCT GGT |
| 15 | VH2bM-SfiI | CTC GCA ACT GCG GCC CAG CCG GCC CAG GTC ACC TTG ARG GAG TCT GG |
| 16 | VH3aM-SfiI | CTC GCA ACT GCG GCC CAG CCG GCC GAR GTG CAG CTG GTG GAG TCT G |
| 17 | VH3bM-SfiI | CTC GCA ACT GCG GCC CAG CCG GCC CAG GTG CAG CTG GTG GAG TCT G |
| 18 | VH3cM-SfiI | CTC GCA ACT GCG GCC CAG CCG GCC GAG GTG CAG CTG TTG GAG TCT G |
| 19 | VH3dM-SfiI | CTC GCA ACT GCG GCC CAG CCG GCC GAG GTG CAG CTG GTG GAG WCY G |
| 20 | VH4aM-SfiI | CTC GCA ACT GCG GCC CAG CCG GCC CAG STG CAG CTG CAG GAG TCS G |
| 21 | VH4bM-SfiI | CTC GCA ACT GCG GCC CAG CCG GCC CAG GTG CAG CTA CAG CAG TGG G |
| 22 | VH5b'-SfiI | CTC GCA ACT GCG GCC CAG CCG GCC GAR GTG CAG CTG GTG CAG TCT GG |
| 23 | VH6a'M-SfiI | CTC GCA ACT GCG GCC CAG CCG GCC CAG GTA CAG CTG CAG CAG TCA GG |
| 24 | VH7aM-SfiI | CTC GCA ACT GCG GCC CAG CCG GCC CAG GTG CAG CTG GTG CAA TCT GG |

For cloning and expressing the obtained linked fragments comprising the light and heavy chain nucleic acid sequence any suitable cloning and/or expression vector known to one skilled in the art may be used. In a preferred embodiments phagemid vectors are used, which comprise, for example, the pCANTAB 5E. coli phagemid vector.

The phagemid pCANTAB 5E carries both the M13 and ColE1 plasmid origins of replication and, thus, can be conveniently multiplied as plasmid or alternatively packaged as recombinant M13 phage with the aid of a helper phage, such as M13KO7. *Sfi* I and *Not* I digested antibody variable region genes are cloned between the leader sequence and the main body of the M13 gene 3 in pCANTAB 5E phagemid vector. The resulting fusion protein retains the functions of both parent proteins. The g3p leader sequence directs transport of the protein to the inner membrane/periplasm of *E.coli* where the main g3p domain attaches the fusion protein to the tip of the assembling phage. pCANTAB 5E also contains an amber translational stop codon at the junction between the cloned ScFv and the sequence for the g3p. The resulting pool of pCANTAB 5E plasmid derivatives, containing scFv fragments, is used for the transformation of *sup*E strain of *E*. *coli,* such as TG1. In *sup*E *E. coli* strains translation continues through the amber stop codon in pCANTAB 5E to produce the ScFv-g3p fusion protein displayed on the phage tip.

**Table 7. Oligonucleotide primers used for reamplification of scFv fragment mixtures.**

| **Primer** | **5'-3' nucleotide sequences** |
|---|---|
| VH12467*Sfi*IReampl | TGC **GGC CCA GCC GGC** CSA G |
| VH35*Sfi*IReampl | TGC **GGC CCA GCC GGC** CGA RG |
| JL1235*Not*IReampl | GAC **TTG CGG CCG C**TA GGA CG |
| JL4*Not*IReampl | GAC **TTG CGG CCG C**AC CTA AAA TG |
| JL67*Not*IReampl | GAC **TTG CGG CCG C**GA GGR C |
| JK1234*Not*IReampl | GAC **TTG CGG CCG C**TC GTT TG |
| JK5*Not*IReampl | GAC **TTG CGG CCG C**TC GTT TAA TC |

Recognition sites for *Not*I restriction endonuclease are marked blue; recognition sites for *Sfi*I restriction endonuclease are marked green.

In non-suppressor strains, such as HB2151, the stop codon is recognized, protein synthesis is aborted at the end of the scFv gene, and the g3p fusion protein is not synthesized. In this case, the resulting ScFv protein is transported into the periplasmic space but is not assembled into a phage particle since it lacks the gene 3 domain. Rather, the soluble antibody fragment accumulates in the periplasm and upon extended incubation, leaks into the medium. Therefore, HB2151 and similar *E*. *coli* strains are used for the production of the soluble antibodies after their infection by selected antigen-positive phages and cannot be used in current application. The steps of scFv libraries creation are presented in Example 1.

### 2. The Enrichment of Recombinant Phagemid Library presenting HIV-Specific Antibody's scfv fragments (biopanning)

Expression of antibodies may be obtained in suitable hosts to obtain polypeptides capable to bind antigen and the polypeptides thus obtained with recombinant gp120-, gp 41- and native HIV-polypeptides isolated from different donors. Thereby, it may be advantageous, however not necessary, when the expressed polypeptide is presented on the host's surface. Suitable hosts for expression of antibodies include viral systems, prokaryotic and eukaryotic cells and/or cell cultures.

In a preferred embodiment said antibody's fragments are expressed in bacteriophages M13 creating a phage display library, which enables display of a huge number of different constructs each represented by different phage for use in phage display technology. The phage display approach is a powerful tool for cloning immunoglobulin genes and for expressing and detecting functional antibodies. It allows obtaining variable heavy and light chain fragments of antibodies as fusion proteins displayed on the phage surface as a pool or library of HIV-specific antibodies without stage of monoclonal antibodies selection. This approach makes it possible to quickly find antibodies to any antigen and to produce, in case of need, soluble variants thereof with and/or without glycosylation in other expression systems.

Phagemid library panning is an in vitro technique which allows to screen a large number of clones very quickly, wherein phages presenting antibodies on their surface showing a binding affinity to selected HIV polypeptides may be identified and used for maintaining the recombinant phagemid and producing new phages for further screening step. Phage-presenting antibodies library may be analyzed for the binding affinity with cross cycles of SDS-PAGE, Western blot and ELISA screening in the art to identify antigen-positive clones.

Since the displayed ScFv antibody fragments retain their antigen-binding capability, it is thus possible to enrich for recombinant phage expressing specific antibodies by affinity selection. With this approach, antibodies of defined specificity and affinity are quickly selected from a population. The obtained antibody genes library is being screened to improve antigen binding ability. This step of technology is called panning and comprises that phages presenting HIV-specific ScFv fragments are subsequently bind and collected with
i) recombinant gp120-, gp140-, gp160-, gp41 HIV-1 subtype A and B env peptides
ii) native HIV *env* polypeptides isolated from different HIV-infected donors.

In a preferred embodiment since phage library is selected that show a binding affinity to all of the polypeptides listed above the number of displayed antibodies decreases from 10⁷ - 10¹² to 10² - 10³. Due to independent cycles of contacting said polypeptides with specific recombinant HIV polypeptides, wherein the sequence of those polypeptides are (i) known and are (ii) constant, and with native HIV-polypeptides isolated from different donors, wherein in these polypeptides mutations may have occurred, it was possible to select antibodies, which bind to essentially all of HIV mutants known, indicating, that the antibodies may recognize essentially constant conformations on said HIV polypeptides.

Proceeding accordingly makes it possible to derive HIV-specific recombinant antibodies library presented on phage's surface, and being selected from the huge pool of ones from infected individuals they exhibit a binding affinity to selected HIV polypeptides, even if mutations have occurred in these polypeptides, with two different methods:
i) Standard biopanning procedure [4]
ii) Embedding at nitrocellulose membrane with solid state immobilized native HIV peptides.

According to the first method i) for recombinant phage production 4 x 10¹⁰ pfu of M13KO7 helper phage was added to prepared log phase transformed TG1 E. coli culture for 1 hour pre-incubation and 12 hours incubation in presence of 100 µg/ml Ampicillin and 5µg/ml Kanamycin at 37 °C stirring 250 rpm (the typical phage yield is 101° to 10¹¹ ampicillin-transducing units per ml). Polypropylene tubes are recommended since phage may adsorb nonspecifically to other plastic surface.

Then PEG precipitation was performed. Bacterial culture is spinned at 1000g for 10 min., supernatant collected and cooled. 1/5 v/v cool solution 20%PEG/ 2,5MNaCl is added to supernatant and incubated at 0°C for 60 min., then spin at 10000g in a Beckman JA-20 rotor for 20 minutes at 4°C. Discard the supernatant. The pellet (which may not be easily visible) is resuspended in 16 ml of 2x YT medium with 0.01% timerosal. We recommend the supernatant to be filtered through a 0.45 µm filter if it will be stored (at 4°C). The solution containing recombinant phage is used for panning.

PEG precipitation and cycles of phage panning should be performed as soon as possible following rescue since some phage-displayed recombinant antibody preparations may be unstable. Log phase TG1 cells colony from a minimal medium plate was transferred to 5 ml of 2x YT medium and incubated overnight at 37°C with shaking at 250 rpm. Then 10 ml of fresh 2x YT medium with 100 µl of the overnight culture was inoculated and incubated at 37°C with shaking at 250 rpm until the culture reaches an A600 of 0.3.

A 25 cm2 tissue culture flask is coated with 5 ml of antigen diluted to 10 µg/ml in an appropriate buffer, e.g. PBS or 0.05M Na₂CO₃ (pH 9.6). Coating with antigen may be performed for 1-2 hours at room temperature or overnight at 4°C. The conditions for coating the plate, i.e. buffer and incubation temperature and time, depend on the antigen and should be similar to the immunoassay conditions used for the original polyclonal or monoclonal antibody from which the new recombinant was derived. The coating concentration of the antigen can be varied depending on the affinity (antigen-binding capability) of the recombinant phage antibody desired. Less amount of antigen is required for high affinity antibodies than for those with low affinity. However, solution-based selection may be preferable to solid-phase selection for isolating antibodies with specific affinities since the amount of antigen used in the selection can be more accurately controlled.

The flask is washed three times with PBS, emptying it completely after each wash. Then the flask is filled completely with blocking buffer to block any remaining sites on the flask surface and incubated at room temperature for 1 hour. The flask is washed again three times with PBS, emptying it completely after each wash.

Blocking buffer containing 0.01% thimerosal or 0.01% sodium azide as a preservative is freshly prepared. 16 ml of PEG-precipitated recombinant phage is diluted with 14 ml of blocking buffer (which contains a preservative) and incubated at room temperature for 10-15 minutes. Non-specific, hydrophobic protein-protein interactions may occur between native M13 phage proteins and some antigens during the panning step. This interaction can be reduced if Triton X-100 is added to the diluted phage supernatant to a final concentration of 0.1%. Alternatively an elution specific bind phage by glycine or trypsine solution can be performed. 20 ml of the diluted recombinant phage are pored into the flask and incubated for 2 hours at 37°C. Then the flask is emptied and washed 20 times with 30-50 ml of PBS and 20 times with PBS containing 0.1% Tween 20 (a wash bottle works well for dispensing the wash solutions). The flask is emptied completely each time.

The entire 10 ml of log-phase TG1 cells (see step 1) are added to the flask or panning vessel and incubated at 37°C for 1 hour. After 1 hour, 100 µl of the 10 ml cell suspension are removed. From them tenfold dilutions of the cell suspension in 2x YT medium (1:10, 1:100, 1:1000) are prepared. 100 µl of undiluted cells and 100 µl of each dilution are placed onto separate SOBAG plates using a sterile glass spreader. When dry, the plates should be inverted and incubated overnight at 30°C. If the colonies are too small to pick after incubation, the plate can be left at 30°C for an additional 4-8 hours. The SOBAG plates can be handled as follows: a) Scrape the cells from the plate to generate stock cultures. Flood the plate with 5 ml of 2x YT medium and scrape the cells into the medium with a sterile glass spreader. Add glycerol to a final concentration of 15-30% and store at - 70°C. b) Seal the plates and store for up to 2 weeks at 4°C for rescue at a later time

According to a second method, modified from [25] ii) the mixture of native HIV peptides is run on a 10% SDS-PAGE gel, followed by electrotransfer onto nitrocellulose membrane in Western transfer buffer (25 mM Tris, 193 mM glycine, and 20% methanol). The location of the antigen is determined by staining the membrane with either Ponceaus red or Coomassie brilliant blue. A 7 * 30-mm2 section of membrane containing the protein band is excised and blocked by incubation with 10% porcine gelatin, 5 * 10¹¹ CFU/ml helper phage at 4°C overnight. After blocking, the membrane is transferred to the binding buffer (5% gelatin, 3 * 10¹¹ CFU/ml helper phage, 0.5 M NaCl) and 10¹² CFU of scFv phagemid antibody library added. Phage library is incubated with membrane at 4°C for 4 h with gentle rocking. The membrane is washed six times with PBS, 0.1 % Tween 20 (100-ml volume for each wash) and six times with PBS (100-ml volume for each wash). Alternatively, the spots are washed three times with PBS containing 0.1% Tween 20 (PBST) for 5 min, five times with 10% MPBS containing 25% glycerol for 20 min, and finally three times with PBS for 5 min. Membrane containing the protein band is excised with a razor blade and phages are eluted with 100 mM TEA at RT for 10 min. After neutralization, eluted phage particles are incubated with a gelatin-blotted membrane or gelatin-coated immunotube at RT for 30 min. The supernatant is then used to infect TG1. Phage is prepared from *E*. *coli* for the next round of selection as previously described.

### 3. Multiplication of viral material isolates of antiretroviral therapy naïve patients with PBMC-MT method

It is known that HIV can successfully multiply in CD4-CCR5-CXCR4 receptors enriched cell cultures, however in practice this method has many limitations. First, infection titer for native viral material from patients or laboratory strains for in vitro infection never comes for more than 1-2 percent of total virus concentration measured with different methods (RealTime RT PCR, p24 ELISA, etc.). It means, for example, that if number of virus copies in infection material is 10⁵ the initial number of copies which researcher will be able to analyze from in vitro multiplication is 10³ only, the rest 10² original HIV possible variations will be lost for analysis. Second, the number of HIV variants passed in vitro infection selection is in the best cases several sequence variants from original 10³, therefore laboratory viral strains never represent the real situation with HIV genetic and peptides variability. And third, HIV from treated with HAART or other antiretroviral therapy patients looses ability to multiply in vitro, therefore resistant HIV variants cannot be cultivated in vitro. Second, our experience of native virus cultivation proves that the best results are obtained when:
i) Lymphocytes of HIV-infected patients are incubated with healthy donor's lymphocytes isolated from heparinized fresh blood using Ficoll-paque solution as described [19]. Worth to mention that for HIV-1 subtype A widespread in the territory of Russian Federation in most cases infection is successful if HIV-infected lymphocytes are incubated with monocytes isolated from healthy donor's fresh blood as described [19].
ii) MT-2 or MT-4 or any other cell line being used for the incubation with HIV (CCR5F-CEM, PM-1, HeLa, U937, etc.) prepared in concentration 0,25x10⁶/ml has been co-cultivating then with equal number of HIV-propagated monocytes harvested twice by adding RPMI-1640 medium to a total volume of 50 ml and spinning at 425g for 10 min. Cell mixture is re-suspend in CL medium with addition IL-2 10 µl/ml and incubated at 37°C in an upright position in 25 cm² tissue culture flasks. Virus-containing medium is being collected every 3-4^{th} day by taking out the half of culture medium replacing it with the same volume of a fresh medium (RPMI + 10 % FCS).

Effectiveness of viral infection activity is controlled with microscopic analysis of cell death and syncytium formation and also p24 ELISA test. Harvested culture medium was cleared from cells with spinning at 3000 rpm (1000g) for 15 minutes and stored at - 80°C.

### 4. Concentration of HIV pellets (by ultrafiltration, ultracentrifugation), virus inactivation and disruption

Stock solution containing about 20 %/weight of viral particles is produced from blood plasma or culture supernatant. First supernatant is run spinning at 3000 rpm (1000g) for 15 minutes, then the obtained supernatant is run spinning at 13200 rpm (16000g) for next 15 minutes. About half of total sample volume of 20% sucrose is stratified to the bottom of ultracentrifuge tubes (the density of the sucrose solution is 1,16-1,18 g/sm³), then supernatant containing retroviral particles is pored above into the tube. Tubes were spinned at 38000 rpm MLS-50 rotor Optima MAX, Beckmann (160000g) during 1 hour 35 minutes [19]. The pellet is dissolved in small volume of culture media (for example, RPMI 1640).

### Inactivation of HIV

### Lysis of HIV pellets and obtaining HIV proteins

The first method is performed according to the described in [1]. Composition of HIV lysis buffer (radioimmunoprecipitation buffer) includes 20 mM Tris-Cl, pH 8.0, 120mM NaCl, 2mM EDTA, 0.5% Deoxycholate, 0.5% NP-40, 2µg PMSF, 10µg\ml apoprotein, 10µg\ml pepstatin A. After adding detergents mix gently on magnetic stirrer with low heating (50° C)

The second method is standard for preparation of peptide's mixtures for masspectrometry and crystal analysis. pH of Obtained HIV-1 protein mixture was adjusted to 2.5 with 2N HCl and incubated with 0.15% (wt/vol) porcine pepsin (Sigma Chemical Co., St Louis, MO) for 4 h at 37°C. Hydrolysis was stopped with heating to 80°C for 15 min, then pH adjusted to 7.5-8 with addition of 2M NaOH. Then hydrolyzed protein mixture was run through ultrafiltration with 10 kDa hydrolysis membrane and pepsin with the rest of non-hydrolysed proteins were removed. Filtered hydrolyzed protein mixture was lyophilized and stored at -80°C.

### 5. Collecting the native HIV-1 env peptides with HIV-specific recombinant ScFv presenting phagemid library with reverse panning technique.

The approaches to exploit phage display technique for vaccine development using antigen phage presented libraries is vaguely expressed in [35]. Before starting the procedure of recombinant phage ScFv libraries column embedding the M13 presented libraries are checked for specificity with modified Western blotting method. Probes are run on a gradient SDS-PAGE followed by electrotransfer onto nitrocellulose [25]. The antigen spots are first soaked in PBS containing 1% Tween 20 for 1 h for renaturation of the blotted proteins. The membranes are further blocked with 4% gelatin solution in PBS at 37C for 2 h and incubated with 10¹² CFU/ml of phages (preincubated for 30 min at RT with 1.5% BSA in 4% gelatin solution) at RT for 1 h. Then membranes are being washed three times with PBS, 0.1% Tween 20 and three times with PBS, phage binding is detected with incubation with a 1:8000 dilution of HRP-conjugated anti-M13 in 5% skimmed milk/PBS at RT for 1 h. After washing three times with PBS/0.1% Tween 20 and three times with PBS, the bands are visualized with ECL detection (Amersham). After extensive washing with TPBS-blot, the membranes are incubated for 1 min in ECL reagens. Each membrane is subsequently incubated with a Hyperfilm-ECL and developed.

Recombinant mAbs usually show about 10-30 percent of affinity compared to native antibodies isolated from the organism. However created with phage display technique panel of individual for each cohort of virus variants (patients) HIV-specific mAbs (phagemid library) is sufficient for selection of the majority of HIV env and other peptides and proteins for development of anti-HIV-1 immunogenic compositions (Fig 7 a,b, 8 a,b). For phagemid library presenting recombinant phage production M13KO7 helper phage is added to overnight TG1 E. coli culture for 1 hour pre-incubation and 12 hours incubation in presence of 100 µg/ml ampicillin and 50 µg/ml kanamycin at 37 °C (the typical phage yield is 10¹⁰ to 10¹¹ ampicillin-transducing units per ml). The culture is spinned at 1000g for 10 min., supernatant was collected and cooled. Then 1/5 v/v of PEG8000/NaCl (20%PEG/ 2,5MNaCl) solution is added to supernatant and incubated 1 hour at ice, then precipitation performed with spinning 10000g at 4°C for 20 minutes. The pellet is dissolved in LB or 10MM TrisHCl pH 8.0 and filtered through 0.45µm. Recombinant phage can be stored at 4°C if 0.01% timerosal is added.
i) Embedding at supermacroporous monolithic epoxy-activated cryogel (Protista Biotechnology) chromatography columns with immobilized M13-specific mAbs. M13-specific mAbs are embeded at the supermacroporous monolithic epoxy-activated cryogel (Protista Biotechnology). For that the dry sorbent is re-suspended in 0.1 M NaHCO3 pH 8.3 containing 0.5 M NaCl buffer. M13-specific mAbs are dissolved with the same buffer to concentration 10 mg/ml, added to the sorbent and incubated 1 hour at room temperature with mechanical stirring. After incubation the sorbent is washed with 5 volumes of the same 0.1 M NaHCO3 pH 8.3 / 0.5 M NaCl buffer. For non-specific reactive groups blockage the sorbent is incubated with 0.1M Tris-HCl buffer, pH 8.0 or 1 M ethanolamine, pH 8.0 for 2 hours at room temperature, then adjusted into 5ml chromatography columns.

For both methods first phage M13 particles specific to gp120, gp140, gp160 and their fragments, gp41, p24 were incubated at 37°C for 40 min. with hydrolyzed HIV-1 peptides mixture obtained as described above (stage 4). Then phage particles were embedded with help of immobilized M13 phage-specific antibodies either at:

Prepared supermacroporous monolithic epoxy-activated cryogel column with embedded M13-specific mAbs is balanced with 0.05 M Tris-HCl, pH 8.0 buffer, then recombinant M13 in the same buffer is adjusted for 5 hours with speed 0.5 ml/min using liquid chromatography system ActaPrime Plus (GE Healthcare). Then the column is washed with 5 volumes of the same 0.05 M Tris-HCl, pH 8.0 buffer.

Recombinant phage embedding is studied with scanning probe microscopy method (atomic force microscopy). The cryogel with successfully embedded phage HIV-specific ScFv library is presented at Fig. 6b, control supermacroporous monolithic epoxy-activated cryogel column structure is shown at Fig. 6a.

Hydrolyzed in 0.05 M Tris-HCl, pH 8.0 buffer HIV-1 peptides mixture is pored at embedded affine column for 5 hours with speed 0.5 ml/min. Then the column is washed with 5 volumes of the same 0.05 M Tris-HCl, pH 8.0 buffer.

The phage which binds HIV peptides was eluted with 0.1M glycine pH 2.2 gradient. Obtained fractions are incubated in glycine elution buffer with presence of 0.001M PMSF for 5 hours at room temperature until phage-antigen complexes are re-adjusted completely.

The HIV peptides were analyzed [2] and purified using high performance liquid chromatography (HPLC, Waters). Analytical reversed-phase HPLC was performed on a Waters 1525 HPLC system equipped with a Symmetry C 18 column (5 µm, 4.6 mm x 150 mm, flow rate 0.5 ml/min). Preparative reversed-phase HPLC is performed on Waters 1525 HPLC system using Symetry C-18 columns (10 µm, 5.0 cm x 25 cm) and a Waters UV detector. Linear gradients of acetonitrile in water/0.1% trifluoroacetic acid (TFA) were used to elute bound peptides.

### 6. Quantitative and sequence analysis of env peptides variability and frequency with Liquid Chromatograph Mass Spectrometry (LC-MS) method;

Native HIV-1 peptides were collected as a source of samples from reverse panning HIV-specific phage library. Quantitative selection, mass distribution and characterization of *env* peptides were performed with mono-dimensional Liquid Chromatography-Mass Spectrometry (LC-MS-MS analysis).

The protein stripes from SDS-PAGE gels looking similar to [10] and single spots from 2D that were not identified as peptide mass fingerprint were analyzed with ion electrospray quadrupole mass analyzer trap method using Esquire 6000plus instrument (Bruker Daltonics, Bremen, Germany). The samples acquisition was provided from Low Pressure Chromatography system Ultimate LC Packing and samples selector Famos LC Packing (Dionex, CA, USA) on-line regime. The chromatography part consists of two consequently connected columns with an electromagnetic valve between them. The first column (100µm x3sm) with hydrophobic polymer phase Poros R2, large pore's diameter, analogous C₈, is used for samples concentration and desalination. The second column (75 µm x25sm) with Phenomex sorbent, grain size 5µm, pore diameter 300A, analogous C₁₈, is used for separation of desalinated mixture of triptic peptides. The conditions for chromatography separation are as follows: 200µl/min with actual exaust velocity 900nl/min before splitter and 200 nl/min during separation. Linear gradient from 5% to 60% solution B (75% acetonitryl, 25% isopropanol, 0.1% formic acid) is running for peptides separation for 48 minutes.

All measurements are taken between 300-2500 m/z with trap mass optimization equal 700. Ions with charge number equal 2 or higher and intensity higher than the threshold are taken for tandem experiments. The obtained mass-prints are being sent into MASCOT search system. The search is run through proteomics database, results are verified with software complex Scaffold 01-07-00 (http://www.proteomesoftware.com) for peptides identification confirmation. Peptides with identification expectancy more than 95% are listing in the final schedule. All observed peptide masses matched with the calculated average masses within 0.5 Da.

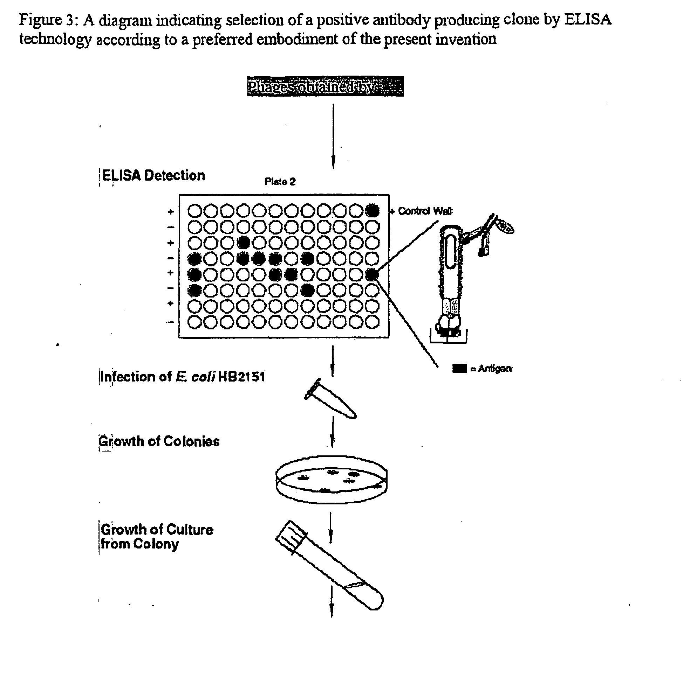

The charts represent distributions of hydrophobic (axis Y positive indexes) and hydrophilic (negative indexes) fragments of polypeptide sequence in env protein molecule (Fig. 3).
onf: Confidence (0=low, 9=high)
Pred: Predicted secondary structure (H=helix, E=strand, C=coil)
AA: Target sequence

### Hydrophobic aminoacids relative indexes :

Ala: 1.800 Arg: -4.500 Asn: -3.500 Asp: -3.500 Cys: 2.500 Gln: -3.500 Glu: -3.500 Gly: -0.400 His: -3.200 Ile: 4.500 Leu: 3.800 Lys: -3.900 Met: 1.900 Phe: 2.800 Pro: -1.600 Ser: -0.800 Thr: -0.700 Trp: -0.900 Tyr: -1.300 Val: 4.200 Asx: -3.500 Glx: -3.500 Xaa: -0.490
a) #A1.RU.03.03RU20_06_13_AY500393 
gp120 inner domain v1 v2
CKAAENLWVTVYYGVPVWRDAETTLFCASDAKAYDKEVHNVWATHACVPTDPNPQEIALE
NVTEKFDMWKNNMVEQMQTDIISLWDQSLKPCVKLTPLCVTLNCAEPNSTSSNNNSVNSN
SSDSVFEEMKNCTFNMTTELRDKRKTVHSLFYKLDIVSTGSNGSGQYRLINCNTSAMTQA
CPKVTFEPIPIHYCAPAGFAILKCKDTNFTGTGPCKNVSTVQCTHGIKPV
 
gp120 outer domain    v3  v4  v5
VSTQLLLNGSLAEKEVMXRSENITDNGKXIIVQLTEPVNITCIRPGNNTRTSIRIGPGQT
FYATXDVIGDIRKAYCXVSRAAWXSTLQKISTQLRKYFNNKTIXFKNSSGGDLEVTTHSF
NCGGEFFYCNTTDLFNSTWDGXGTXTXITXANGT-
ITLPCRIKQIINMWQRVGQAMYAPPIKGSIRCESNITGLXLTRDGGGTNXXXETFRPIG
GN
   gp120 inner domain
      GGNMRDNWRSELYKYKVVKIEPIGVAPTRAKRRVVEREKR
b) B.RU.04.04RU128005_AY682547 
gp120 inner domain v1 v2
CSAAGNLWVTVYYGVPVWKEADTTLFCASDAKGXSTEVHNVWATHACVPTDPNPQEIDLE
NVTENFNMWQNNMVEQMHEDIISLLDQSLKPCVKLTPLCVTLNKXNMVEQMHEDIISLWD
QSLKPCVTLTPLCVTLNCTDLRNSTTDNTTNNSTEMGKEEIKNCSFNTTTNIRNKMQKEY
ALFYKLDIVPIDGDNTSYMLTSXNTSVITQACPKVSFEPIPIHYCAPAGFAILKCKENKF
NGTGPCKNVSTVQCTHGIRPV
 
gp120 outer domain    v3  v4  v5

VSTQLLLNGSLAEEEVVVRSRNFSDNAKNIIVQLKDPVQINCTRPSNNTRKSISIGPGXA
FYATGDIIGDIRXAHCNLSGADWTKTLEQIVKKLXEQYNKTIVFKQSSGGDPEIXMHSFN
CGGEFFYCNTTKLFNSTWENNSTNSTXNETGNXTITLPCRIKQIINRWQEVGKAMYAPPI
KGQIKCSSNITGLLLTRDGGSNSTNNETFRPAGGD
   gp120 inner domain
      GGDIRDNWRSELYKYKVVKIEPLGVAPTMAKRRVVQREKR
c) #B.RU.04.04RU129005 AY751406 
gp120 inner domain v1 v2
SSXAEQLWVTVYYGVPVWKEATTTLFCASDARALNTEXHNVWATHACVPTDPNPQEXLLE
NVTENFNMW[?][?][?][?][?][?][?][?][?][?][?][?][?][?][?][?][?][?][?][?][?][?][?][?]KXGEMKNCSFNITTSTRDRXKKEYALF
YKLDIVPIDDAKNSTNYRLISCNTSVLTQACPKVSFEPIPIHYCAPAGFAILKCKDKKFN
GTGPCTNVSTVXCTHGIRPV
 
gp120 outer domain v3 v4 v5
VSTQLLLNGSLAEEEVVIRSANFTNNAKTIIVQLNESXVINCTRPXNNTRKSIPIGPGRA
FYTTGDIIGDIRQAHCXLSSTKWNDTLRQIVEKLREQFGNKTIKFNQSSGGDPEIVMHSF
NCGGEFFYCNSTPLFNSTWNSSHGDSTERSNTNESTITLTCRIKQIINMWQKVGQAMYAP
PIRGQISCSSNITGLLLTRDGGANNSTTEVFRPGGGX
   **gp120 inner domain**
      GGXMRDNXRSELYKYXVVKIEPLGVXPTKAKRRVVQREKX

### 7. Cloning of major HIV env peptides and production of recombinant peptides for vaccine development in Leishmania tarentolae

HIV lifecycle is taking place in humans, monkeys or rodents and glycosylation of its proteins is closer to mammalian metabolism. Eukaryotic expression systems comprise yeast systems, filamentous fungi, but also cell cultures from insects, mammals and/or plants. Both gp120 and gp41 are highly glycosylated in their outer domains. If glycosylation of the expressed fragment or protein is desired, expression should be carried out in eukaryotic systems, for example in yeasts, mammalian cell cultures, leishmania cell cultures, baculovirus expression cultures. Expression in mammalian cells such as CHO-K1 (Chinese hamster cells) or Cos-7 (Green African monkey renal epithelium cells) is possible but as mammalian cell has millions of proteins in cell metabolism the expression of recombinant ones is rather low, and produced recombinants are difficult for chromatography isolation. Consequently we made our choice at Leishmania tarentolae as the *env* peptides production system. According to the method of the invention, the expression system is a suitable host with eukaryotic glycosylation.
After quantitative mass-spectrometry analysis gp120 variants representing the overwhelming majority in the pool are sequenced and being proceeded for cloning. As it was shown in a number of publications gp41 sequence variations are not crucial for HIV-specific immune response (Fig. 10d, Example 4). As gp41 glycosylation level and coupling to gp120 matters for eliciting HIV-specific antibodies more than its sequence variability [31] we considered to take only gene of one variant from patients cohort as a standard component for cloning. On the basis of the obtained gp120 protein sequences, the corresponding proviral DNA fragments, encoding gp120 *env* peptides genes are amplified with two-round nested PCR from patient's lymphocytes cDNA matrix using specific primer pairs (Tab. 8). Primers itself and their sets can be vary depending on results of LC-MS analysis.

It is possible to make cloning of DNA fragments encoding the whole gp120 and gp41 peptides, gp120 inner domain and gp120 outer domain, gp41 ecto- domain (please see gp120 structure Fig. 10 a,b,c). The PCR amplification scheme of HIV-1 DNA fragments, encoding gp120, gp41 and their major domains is presented on Fig. 11 a, b. The set of primers for env gp120, gp41 and their domain amplification are presented in Table 8. Restriction sites are chosen according to cloning vector variant and for *NcoI* are marked with pink, for *Xba*I - with blue, for *Not*I *-* with orange, for *Nhe*I *-* with green. What regions are the most suitable for cloning for the best immunization results in each case is rather a matter of art or experienced researchers choice.

**Table 8. Oligonucleotide primers used for amplification of HIV-1 gp120, gp41 and DNA regions, encoding their major domains.**

| Primer | 5'-3' nucleotide sequences | Amplified fragment |
|---|---|---|
| *Env* For *Nco*I | AAT **ACC ATG GAA** GCG AGG GGG ATG CAG AGG | *Env* |
| *Env* For *Xba*I | ATA **TCT AGA** GCC GCA GAA AAC TTG TGG GTC AC | *Env* |
| Env Reverse *Not*I | AT AGC GGC CGC TCA TTA TTG CAA AGC CTT TTC TGC GCC YTG TCT G | *Env* |
| *Env* Reverse *Nhe*I | ATA ATA GCT AGC TTG CAA AGC CTT TTC TGC GCC YTG TCT G | *Env* |
| gp120 For | ATA **CCA TGG** GCC GCA GAA AAC TTG TGG GTC AC | gp120 |
| gap120 Rev | AT AGC GGC CGC TCA TTA TCT TTT TTC TCT CTC CAC CAC TCT TC | |
| gp120 Rev Inner | AT AGC GGC CGC TCA TTA GCC ATT TAA CAG CAG TTG AGT TGA TAC | gp120 inner* domain |
| gp120 For Outer | ATA CCA TGG GTA GTA TCA ACT CAA CTG CTG TTA AAT GGC | gp120 outer domain |
| gp 120 Rev Outer | AT AGC GGC CGC TCA TTA CCT CAT ATT TCC TCC TAT A GG TCT G | |
| gp41 For | ATA **TCT AGA** GCA ATT GGA CTG GGA GCC GCC | gp41 |
| gp41 Rev | ATA **GCT AGC** TCA TTA TTG TAA AGC CTT TTC TRC GCC | |
| gp41 Rev Ecto | ATA GCT AGC TCA TTA TAT TTT TAT ATA CCA TAA CCA ATT TGA TAT G | gp41* ectodomain |
| V2 For | ATA **CCA TGG** ACT TTC AAC ATG ACC ACA GAA YTA AGA G | gp 120 V2 loop |
| V2 Rev | AT AGC GGC CGC GCA ATT TAT TAG TCT ATA CTG CCC ACT AC | |
| V3 For | ATA **CCA TGG** TGT ATC AGA CCT GGC AAC AAT ACA AG | gp120 V3 loop |
| V3 Rev | AT AGC GGC CGC ACA ATA TGC TTT TCT TAT GTC CCC TAT TAC | |
| V4 For | ATA **CCA TGG** TGC AAT ACA ACA GAC CTG TTC AAT AG | gp120 V4 loop |
| V4 Rev | AT **AGC GGC** CGC GCA TGG CAG AGT TAT AGT TCC ATT G | |

| | | |
|---|---|---|
| • 120 For primer was used as forward primer for amplification of gp120 inner domain; 41 For primer was used as forward primer for amplification of gp41 ectodomain. | | |

Several features are crucial for choice of expression system for recombinant proteins production for vaccine development. Their expression has to be: i) inducible; ii) similarly glycosylated or passing mammalian posttranslational modification.
i) Inducible expression is necessary for achieving a reasonable amount and concentration of recombinant peptides. As it is shown on Fig. 12 in an inducible system the expression of recombinant protein is visible on SDS-PA gel electrophoresis scans (Fig. 12a). In case cells are transfected with non-enducible expression vector usually it has to be detected with Western blotting because it is not evident in SDS-PAGE (Fig. 12b).
ii) Glycosylation of recombinant peptides produced for vaccination should match the natural typical for virus host - eukaryotic lymphocyte cells - as much as possible. To obtain any sufficient production of recombinant proteins in eukaryotic cell cultures among millions of their own proteins is difficult and too expensive. Therefore it is possible to run production of HIV-1 envelope proteins (gp120, gp41 and the entire gp160) in yeasts strain, insect cells or eukaryotic cellular parasite system. Our considered choice is trypanosomatid protozoan host *Leishmania tarentolae,* which combines eukaryotic protein expression/folding/ modification type with easy handling and is also not pathogenic to mammals. The main advantage of this expression system is the mammalian-type posttranslational modification of target proteins, such as glycosilation, phosphorylation or prenylation (Fig. 13).

The most convenient method is cloning of HIV-1 envelope proteins in the family of pLEXSY vectors from LEXSYcon2 Expression and LEXSinduce2 Expression Kits designed by Jena Bioscience GmbH. In trypanosomatid protozoa mRNAs are transcribed as polycistronic precursors which are posttranscriptionally processed into individual mRNAs by trans-splicing and polyadenylation within the intergenic regions. Regulation of protein expression in these species occurs mainly on the level of RNA and may be influenced by the structure of the intergenic regions. In pLEXSY vectors intergenic regions are used which were optimized for expression of heterologous proteins in *L*. *tarentolae* (Jena Bioscience GmbH).

The pLEXSY-2 vectors allow constitutive expression of target proteins either with or without secretory signal peptide (SP on fig. 14), following integration of the expression cassette into the chromosomal 18S rRNA locus (*ssu*). Thus, the same vector can be used for cloning of ORFs either for cytosolic or for secretory expression. The LmSAP signal peptide encoded on these vectors was derived from the gene for secreted acid phosphatase *(lmsapl)* of *Leishmania mexicana.* In-frame fusion of the ORF of a target HIV-1 protein to this signal peptide allows secretory expression in LEXSY hosts, whereas cloning into any of the restriction sites at the 5' end of the signal peptide-encoding sequence will result in cytosolic expression.

### Insertion of target gene into pLEXSY expression vector.

The pLEXSY-2 vectors allow directional insertion of the target gene cassette by replacement of a 1 kb stuffer fragment. The obtained ligation mixture is used for transformation of the competent *E*. *coli* cells which tolerate *Leishmania* sequences (Stb12, Stb14, XL-1, XL-10, SURE etc.). Selection of the recombinant *E*. *coli* clones is performed with Ampicillin. Following construction in *E*. *coli* the expression plasmid is linearized by complete digestion with *Swa*I and after that the expression cassette with the target gene is integrated into the chromosomal 18S rRNA *ssu* locus of the LEXSY host P10 by homologous recombination. There are no signals for transcription and/or translation in *E*. *coli* preceding the target gene insertion site and, thus, the lack of gene expression in *E. coli* is of advantage for generation of constructs for proteins toxic for *E. coli.*

For constitutive cytosolic or for constitutive secretory expression supported by HIV-1 envelope signal peptide HIV-1 envelope genes (gp120, gp41 and the entire *env* gene, encoding signal peptide, gp120 and gp41) are amplified with primers containg *Nco*I (for) and *NheI* (rev) sites (Table 8), digested with *Nco*I / *Nhe*I and cloned in pLEXSY-2 vectors. In such configuration the target HIV-1 protein is fused to a C-terminal His6 stretch. Otherwise, HIV-1 envelope genes are amplified with primers containg *NcoI* (for) and *NotI* (rev) sites, digested with *Nco*I / *Nhe*I and cloned in pLEXSY-2 vectors. In this case the obtained target HIV-1 protein lacks C-terminal His6 stretch.

For constitutive secretory expression ensured by LmSAP signal peptide from the pLEXSY-2 vectors HIV-1 envelope genes (gp120, gp41 and the entire *env* gene, lacking signal peptide part) are amplified with primers containg *Xba*I (for) and *Nhe*I (rev) (Table 7), digested with *Xba*I / *Nhe*I and cloned in pLEXSY-2 vectors. In such configuration the target HIV-1 protein is fused to a C-terminal His6 stretch. Otherwise, HIV-1 envelope genes are amplified with primers containg *Xba*I (for) and *Not*I (rev) sites, digested with *Xba*I / *Not*I and cloned in pLEXSY-2 vectors. In this case the obtained target HIV-1 protein lacks C-terminal His6 stretch.
Note: the *Xba*I, *Nco*I, *Nhe*I and *NotI* restriction sites are rare for HIV-1 subtype A1 *env* genes from the former SU. Map of the pLEXSY-2 vector is presented on Fig. 14. LEXSinduce2 Expression Kit contains pLEXSY_I-neo2 (encoding aminoglucoside phosphotransferase) ) and is suitable for tetracycline-inducible bacteriophage-T7polymerase-driven expression in the LEXSY host T7-TR.

### Recombinant proteins expression.

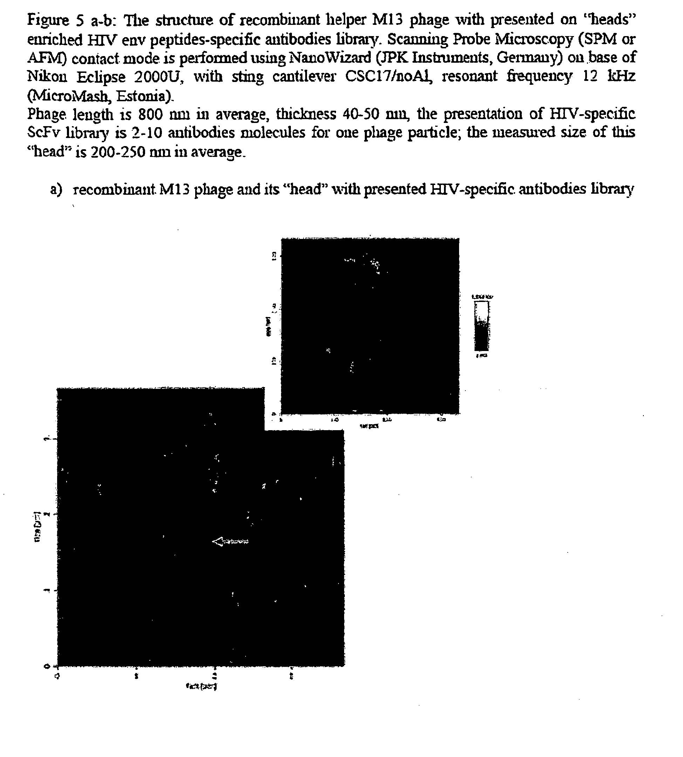

The pLEXSY_I-2 vectors allow inducible expression of target proteins either with or without secretory signal peptide. Thus, the same vector can be used for cloning of ORFs either for inducible cytosolic or for inducible secretory expression. The LmSAP signal peptide encoded on these vectors was derived from the gene for secreted acid phosphatase (lmsap1) of *Leishmania mexicana.* In-frame fusion of the ORF of a target protein to this signal peptide allows secretory expression in LEXSY hosts, whereas cloning into any of the restriction sites at the 5' end of the signal peptide-encoding sequence will result in cytosolic expression (Fig. 5). pLEXSY_I-2 vector family ensure the inducible expression of target proteins following integration of the expression cassette into the chromosomal ornithine decarboxylase (*odc*) locus of the *Leishmania tarentolae* T7-TR recipient strain, which constitutively expresses bacteriophage T7 RNA polymerase and TET repressor under the control of host RNA polymerase I. In the first cloning step the target gene is supplied with linker sequences containing restriction sites that allow insertion into the pLEXSY_I-2 vectors downstream of the T7 promoter/TET operator arrangement. These vectors contain optimized non-translated regions flanking the target gene insertion sites, which provide the splicing signals for posttranscriptional mRNA processing. Following construction in *E. coli* the expression plasmid is linearized and integrated into the *odc* locus of the LEXSY host T7-TR by homologous recombination.

For tetracycline inducible cytosolic or for tetracycline inducible secretory expression ensured by HIV-1 envelope signal peptide HIV-1 envelope genes (gp120, gp41 and the entire *env* gene, encoding signal peptide, gp120 and gp41) are amplified with primers containg *NcoI* (for) and *NheI* (rev) sites, digested with *Nco*I / *Nhe*I and cloned in pLEXSY-2 vectors. In such configuration the target HIV-1 protein is fused to a C-terminal His6 stretch. Otherwise, HIV-1 envelope genes are amplified with primers containg *NcoI* (for) and *NotI* (rev) sites, digested with *Nco*I / *Not*I and cloned in pLEXSY-2 vectors. In this case the obtained target HIV-1 protein lacks C-terminal His6 stretch.

For tetracycline inducible secretory expression ensured by LmSAP signal peptide from the vector HIV-1 envelope genes (gp120, gp41 and the entire *env* gene, lacking signal peptide part) are amplified with primers containg *Xba*I (for) and *NheI* (rev), digested with *Xba*I / *Nhe*I and cloned in pLEXSY-2 vectors. In such configuration the target HIV-1 protein is fused to a C-terminal His6 stretch. Otherwise, HIV-1 envelope genes are amplified with primers containg *XbaI* (for) and *NotI* (rev) sites, digested with *Xba*I / *Not*I and cloned in pLEXSY-2 vectors. In this case the obtained target HIV-1 protein lacks C-terminal His6 stretch.

The entire HIV-1 *env* gene, cloned in pLEXSY vectors family in *Nco*I/*Nhe*I or *Nco*I/*Not*I sites, or HIV-1 *env* gene lacking inherent signal peptide and, instead, fused with LmSAP signal peptide from pLEXSY vectors (cloned in pLEXSY vectors in XbaI/NheI or XbaI/NotI sites) can be used for creation of plasmid constructions, allowing rapid replacement of particular gp120 sequence by other gp120 sequence variants obtained from different HIV-1 viral strains. For this purpose the additional *Xba*I site is introduced by site-specific mutagenesis into the *env* gene sequence between gp120 end and gp41 start. After that the pLEXSY::HIV-1 *env* plasmid construction is digested by *Nco*I/*Xba*I (when the entire *env* gene was cloned in *Nco*I/*Nhe*I or *Nco*I/*Not*I sites) or by *XbaI* alone (when HIV-1 env lacking its inherent signal peptide was cloned in XbaI/NheI or XbaI/NotI sites) and gp120 sequence is removed. The obtained plasmid derivative is suitable for cloning of gp120 sequences, obtained from other HIV-1 viral variants by PCR amplification with primers containing *NcoI* (for) or *XbaI* (for) and *XbaI* (rev) sites.

### Cultivation of LEXSY-2 host and expression strains.

Leishmania grows in aerobic conditions in two stages: promastigote with flagella (wild types in insect host) and amastigote in vertebrate host. *In vitro* both stages in T7-TR LEXSY-2 host can be cultivated in the dark at 26°C in complex media (LEXSY BHI, or LEXSY YS) or chemically defined media (Synthetic LEXSY medium), Media is being prepared from powder LEXSY BHI 37 g/l, autoclaved (amber color) and stored up to 6 months. Before use media is supplemented with 5 µg/ml Hemin, and with 100 µg/ml Penicillin and 50 µg/ml Streptomycin to prevent bacterial infections. The media can be stored at 4°C in the dark and used within 2 weeks after supplementation. There is no need to add sera to complex media, fetal calf serum do not enhance growth of *L. tarentolae.* In case of growth inhibition of the host or LEXSY strains cells should be spinned 5 min at 2000g, resuspended carefully in fresh medium, and incubation is continued. The strain can be maintained as continuous suspension culture with regular dilutions at 1:10 to 1:50 rates. Best results are obtained with inoculations during mid-late growth phase (OD 2-3; 8x10⁷-1.4x10⁸ cells/ml). For strain maintenance it is convenient to dilute 10 ml cultures 1:20 on Monday and Friday and incubate TC flask upright. Cells viability is visible under the microscope as a motile promastigote with moving flagella; dead cells are of round or disrupted form, they don't move.

Recombinant protein expression cultivation may be performed in ventilated tissue culture (TC) flasks for suspension cultures, culture volume 10 to 200 ml or in Erlenmeyer flasks, agitated in an incubator at approx. 140 rpm, culture volume of 50 ml to 1 liter in standard bioreactors, up to 100 liter. The selection of recombinants for vector pLEXSY-neo2 is in presence of 50 µg/ml Neomycin.

The LEXSY host and LEXSY expression strains may be stored at -80°C in 20% glycerol for at least one year. ¼ of volume of autoclaved Glycerol (80%) and ¾ of volume of culture grown in LEXSY BHI* medium from mid growth phase 4 - 8 x 10⁷ cells/ml (OD 1.2 - 1.8) are added to a 15 ml Falcon tube, mixed with glycerol and distributed into sterile cryovials. Vials are kept 10 min at room temperature, then 1 hour on wet ice, at -20°C for some time and transfered to -80°C for long term storage. For the reactivation of glycerol stocks cryovials are thawed on ice, the content is pored into 10 of supplemented media and incubated in upright ventilated flasks at 26 °C in static position for 2 days until culture gets turbid.

### Preparation of the expression plasmid for LEXSY host transfection.

1 - 5 µg of expression plasmid containing the target gene obtained from E.coli is digested to completion with *Swa*I*.* Generated 2.9 kbp fragment representing the *E*. *coli* part and a larger fragment representing the linearized expression cassette with the target gene to be integrated into the chromosomal *ssu* locus of the LEXSY host are run in Agarose gel. Larger fragment expression cassette is isolated using Agarose Gel Extraction Kit. Enzymes and buffer salts may be removed with a PCR Purification Kit. Alternatively, precipitate the digest with ethanol, wash with 70% ethanol and redissolve in max. 50 µl sterile double distilled water or 10 mM Tris pH 8 per transfection.

### Transfection of the LEXSY host strain by electroporation

For efficient transfection *L. tarentolae* pre-culture is inoculated 1:20 in 10 ml LEXSY BHI medium and incubated in tissue culture (TC) flask upright @ 26°C, two days after pre-culture is diluted 1:10 in 10 ml medium and incubate overnight at the same conditions. Grown culture should contain 6 x 10⁷ cells/ml (OD 1.4 wavelength between 550 and 600 nm, 3% formalin); ensured by microscopy that the cells are vital and of droplike shape. Cells are spinned for 5 min, 2000g at room temperature and ½ volume of supernatant is removed. The pellet is resuspended in remaining medium (10⁸ cells/ml) and put on wet ice for 10 min. 0.1 - 5 µg transforming DNA in max. 50 µl water or Tris buffer is ready on wet ice in parallel tubes. 350 µl pre-chilled cells are added to the tube with DNA and transfered to the electroporation cuvette d=2 mm on wet ice avoiding air bubbles. Electroporation parameters are 450V, 450µF, pulse time 5-6 msec. After electroporation the cuvette is back on ice for exactly 10 min. Thereafter electroporated cells are transferred with capillary to 10 ml LEXSY BHI and incubated overnight 26°C (ca. 20h, OD 0.3-0.4)

### Selection of transgenic LEXSY strains

For establishment of expression strains it is possible to use routinely two methods described below in parallel. The similar expression levels are repeatedly found when comparing cultures derived from clonal or non-clonal selections following transfection with linearized expression cassettes designed for chromosomal integration. However, transfection of circular expression plasmids requires clonal selection, since the episomes tend to amplify and to eventually integrate into the genome in a heterogeneous manner. Non-clonal selection in suspension cultures following transfection with circular DNA usually resulted in reduced expression levels.

### Clonal selection by plating on solid media

LEXSY host cells are selected onto freshly prepared agar plates. 1 - 4 batches of 2 ml from the transfected 10 ml o/n culture are withdrawn, the remaining culture may be used in parallel for non-clonal selection. Cells are spinned for 5 min at 2000g and 20°C, the pellet is resuspended in 50- 100 µl residual medium, resuspended cells are spreaded onto freshly prepared LEXSY BHI agar supplemented with 50 µg/ml Neomycin with method of streaking the cells onto nitrocellulose filters placed on the surface of the agar. Plating is easier on these membranes than directly on the 1% agar, and swarming of cells is diminished. Except that, plating on membranes allows filter lifts for testing expression profiles of clonal populations e.g. by fluorescence scanning or specific detection methods for the given target protein. Plates are sealed with parafilm and incubated bottom up at 26°C.

5 - 7 days after plating small, defined colonies begin to appear, on 7 - 9 days after plating when colonies have grown up to 1 - 2 mm d they can be transferred to 0.2 ml of selective growth medium in a 96-well plate using a pipette tip, after 1 day of incubation - into 1 ml selective medium in a 24-well plate. After another 24 - 48h incubation at 26°C- the cultures are expanded into 10 ml selective medium in TC flasks and can be used for evaluation and cryoconservation.

### Selection in suspension culture

As soon as the 10 ml o/n cultures obtained from the transfection experiments (see 4.4.) start to get slightly turbid (OD₆₀₀ 0.4, ca. 10⁷ cells/ml; usually approx. 20 h after electroporation) 50 µg/ml Neomycin is added and incubation continued for 7 days at 26°C. Recombinant cells are motile under the microscope, of drop-like shape and grow as a "cloudy" suspension culture whereas the cells in the negative control begin to die during the selection period and appear as spherical or irregular forms without flagella under the microscope. Usually one consecutive transfer into fresh medium with Neomycin at 1:10 inoculation rate at 7th day of selection is enough to get a turbid culture of antibioticresistant, recombinant cell line.

### Confirmation of genomic integration and recombinant env peptides expression

Integration of the expression cassette into the ssu locus can be confirmed by diagnostic PCR or sequencing using genomic DNA of transgenic strains as template. For pLEXSY_I-2 vectors diagnostic PCR (annealing temperature 55°C) is performed with the antibiotic resistance cassette forward primer and *odc* reverse primer P 1510 (Table 9). Integration of the expression cassette into the *odc* locus will result in a characteristic fragment (1.9 or 2.0 kbp resp.), which is not observed in control reactions. In addition, you may perform diagnostic PCR (annealing temperature 60°C) with *odc* forward primer A1304 and *aprt* reverse primer A1715 (hybridizing within the 5'utr of the target gene). Integration of the expression cassette into the *odc* locus will yield a characteristic 1.1 kbp fragment not obtained in control reactions where the template is the expression plasmid or genomic DNA from the LEXSY host strain.
Expression of the target protein in recombinant LEXSY strains is evaluated by SDS-PAGE and Western blotting of cell extracts or, in case of secretory expression, aliquots from supernatants. For obtaining optimal expression it is optional to calibrate average 1 µg/ml Tetracycline induction of expression in different Tetracycline concentrations, cultivation conditions and time of harvest for each individual protein.

**Table 9: Sequences of the primers available for LEXSinduce2 kits (Jena Bioscience).**

| Primer | 5'-3' nucleotide sequences | |
|---|---|---|
| Insert sequencing forward P1442 | CCG ACT GCA ACA AGG TGT AG | all "AP" expression vectors with 5' utr aprt |
| Insert sequencing reverse A264 | CAT CTA TAG AGA AGT ACA CGT AAA AG | all LEXSI expression vectors |
| Odc forward primer A1304 | TCC GCC ATT CAT GGC TGG TG | Integration diagnostic of all odc expression vectors |
| Aprt reverse primer A1715 | TAT TCG TTG TCA GAT GGC GCA C | Integration diagnostic of all aprt expression vectors with 5'utr aprt |
| Neo forward primer A1432 | GCA TGG CGA TGC CTG CTT GC | Integration diagnostic of all odc expression vectors |
| Odc reverse primer P1510 | GTG CAC CCA TAG TAG AGG TGC | Integration diagnostic of all ssu integration vectors |

### 8. Recombinant HIV env peptides production and chromatography purification

For protein production the recombinant strain is grown in Complex LEXSY broth BHI (Jena Bioscience) to OD600D2 (10⁸ cells/ml). The protein production was induced by addition of 5mg/l tetracycline 1h after cells transfer into fresh media and the cultures were incubated at 26 °C with agitation 130 rpm in MultitronII incubator-shaker (Infors AG, Switzerland) for 24-72 h until the OD reached ca. 1.8. The presence of recombinant gp120 in the culture supernatants and in the cells was determined by polyacrylamide gel electrophoresis in the presence of dodecyl-sodium-sulfate (SDS-PAGE) and Western blotting. For confirmation of the presence of the N-glycosylation we treat the culture supernatant or the cells with N-glycosidase and analyse the electrophoretic mobility of treated protein.

The Leishmania cells expressing protein are spinned for 10 min at 2500g and the pellet is resuspended in 20mM Tris, pH 8.0, 150mM NaCl, 5mM EDTA, 1mM PMSF. The cells lysis is run using a sonicator at 2OkHz, with a 19mm probe, appying 10 one-minute pulses in ice, with 2min intervals between pulses. The clarified supernatant is collected, filtered through a 0.45µm pore membrane and used for affinity- purification of recombinant gp120 in chromatography column containing immobilized metallic ions using nitrilotriacetic acid (Ni-NTA) coupled to agarose and charged with nickel (GE Healthcare). Briefly, the Ni-NTA column is rinsed with three volumes of the buffer 20mM Tris, pH 8.0, 150mM NaCl, 5mM EDTA, 1mM PMSF with 1mL/min flow rate (LC Akta Prime Plus, GE Healthcare). The column is then charged with the filtered supernatant containing the recombinant gp120 (r-gp120) utilizing a 0.25mL/min flow rate. After charging the column is rinsed with three volumes of washing buffer (20mM Tris-HCl, 500mM NaCl, 5mM imidazol, pH 7.4). The r-gp120 is cleaved with enterokinase inside the column to remove the poly-histidine tail. To this end, one international unit (IU) of Ek is introduced into the column in a buffer containing 10mM Tris-HCl, 10mM CaCl₂, pH 8.0 and the cleavage reaction was allowed to proceed for 18h at 25 0C. Alternatively, targeted protein with the poly-histidine tail was eluted in imidazole gradient ( 0 - 0,5M Imidazole in 100mM Tris-HCl pH 8.0, containing 150 mM NaCl). Protein containing fractions are pooled and concentrated by ultrafiltration. Fractions are analyzed with SDS-PAGE/silver staining and Western blotting with anti-human gp120 antibodies. R-gp120 containing fractions are pooled, dialyzed against 0.1M Tris-EDTA buffer pH 8.0) and applied onto a anion exchange column (Q-PEEK 10um AXC Biosuite, Waters) equilibrated with the same bufferusing standard approach [2]. The protein is eluted by a gradient of 0-1M NaCl. R-gp120 containing fraction is finally polished by gel filtration using Sephacryl S-200HR (GE-Healthcare).

N-terminal sequencing of purified rhEPO is performed by automated Edman degradation. The concentration of purified protein is determined by the BCA assay. Analysis of the fractions obtained throughout the different stages of protein expression and purification is carried out by SDS-PAGE. The protein bands are visualized with Coomassie Brilliant Blue R-250 or silver staining.

### a. Preparation of HIV immunogenic composition using sterically stabilized liposomes as vehicles for delivery.

Any pharmaceutical agent for administration either per os, or subcutaneously, intramuscular, intravenously with the purpose to provide a specific immune response against some bacterial or viral infectious disease in case of possible contact with this disease in the future, in most common term "vaccine", must satisfy a number of requirements. The main of these requirements are:
iii) the immune response is highly specific towards the certain pathogenic microorganism or infection;
iv) the immune response is strong enough to fight this particular infection development blocking disease symptoms appearance;
v) the immune response lasts for a long period, months and years;
vi) in spite of all mentioned above the vaccine is not reactogenic (immunotoxic) for the human organism.

The effectiveness of the HIV immunogenic composition produced by the method according to the present invention is
enhanced by associating it with an immunostimulant or an immunogenic carrier such as an adjuvant. Gp120 carbohydrated recombinant versions as well as native HIV env proteins mixtures are highly immunogenic, not easily tolerated being inoculated subcutaneously and provide strong immune reaction by themselves (Example 4). However as for all pure proteins their biodegradation in organism is quick and immune response is being exhausted within two-three weeks in case peptides are not fixed with any preservative or protease-inhibitor adjuvant. Our first idea was to protect env peptides from degradation packing them into sterically stabilized liposomes (SSL) invisible for reticular-endothelial system. But from the very first experiments in mice it occurred that SSL are able to keep peptides enloaded or bound for some period which can be long enough and to diminish their acute immunotoxicity. For peptides sterically stabilized liposomal platform compiles the advantages of low total immunotoxicity and better pharmacodynamics (timely drug release) as it was demonstrated for liposomal drug forms of anthracyclines. Liposomal peptides can be elicited outside from SSL slowly exactly like cytostatics or other low molecular weight agents. The inhibition of immediate immune reaction makes it possible to increase the dosage of peptide for single administration and prolongate the junction contact of viral env peptides with MHC for long enough boosted immunization. This way SSL are exploited simultaneously as an immune boost adjuvant and as a delivery system.

The specific formulation of effective composition produced by the method of the present invention may thus be carried out by any suitable manner which will render the adjuvant biodegradable, safe and effective in the subject when the formulation is administered. Two of the attitudes are described further:
i) env peptides mixture is enloaded into sterically stabilized liposomes (SSL);
ii) env peptides are covalently linked to SSL's PEG-activated groups.
iii) env peptides presented on virosomes of possible constructions (pNL3-4, IRIV, etc.)

### i) Sterically stabilized liposomes preparation and peptides enloading.

Sterically stabilized liposomes are prepared using the method of vacuum drying of chloroform from mixture consisting of Phospholipid: cholesterol approximately 7:3 and 0.2-0.5 Mol/% PolyethyleneGlycol-Distearoyl(Phosphatidyl)Ethanolamine (PEG-DSPE) and vesicles formation under a nitrogen stream [40]. Lipid mixtures used are: DOPC/Chol/DSPE-PEG350, DOPC/Chol/DSPE-PEG400 and so on (Avanti Polar Lipids, Birmingham, AL). The major component of liposomes is Dioleoyl-Phosphatydylcholine (DOPC), which can be extracted from natural sources such as egg yolk, brain tissues or Soya beans or can be prepared synthetically. Cholesterol is necessary to stabilize phospholipid bilayers in liposome's membranes, PE-PEG provides the stabilization and hardness of membranes, it prevents liposomes in suspension from fusion and degradation and makes them able to store their size distribution and the agent enloaded inside without leakage for months. The ideal molecular weight for PEG in SSL is 400-700, longer PEG chains 1000-2000 is not an advantage in SSL design because hardness of liposome's membranes is getting higher than it is necessary for their content delivery and long PEG SSL compositions fail the requirement for self-biodegradation. The percentage of DSPE-PEG is the main fine tuning for obtaining liposomes of desirable characteristics.

Dry lipids are mixed in an organic solvent - chlorophorm or ethanol-chlorophorm - which is then evaporated in rotor evaporator (Buchi R-200), a thin lipid film is formed. Liposomal suspension is prepared during further hydration in an aqueous buffer with dissolved agent (for example, 50 mM NaH2PO4, 400 mM NaCl, pH 8.0), agitation 300-400 rpm and temperature +45 °C for 30 minutes. The mixture of large multilamellar (MLV, 300nm -1µm) and small unilamellar (SUV, 80-250nm) vesicles is being produced. For delivery of any water-soluble agents such as peptides the small unilamellar vesicles are necessary, therefore ultrasonication (600mV, Avanti Polar Lipids), and several cycles of filter extrusion through polycarbonate 0.4-0.2-0.1 µm membranes (Avanti Polar Lipids) are carried out. Additionally made in sterile conditions (laminar and sterile syringes, membranes and flasks) extrusion through 0.2-0.1µm membranes is a preparation ready for immunization. Liposome's size distribution and stability in aqueous suspension are determined with dynamic light scattering laser submicron particle size analysis method using DLS Nicomp-380 instrument (Fig. 17).

The mixture of recombinant peptides for immunization is being introduced in liposomal composition on the stage of hydration of lipid film - peptide's mixture is dissolved in phosphate buffer saline and becoming enloaded as internal water phase of liposomal vesicles [40]. After extrusion process SSL are being transferred through size exclusion gel filtration chromatography using Sephacryl S-200HR and Akta Prime LC system (GE Healthcare) and the excess of peptides that appeared to be outside vesicles is being separated and left in column. Then SSL suspension may be concentrated via dialysis if necessary and comprise the vaccine composition ready for immunization.

Subcutaneous administration of SSL vaccine composition may inhibit the immunization effect due to slowing down the eliciting of env recombinant peptides out of neutral to MHC liposomes. This process can be regulated using thermosensitive liposomes - tSSL. TSSL are distinguished from the others with membrane components special quantitative combination or some additional phospholipid components that make liposomal membrane able to melt as soon as temperature achieves certain degree, usually 40-45°C. At the moment of local heating termosensitive liposomes are getting destroyed and their content - peptides - is being loaded out to the tissue. For example, normal sterically stabilized liposomes have melting temperature around 54-58°C and dry weight mixture for lipid film formation consists of Phosphatydylcholine: Cholesterol:Distearoyl-Phosphatydylethanol-amine-PEG in ratio: for PC:Chol:DSPE-PEG-400 6.85:2.75:0.4 (up to 0.5) Mol/% and for longer PEG chains PC:Chol:DSPE-PEG-2000 6.9:2.95:0.15 (up to 0.25) Mol/%. To prepare thermosensitive liposomes the researcher can vari some parameters, first of all the ratio of lipids in mixture: to increase Cholesterol amount from 27-29 to 30-35 Mol/%, to decrease the percentage of PE-PEG from 2-5 Mol/% to 1.5-2 Mol/%, respectively. The other method to make liposomal membranes softer and to shift their melting point to lower temperature is to use shorter fatty acid tails of phospholipids: Dimyristoyl-Phosphatydylcholine (DMPC, C-14), Distearoyl-Phosphatydylcholine (DSPC, C-16), or rather 30-40 Mol/% DMPC or DSPC instead of equivalent part of DOPC.

### ii) env peptides coupling to SSL's PEG-activated groups

The second type liposomal carrier for env recombinant peptides lipid mixture is represented longer DSPE-PEG2000 versions activated for peptide's binding: PDP-PEG2000-DSPE/Chol/DOPC, Maleimide(Phenylbutirate)-PEG2000-DSPE/Chol/DOPC, p-Nitrophenyl (Carbonyl)-PEG2000-DSPE/ChoUDOPC. PEG-2000 concentration in these lipid mixtures should not exceed 1.5-2 Mol/% because the longer Polyethylene Glycol increases liposomes stabilization more effectively than the shorter versions and the same concentrations can make liposomal membranes too hard for vaccine lease and lipid's harmless biodegradation.

The first method of peptides conjugation with activated distal end of PEG [38] is p-Nitrophenyl(Carbonyl)-PEG-2000-DSPE reaction with peptides aminogroups in liposomal suspension in ratio 1 mg peptides for 25-40 mg lipids in 0.1M citrate buffer at pH 4.0-5.0 (total suspension volume is 5.5-9 ml). Reaction is being terminated at pH increased to 7.5-8.5 with NaOH addition and does not require any special peptides treatment.

The method with Maleimide-PEG-2000-DSPE [39] requires previous peptides thiolation with Trautt reagent (2-iminothiolane). 1 mg of ICO-25 was dissolved in borate buffer contained Na₃BO₃ and EDTA, then 50-70µg of dry Trautt reagent was added, the mixture was incubated for 1 hour at room temperature, then protein exceed was washed by ultrafiltration with simultaneous buffer exchange for PBS pH 8.0. Liposomal fractions homogenous in size and enloaded cytostatic concentrations were extracted by liquid chromatography at Sepharose CL-6B (GE Healthcare, Sweden).

ODN-HIV env peptides coupling to PDP-PEG-PE containing liposomes.
For preparation of PDP -peptide derivative peptides are dissolved in 25mM HEPES, 140mM NaCl, pH 7.4 at a concentration of 10mg/ml, then 25 mM solution of succinimidyl -4- MPB (SMPB) in DMF is slowly added to the peptide solution to the molar ratio is 20:1 (SMPB: peptide) and incubate for 30 min at room temperature. The unbound SMPB is removed at lower pH by gel filtration using Sephacryl S-200HR column (GE-Healthcare) in 25 mM HEPES, 25 mM MES, 140 mM NaCl pH 6.7 buffer. Pyridyldithio- groups on the distal ends of the PEG chains are reduced by adding dithiothreitol (DTT) to a final concentration of 20mM and incubating for 30 min at room temperature. DTT is separated in raised pH by passing the liposomes over a Sephadex G-25 column eluted with 25mM HEPES, 25mM MES, 140 mM NaCl, pH 6.7. Thiolated liposomes are incubated overnight at room temperature with MPB-peptide derivative at peptide\lipid ratio of approx. 1:1000. Unbound peptides are removed by gel filtration passing liposomes through Sephacryl S-200HR column with 25mM HEPES, 140mM NaCl pH 7.4.

ODN-HIV env peptides coupling to COOH-PEG-PE-liposomes.
To 300ul of the suspension of HO2C-PEG-PE - containing liposomes in MES buffer pH 4-5.5 (total 3 umol lipids) add 120 ul of a 0.25 M solution of 1-Ethyl-3-(3-Dimethylaminopropyl)Carbodiimide and 120 ul of 0.25 M N-hydroxysulfosuccinimide in water. The mixture is incubated for 10 min at room temperature and neutralized to pH 7.5 with 1M NaOH. 15µM of the HIV env ppeptides is added to the activated liposomes and the reaction mixture is incubated for 8h at 4°C with gentle stirring. Peptides-bound liposomes are separated from unbound peptides on Sephacryl S-200HR column (GE Healthcare) pre-equilibrated with PBS. Peak fractions of peptides-bound liposomes eluted in the void volume are being collected, pooled and if necessary diluted to the required volume with saline.

It is also possible to bind env peptides to nickel-modified phospholipids settled between PC and PEG-DSPE tails in liposomal composition DOPC/DOGS-NTA-Ni/Chol/DSPE-PEG-2000. However in spite DOGS-NTA are known to stimulate mucosal and other B-lymphocytes immune response the hidden under PEG position of env peptides weakens its anti-HIV specificity. The method is described below.

In the conjugation reaction recombinant (His)6-peptide (10-80 ug) is incubated with liposomes (1 µM) in a total volume of 50 µl phosphate buffer (50 mM NaH₂PO4, 400 mM NaCl, pH8) at 37 °C or at room temperature for 30 minutes under rotary shaking [17]. Protein conjugation to liposomes is quantified indirectly by measuring the amount of free protein at the end of the conjugation reaction. Unbound protein is separated using the Microcon-100 centrifuge device. Before centrifugation, the liposome-peptide mixture is diluted to a final volume of 250µl in phosphate buffer. After centrifugation for 13 min at 12,000 g, 20 ul of the filtrate is assayed for free protein content using the micro-BCA assay. The amount of peptides bound to liposomes is determined by subtracting the amount of free protein from the total amount used. This indirect quantification method of His- protein binding to liposomes is compared with and yielded the same result as the direct method where liposome-bound peptide was directly quantified using the micro-BCA assay after separation of free protein by size exclusion gel filtration chromatography in Sephacryl S-200HR gel matrix (GE Healthcare).

### iii) env peptides presented on virosomes of possible constructions (pNL3-4, IRIV, etc.)

Small viruses and vectors constructed of them is possible to use for expression and presentation of env peptides HIV vaccine. Virosomes are less likely appropriate and HIV-specific immune boost effect is in any case lower than what SSL technology of delivery offers. The disclosed immunogenic composition comprises only HIV env peptides expressed on small viruses surface - virosomes - and not genes of env peptides delivered in viral vectors. Large viruses like adenoviruses, adenoassociated, vaccinia viruses are not good as virosome's candidates because they have hundreds of peptides expressed on their capside, and immune response they boost after administration is more non-specific than specific. Virosome vectors include defect HIV derivative pNL3-4, influenza vector IRIV proved efficacy with malaria and hepatitis A vaccines, measles virus derivatives, alphaviruses of different encephalitis pathogens, yellow fever virus vectors and the other possible variants.

The host animals to which the adjuvant and adjuvant-containing vaccine compositions of the present invention can be usefully administered include primates as well as rodents or the other mammals. BalbC mice were used for first immune response boost validation. Two types of liposomal adjuvant enloading can be used separately or mixed together in different proportions.

3-weeks-old BalbC mice are immunized subcutaneously in doses 20-50 µg of pure peptides for the animal, adjuvant concentration in suspension for dry lipids MW is 5 mg/ml. 7-8 mice or more are taken in each group. The immunization is carried out for the animals which started to eat hard food and weighting 11-14 g the first time at 3 weeks old mice, the second time 2 weeks after when they are 5 weeks old, the third time after 1 month when mice are 9 weeks old. Recombinant gp120 and its domains and recombinant gp41 and its ectodomain are being used for completing compositions separately or together. The titer of HIV env peptides antibodies is being measured with ELISA at r-gp120 (gp110, gp160) variants, at r-gp41 that were used previously for phagemid libraries biopanning, and also at native HIV protein mixtures. ELISA tests are being done at the 3^{rd}, 14^{th} and *28^{th}* days after the last subcutaneous administration. Some results are presented in Example 4.

Two main conclusions can be drawn out from the experiments with mice:
1. gp120 and all its derivatives, recombinant as well as native peptides, are highly immunogenic and elicit strong, HIV-specific and elongated immune response being inoculated subcutaneously in BalbC mice. Recombinant gp41 and its ectodomain variants inoculated the same way elicit several times lower titer of specific monoclonal and polyclonal antibodies. The same situation in Ab titer is observed in HIV-infected people blood serum and in presentation of Ab libraries on phage M13. In patients this situation is provided with inside position of gp41 under gp120 in virus envelop. However the same phenomena with recombinant proteins immunization proves our position that for HIV immunogenic composition development correct identification of gp120 sequences and its recombinant representation is important, and gp41 should be used as a composite peptide "material" but its variations in sequence are not of much importance.
2. Liposomal adjuvant compositions are able to provide the immune response boost for the longer period than only peptides immunization and simultaneously can diminish the immediate immunotoxicity reaction to allow peptides doses increase for HIV-response development.

The dose rate and suitable dosage forms for the immunogenic compositions obtainable by the method of the present invention may be readily determined by those of ordinary skill in the art without undue experimentation, by use of conventional antibody titer determination techniques and conventional bioefficacy/biocompatibility protocols, and depending on the particular type of adjuvant, the desired therapeutic effect, and the desired time span of bioactivity. The administration may include parenteral methods, such as subcutaneous injection, transcutaneous, transdermal, intranasal and intramuscular administration.

The disclosed immunogenic composition is a step on the way to individualized medicine in terms of its activity against infection spreading from HIV variants of cohorts of infected people whose HIV-antibodies libraries were run for its selection and creation. This composition cannot work as universal weapon against HIV infection spreading as a single once developed composition. However all HIV epidemiology knowledge collected for 25 years of HIV researches and fighting AIDS will bring a lot of support to it's practical development.

### EXAMPLES

### Example 1

The electrophoresis results of Figure 18 (a)-(h) illustrate the stages of the creation of human recombinant IgG phagemid library containing HIV-specific ScFv antibody fragments. Bands of interest, excised from the gel, are marked with arrows.

### Example 2

Figure 19 (a)-(c) illustrate the specific quantification results for recombinant HIV-specific libraries

### Example 3

The HIV Env peptides are variable.

HIV is distinguished from other pathogenic viruses with its extremely high heterogeneity of peptide sequences. 3D structure of altered in sequence peptides is getting different too and in many cases these alterations are caused by the same mutations following appearence of resistant phenotypes of virus. Therefore it is possible to collect frequently met variants using monoclanal antibodies library and to obtain recombinant forms of surface viral proteins. Some common variations of HIV env peptides sequences for subtypes A and B are presented bellow. Variable aminoacids are marked with blue color, conservative ones - with red. Several of the sequences were done in our laboratory previously.

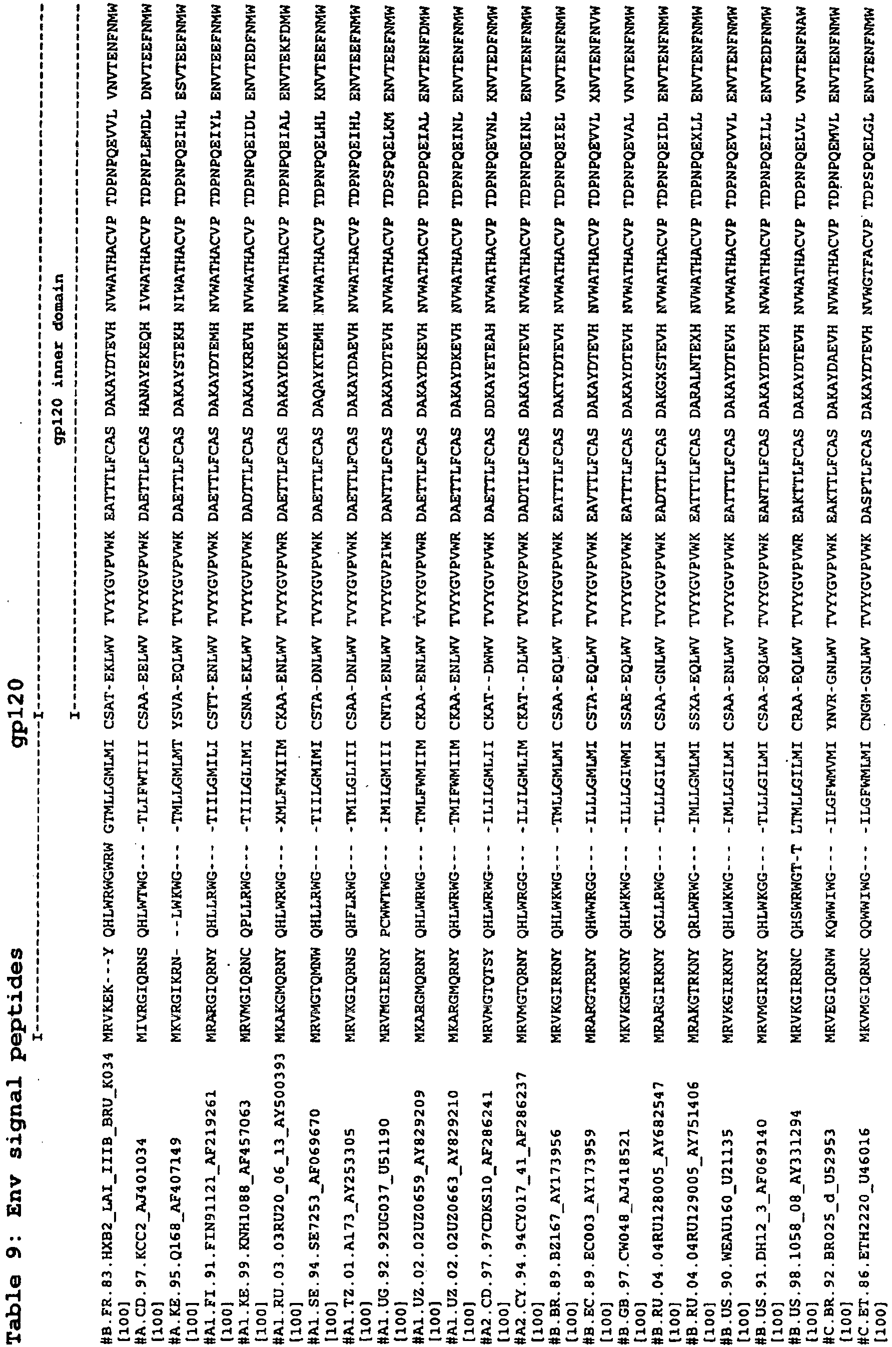

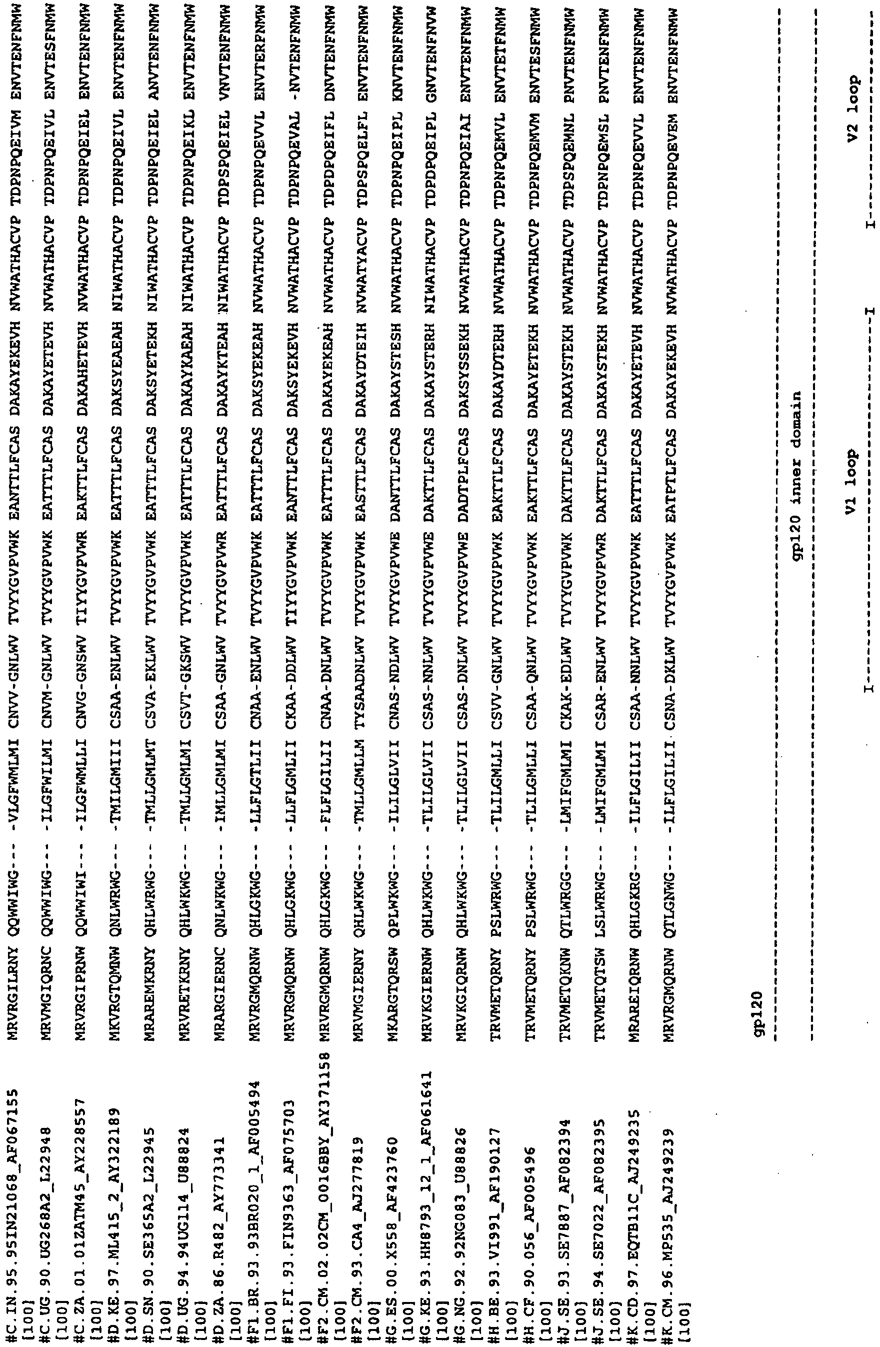

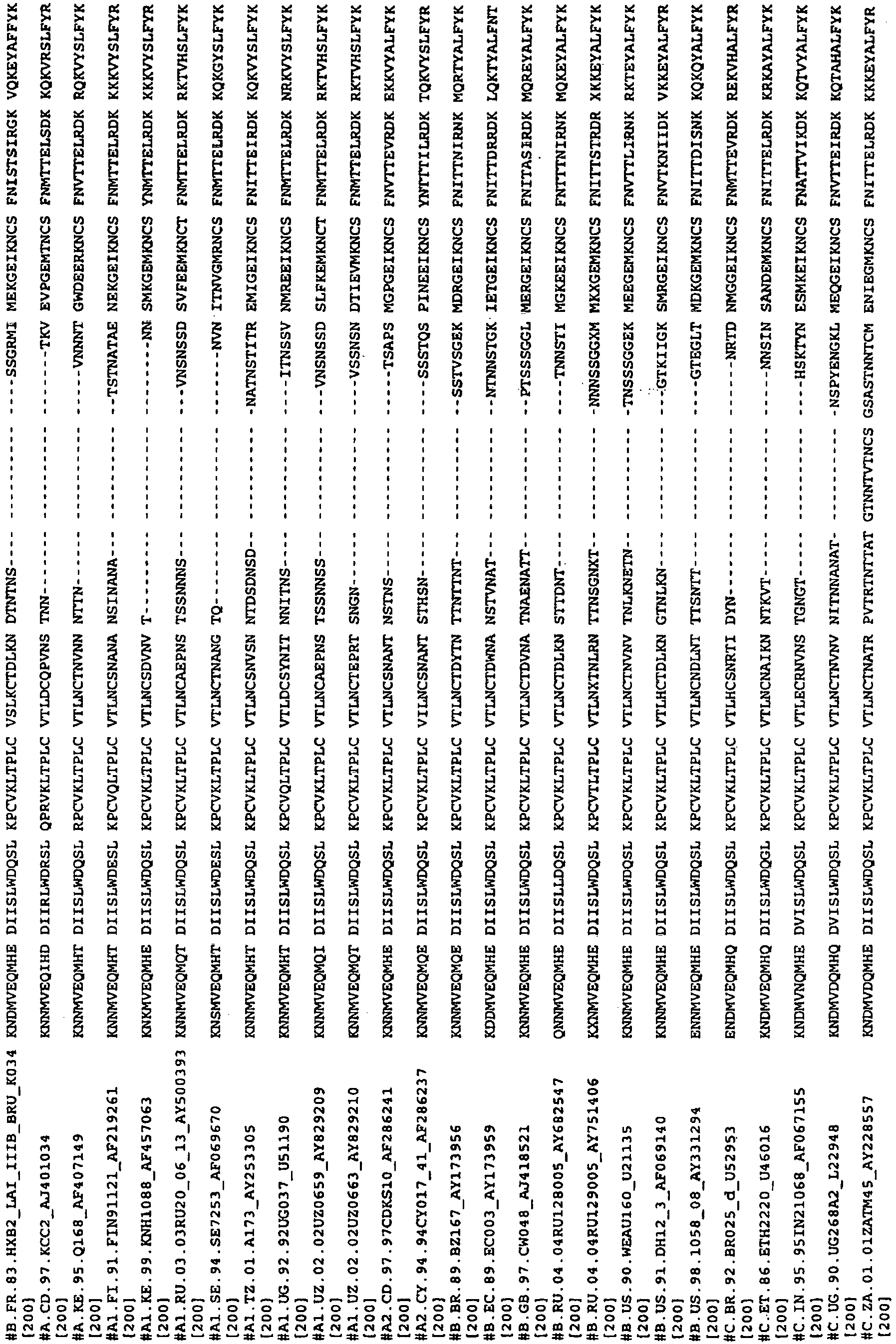

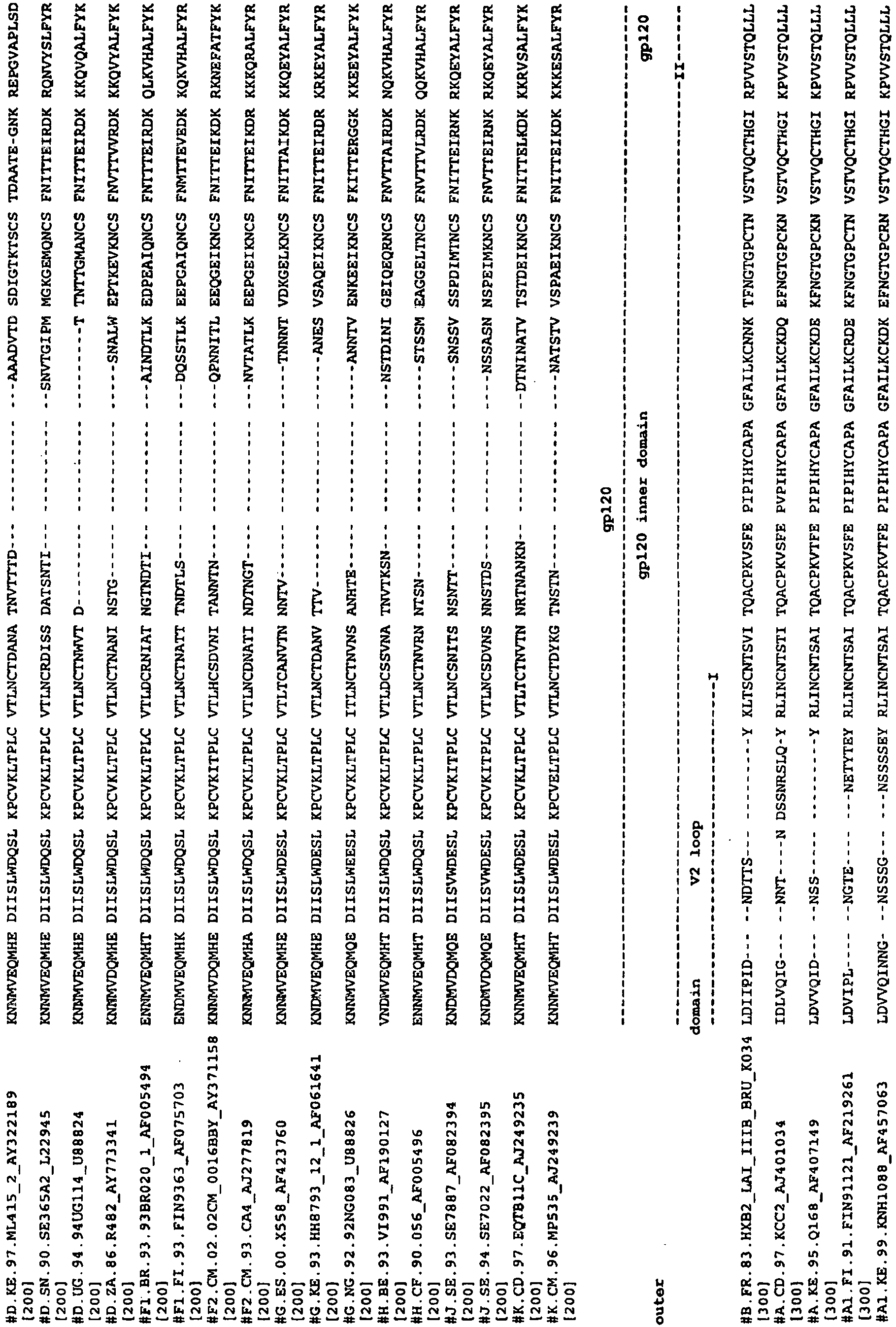

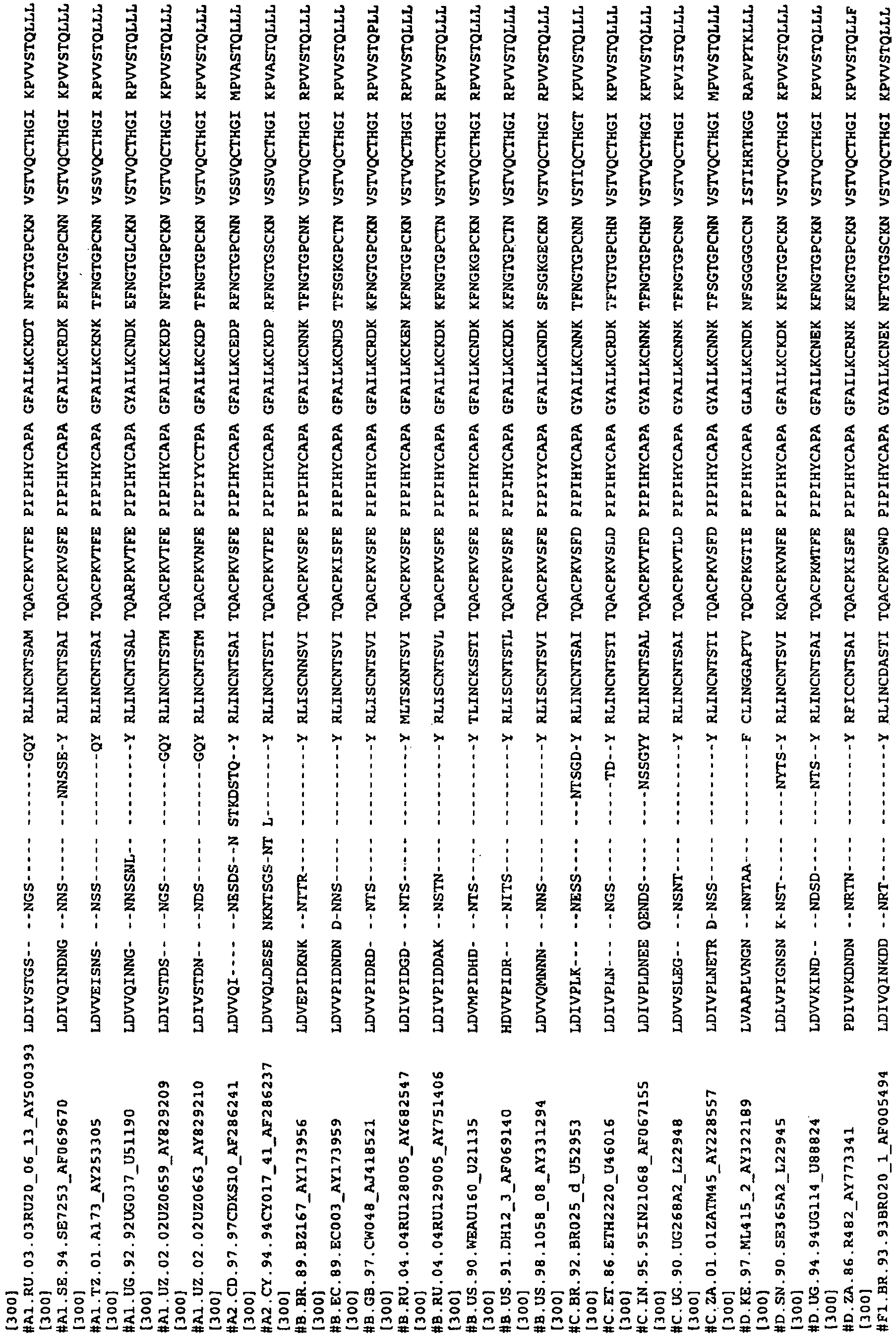

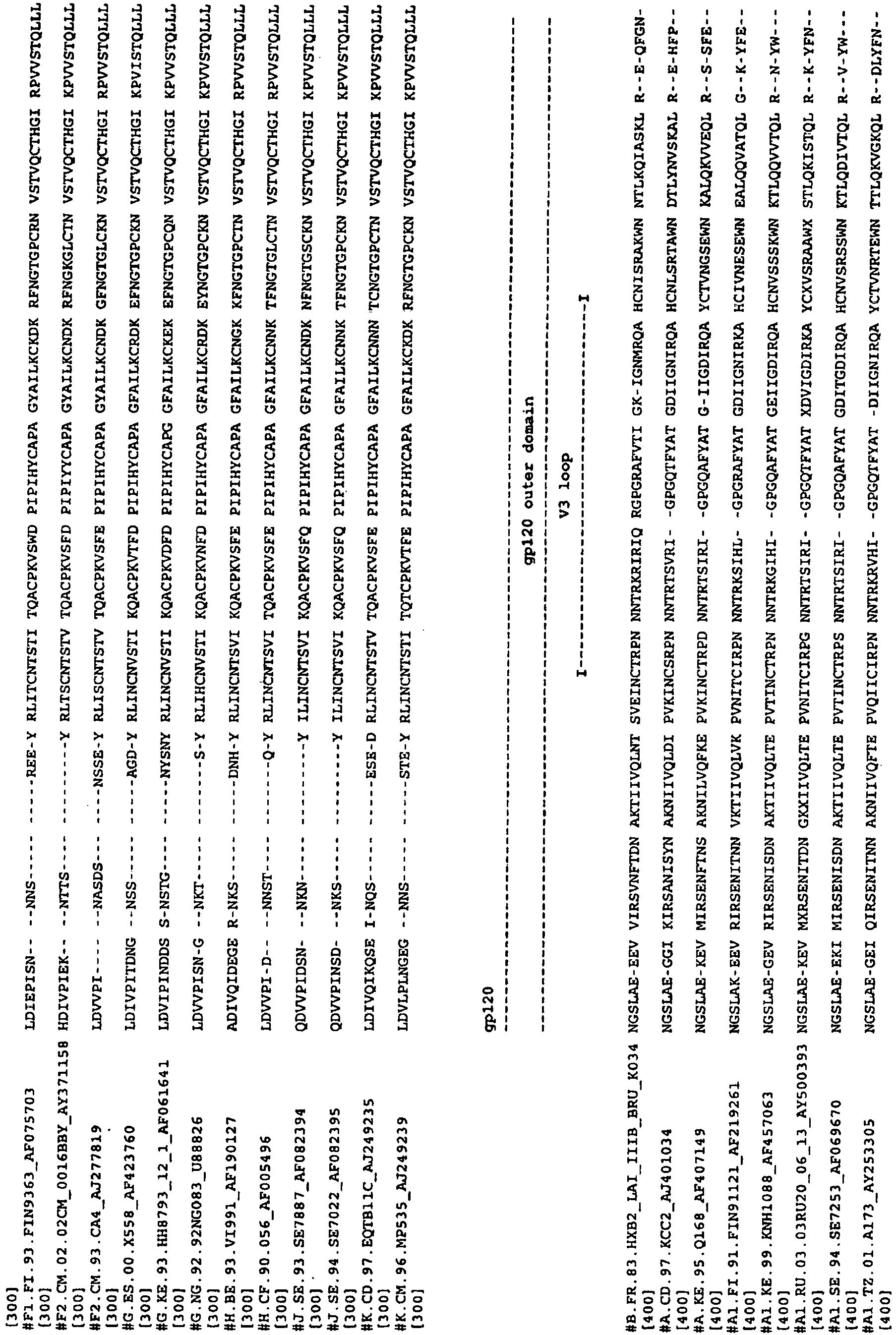

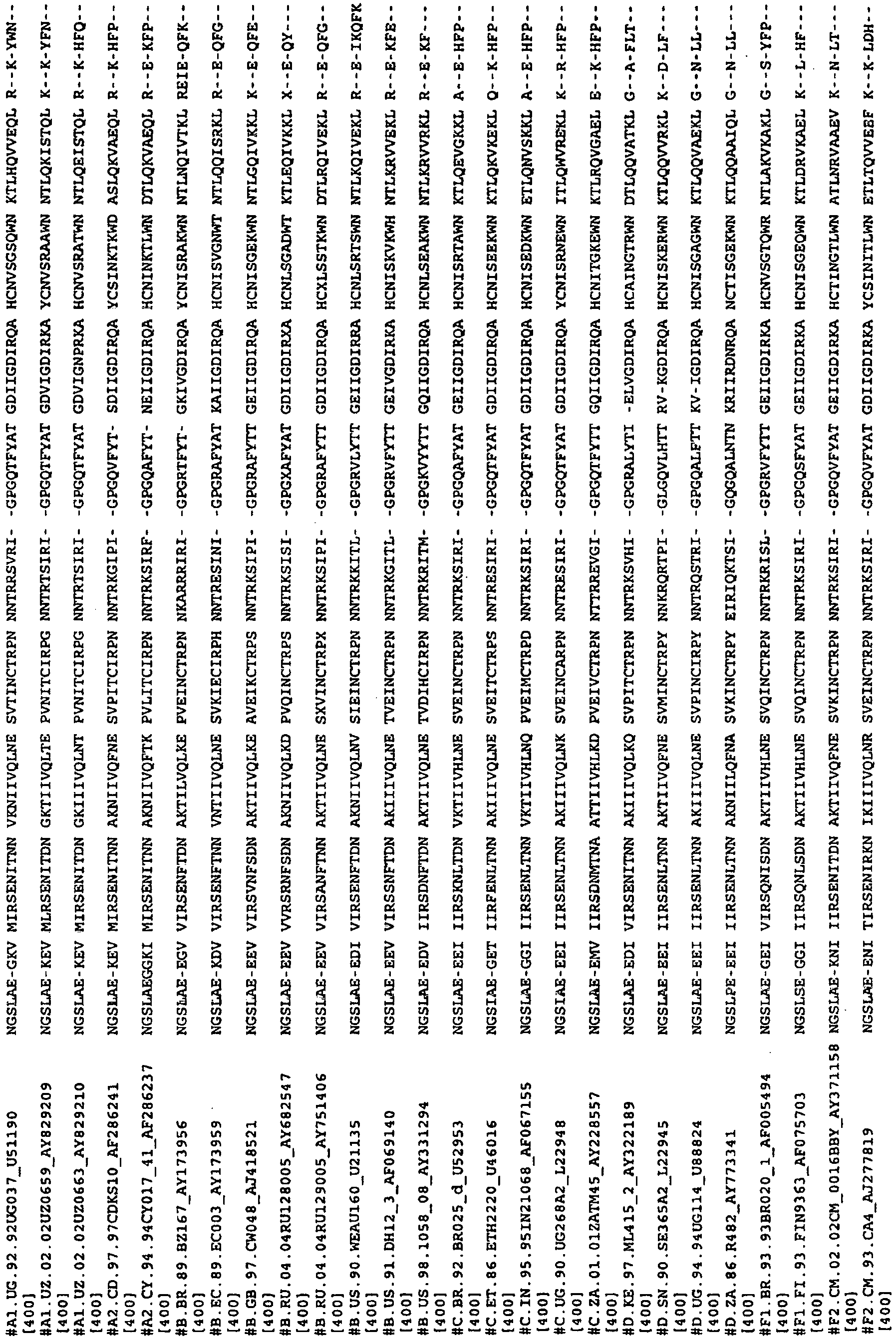

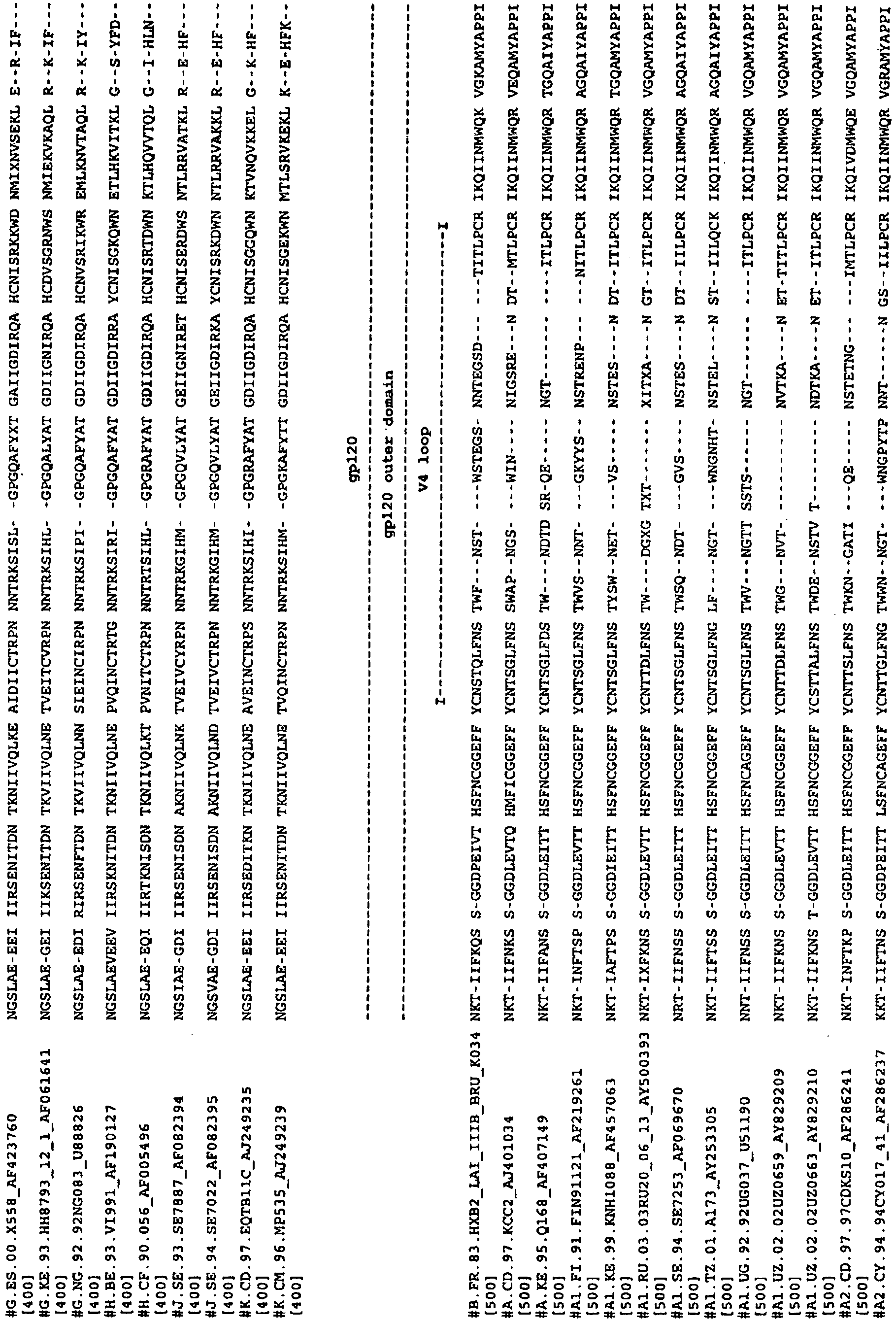

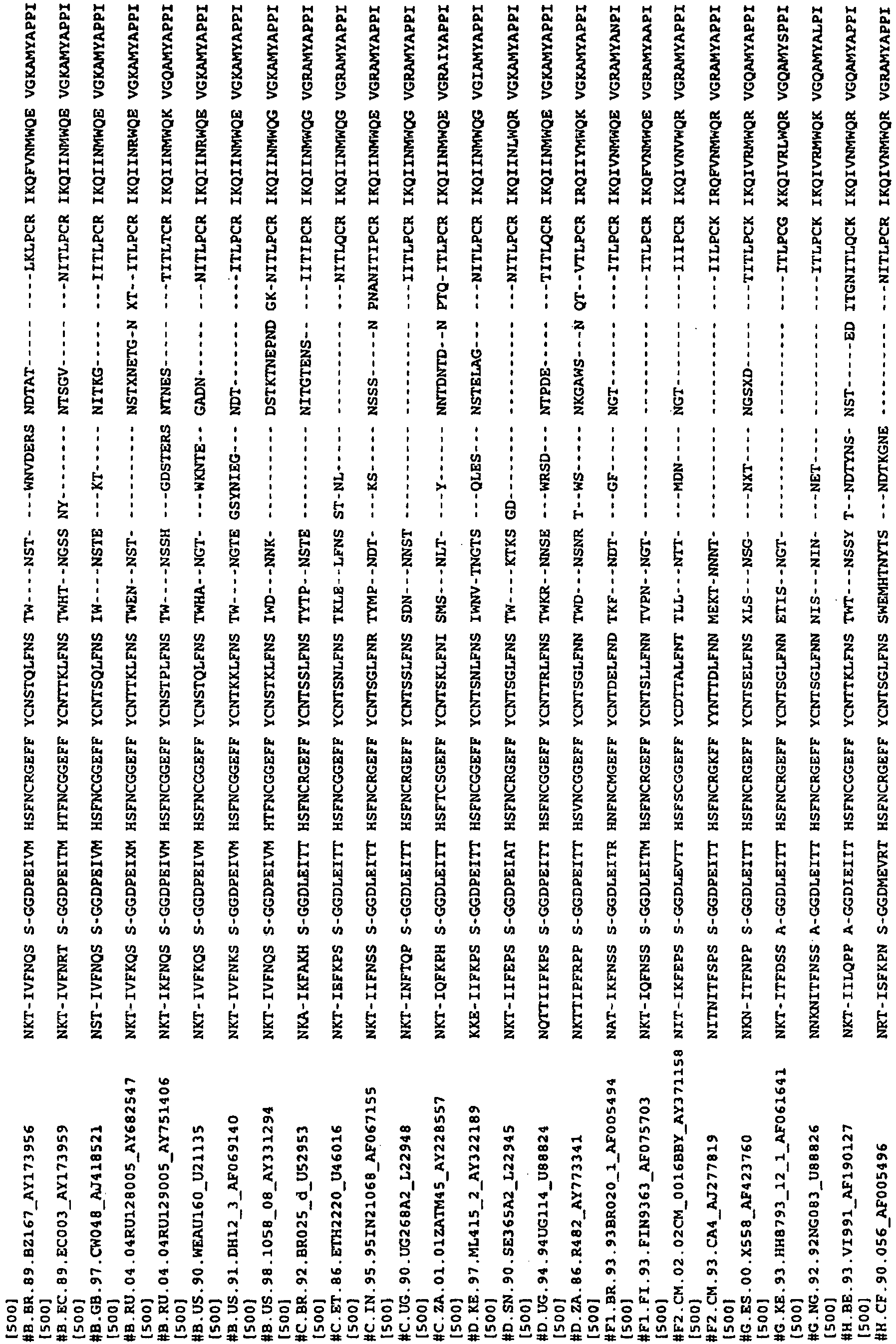

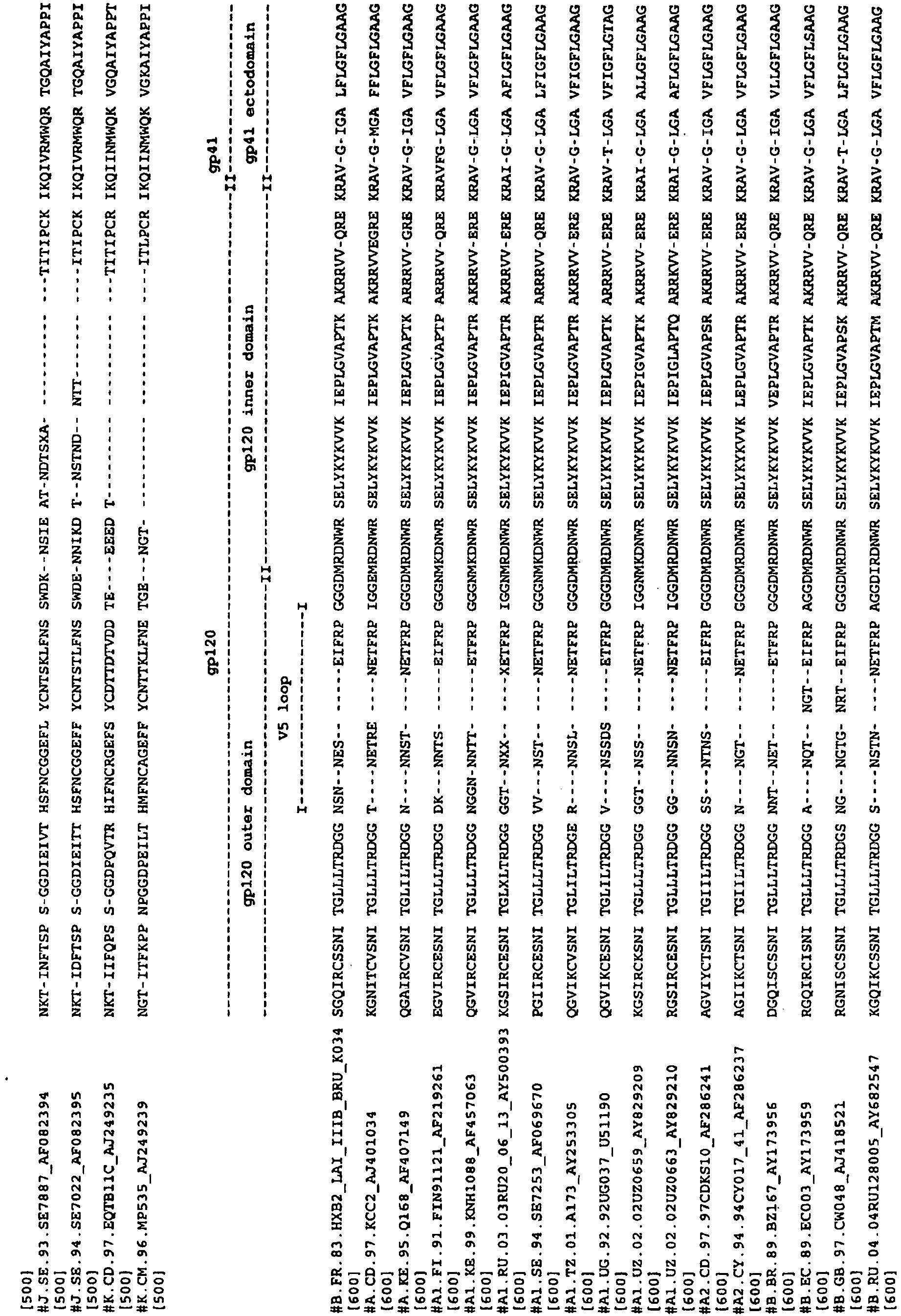

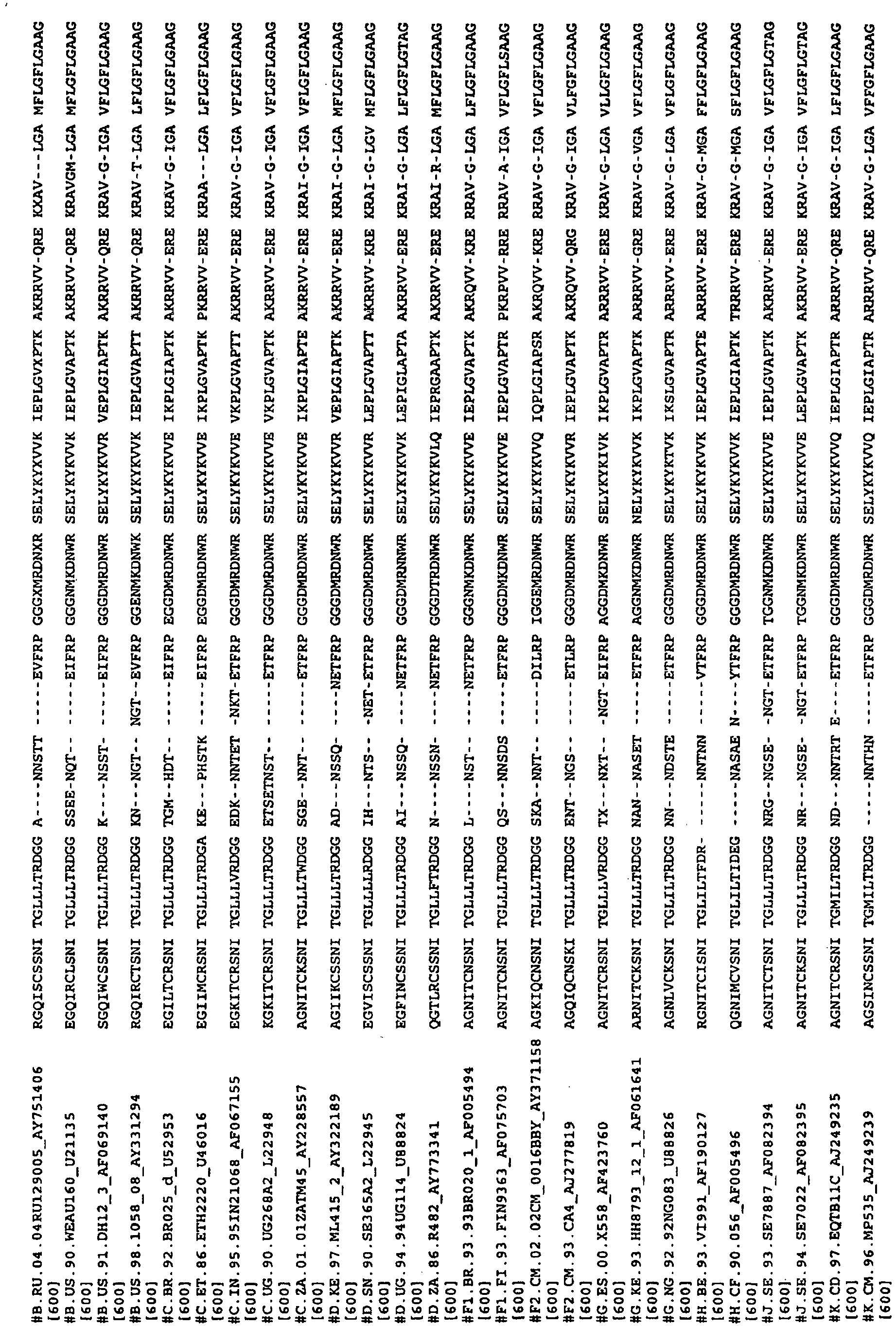

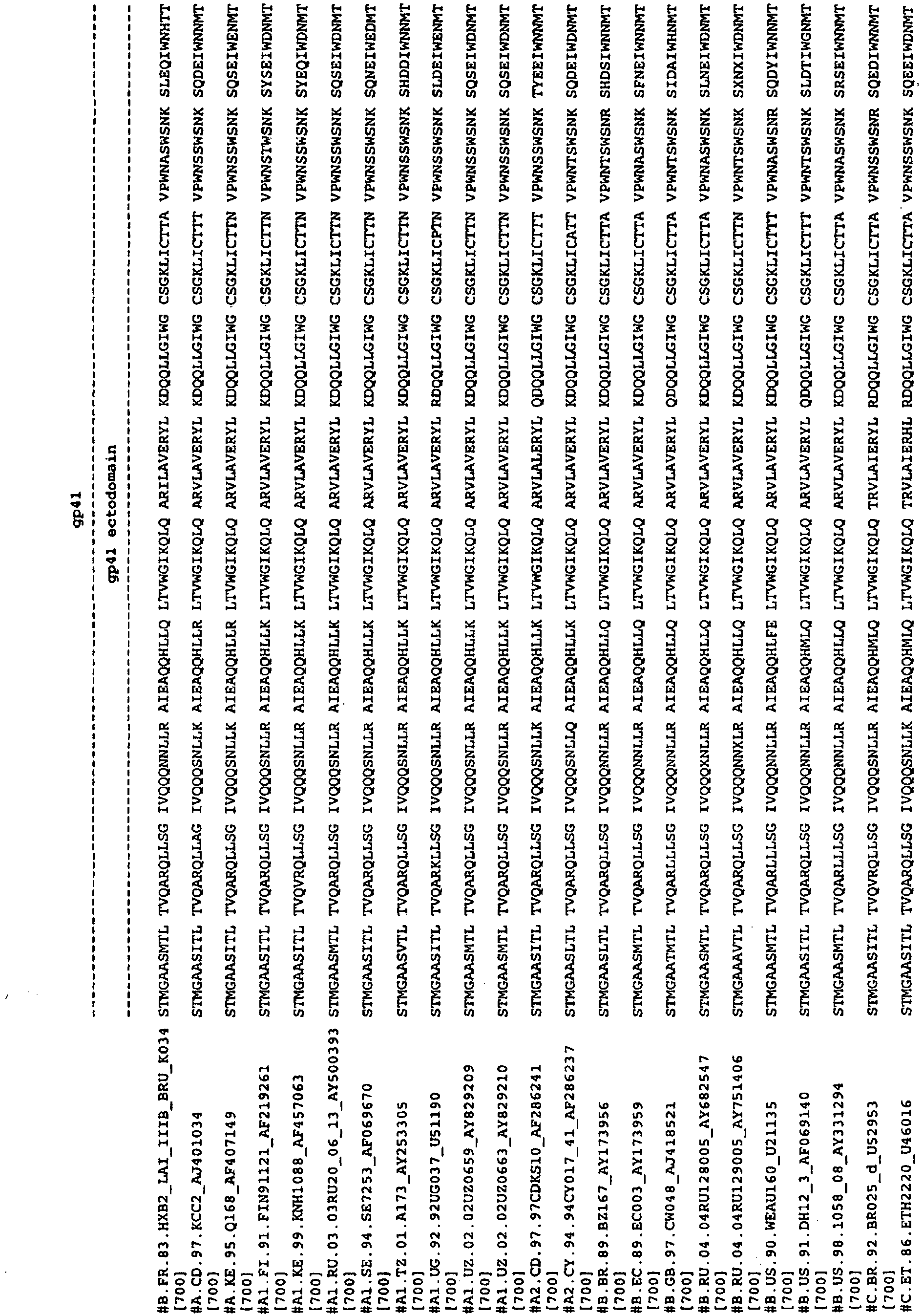

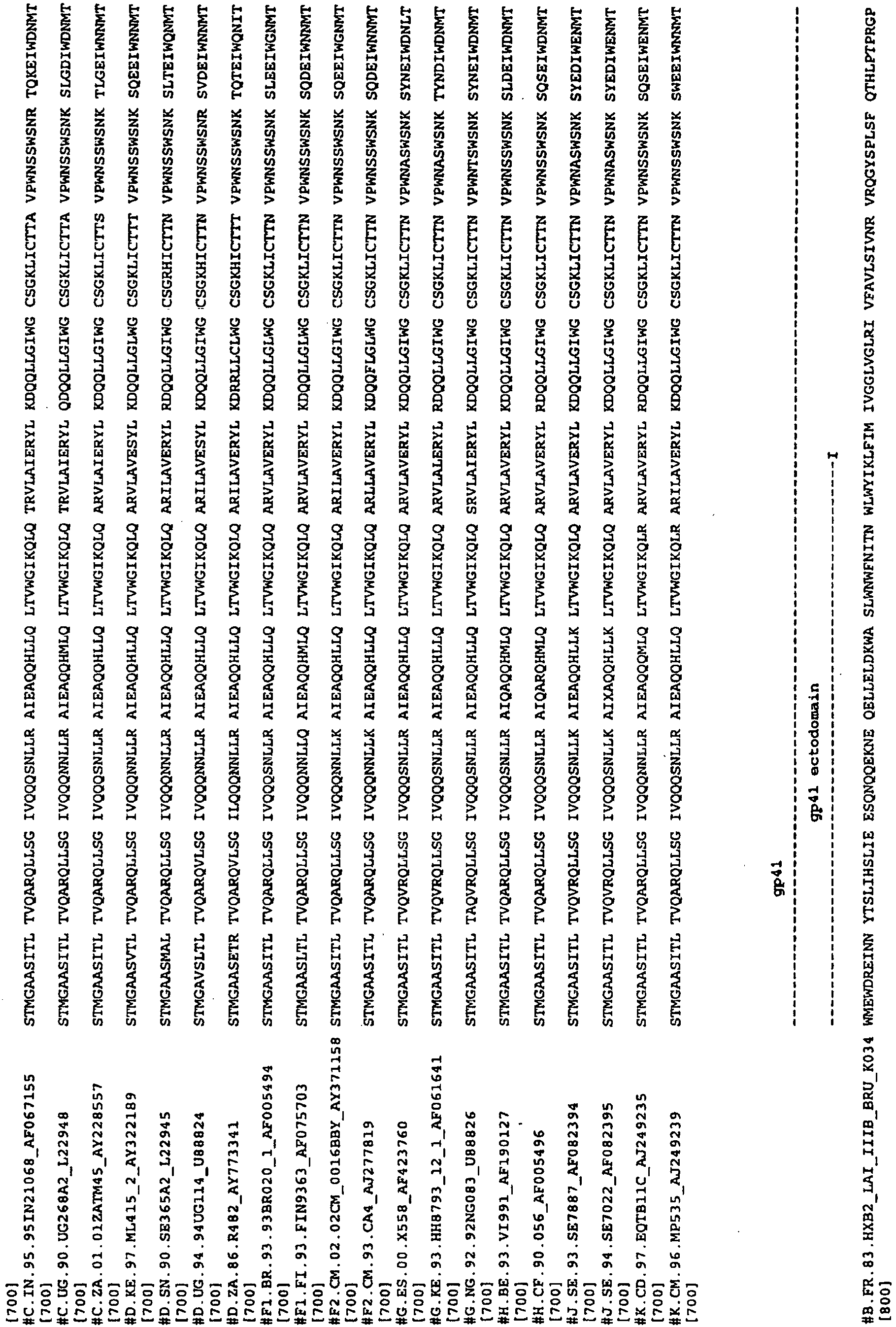

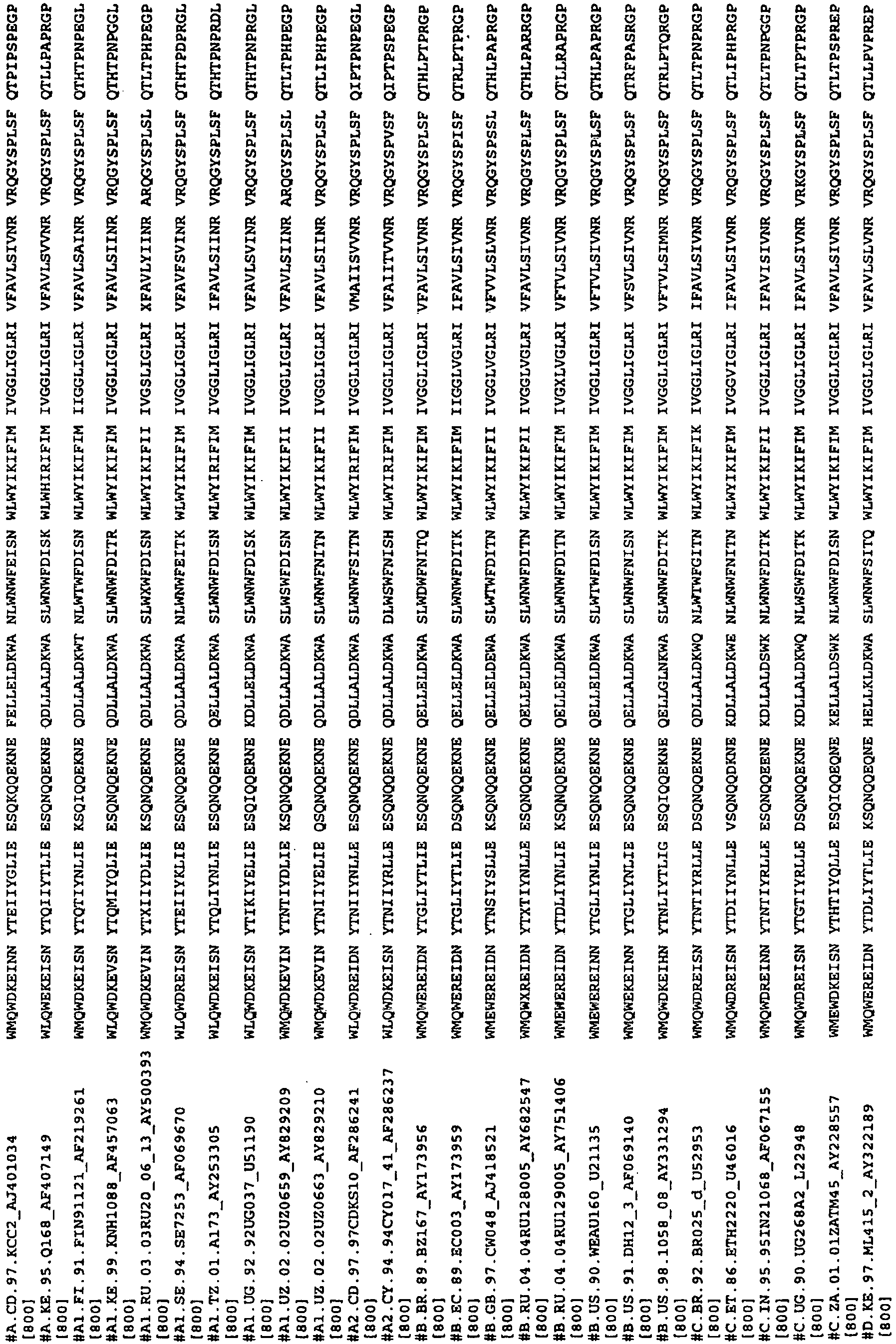

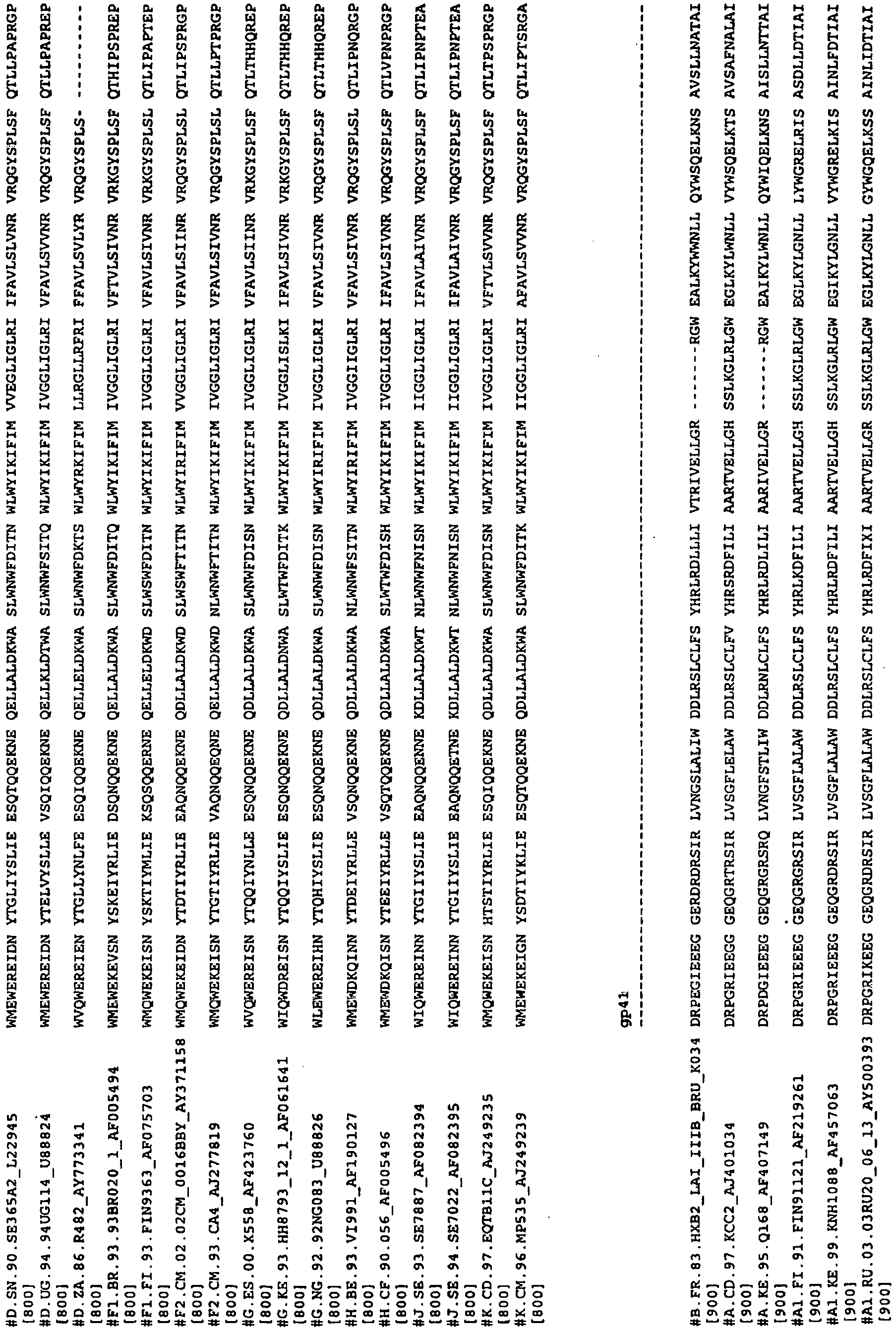

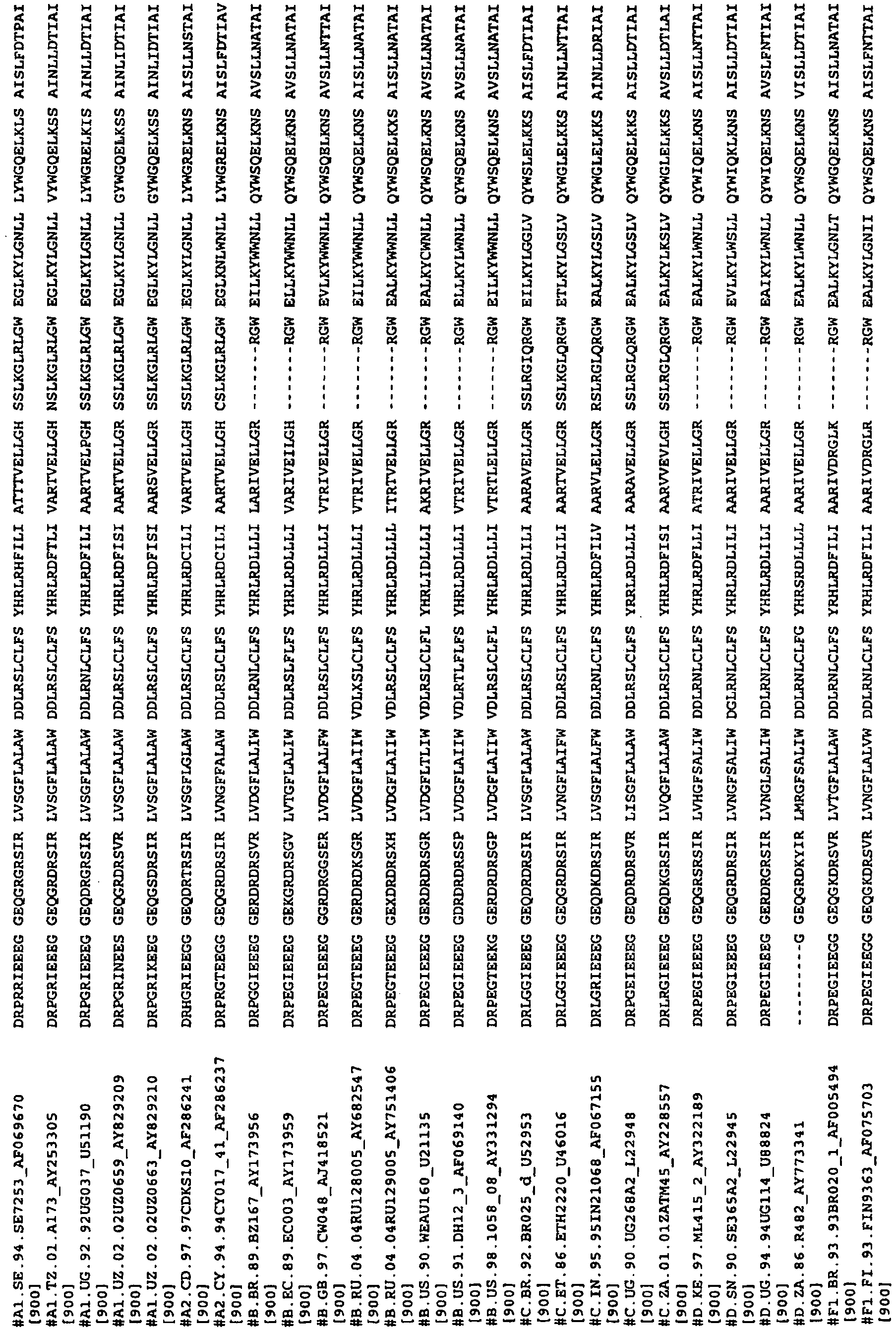

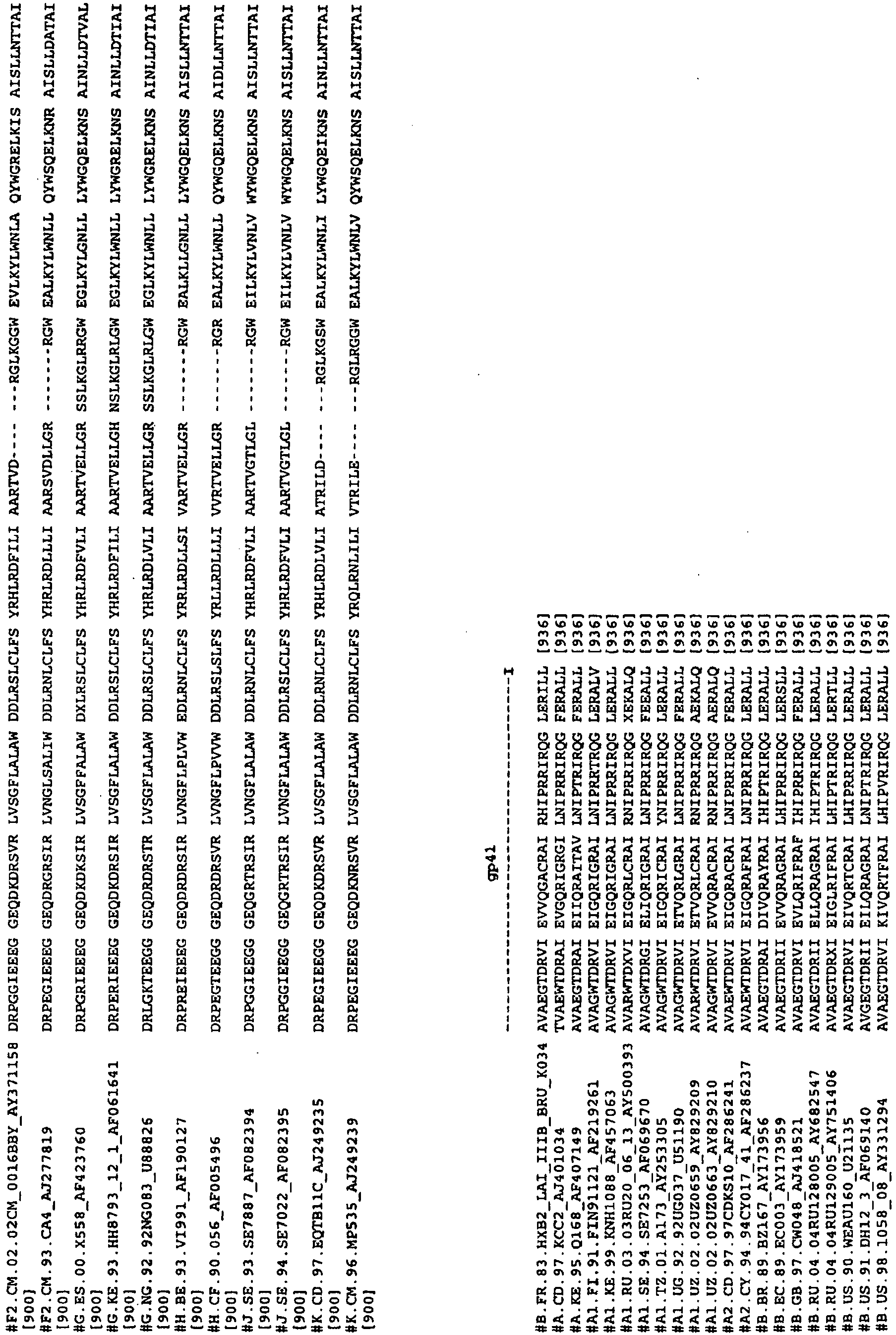

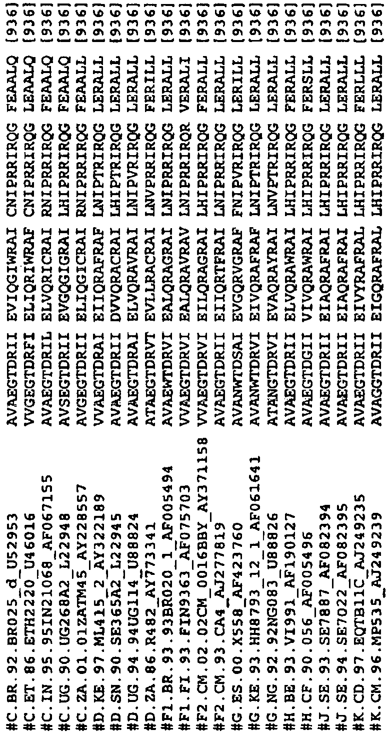

### Example 4

Figure 20 shows the preliminary results of the immunization of animals (SigmaPlot 10.0 statistical analysis).

3-weeks-odd BalbC mice weighting 11-14g are immunized subcutaneously in doses 20-50 µg of pure peptides for the animal, lipids concentration MW is 5 mg/ml. The immunization is carried out at 3 weeks old mice, the second time 2 weeks after when they are 5 weeks old, the third time after 1 month when mice are 9 weeks old. Recombinant gp120 elicited the 5-times higher levels of immune response than recombinant gp4lectodomain in average. The same difference in specific antibodies titr is observed when human polyclonal antibodies isolated from patients blood sera are used for equal concentrations of recombinant gp120 and gp41 ELISA staining.

### REFERENCES:

1. Abram M.E., Parniak M.A. "Virion Instability of Human Immunodeficiency Virus Type 1 Reverse Transcriptase (RT) Mutated in the Protease Cleavage Site between RT p51 and the RT RNase H Domain" Journal of virology, September 2005, v. 79, No 18, 11952-11961;
2. Aguilar M-I. "HPLC of Peptides and Proteins" "Methods in Molecular Biology Seri", v.251, Humana Press, 2004;
3. Amicosante M., Cappelli G. "Method of Antigenic Peptide Identification and Relative Use for the Preparation of a Vaccine Anti HIV-1" Uni Degli Studi di Roma Tor Ve (IT). WO2005075679, 2005-08-18;
4. Barbados C.F. et al. "Phage Display: A Laboratory Manual", 2001, Cold Spring Harbor Laboratory Press;
5. Berman P.W., Fendly B.M. et al. "HIV Env Antibodies" Applicant Genetech INC, US patent US 7041293 B1, 2006-05-09;
6. Berman P.W., Nakamura G.R. "Preparation of an HIV GP 120 Subunit Vaccine Involving Determining a Neutralising Epitope in the V2 and/or C4 Domains", Applicant Genetech INC, US patent NZ267838, 1997-12-19;
7. Bongiovanni M. et al., "Long Term Immunologic Outcome in HAART-Experienced Subjects Receiving Lopinavir/Ritonavir", AIDS Research and Human Retroviruses, 2006, 22(11), pp 1099-1105;
8. Butera S.T. "HIV Chemotherapy. A Critical Review", Caister Academic Press, 2005, U.K;
9. Capodici J. et al., "Large-Scale Beta-Propiolactone Inactivation of HIV for Vaccines" Bioprocess Int., 2006, Feb., pp 36-41;
10. Chertova E, Chertov O, Coren LV, Roser JD, Trubey CM, et al. "Proteomic and biochemical analysis of purified human immunodeficiency virus type 1 produced from infected monocyte-derived macrophages", J Virol., 2006 Sep;80(18):9039-52;
11. Emini, E., Shiver J., Casimiro, D. R. et al. "Therapeutic Immunization of HIV-infected individuals" Appl.: MERCK&CO., INC. A01N 43/04 (2006.01), WO/2005/027835, 31.03.2005;
12.Franchini G., Hel Z., Pavlakis G. et al. "Improved immunogenicity using a combination of DNA and Vaccinia virus vaccines" Appl.: The Government of USA, A61K 39/Z1 (2006.01), WO/2001/082964, 08.11.2001;
13. Haynes B., Desaire F., Heather F. et al. "Carbohydrate-based vaccines for HLV" Appl.: Duke University, US Patent A61K 39/00 (2006.01), WO/2008/033500, 20.03.2008;
14. Herschhorn A. et al., 2003, "Recombinant human antibodies against the reverse transcriptase of human immunodeficiency virus type-1" Biochim Biophys Acta., 1648:154-163;
15. Honda M., Matsuo K. "A Method of Prime-boost Vaccination", Appl.: Japan Science&Tech Agency (JP); JP Nat Inst of Infectious Disease (JP), Patent WO2006057454, 2006.06.01;
16. Humphreys R., Macmillan D., Zinckgraf J. "Ii-key enhanced vaccine potency" US Patent A61K 48/00, Applicant: Antigen Express, Inc. ,WO 2008/060385, 22.05.2008;
17. Ghania G. et al., "Attaching histidine-tagged peptides and proteins to lipid-based carriers through use of metal-ion-chelating lipids", Biochimica et Biophysica Acta 1567 (2002) 204- 212;
18. Grundner,C. et al., "Factors limiting the immunogenicity of HIV-1 gp120 envelope glycoproteins" J. Virology, 2004.Dec.5.;330.(1.):233.-48;
19. Karn J. "HIV Volume 1: Virology and Immunology" Oxford University Press, 1995, reprinted 2001, 63-75;
20. Karp N.M. "Immunogenic Composition and Method of Developing a Vaccine Based on Psoralen Inactivated HIV" Appl.: NMK RES LLC (US), US Patent WO2005040353, 2005-05-06;
21. Kirschner M., Monrose V., Paluch M., Techodamrongsin N., Rethwilm A., Moore J. " The production of cleaved, trimeric human immunodeficiency virus type 1 (HIV-1) envelop glycoprotein vaccine antigens and infectious pseudoviruses using linear polyethylenimine as a transfection reagent" Prot. Expres. Purification, Jul. 2006, 48(1), 61-68;-
22. Kwong P.D. et al. "Structure of an HIV-1 gp120 envelope glycoprotein in complex with the CD4 receptor and a neutralizing human antibody", Nature, (1998) 393:649-59;
23. Maniatis et al., "Molecular Cloning, a Laboratory Manual", 1991,Cold Spring Harbor Laboratory Press;
24. Leitner T. "HIV Sequence Compendium 2006-2007", Los Alamos National Laboratory annual issue;
25. Liu B., Marks J.D. "Applying phage antibodies to proteomics: selecting single chain Fv antibodies blotted on nitrocellulose" Analitical Biochem. 2000, 286, 119-128;
26. Lu X., Dropulic B. "Lentivirus vector-based approaches for generating an immune response to HIV humans" US Patent, Appl.: VIRXSYS Corporation, WO/2005/023313, 17.03.2005;
27. Martin L., Stricher F., Descours A. et al. "CD4 mimic peptides and their usues" Appl.: Commisariat A L'Energie Atomique, C07K 14/705, WO/2007/144685, 21.12.2007;
28. Marks J. D. et al. "By-passing Immunization Human Antibodies from V-gene Libraries Displayed on Phage" 1991, J. Mol. Biol., 222: 581-597;
29. Peluso R., Arnold S., Wustner J. Et al. "Recombinant AAV-based vaccine methods" US Patent C12N 15/864, Applicant: Targeted Genetics Corporation, WO 2006/073496, 13.07.2006;
30. Picker, L. J., Jarvis, M., Nelson, J, A. "SIV and HIV vaccination using RHCMV- and HCMV-based vaccine vectors" US Patent C12N 15/63 (2006.01), Appl.: Oregon Health and Science University, WO/2006/031264, 23.03.2006;
31. Poignard P. et. al., "Heterogeneity of envelope molecules expressed on primary human immunodeficiency virus type 1 particles as probed by the binding of neutralizing and non-neutralizing antibodies" J. Virol.2003.Jan.;77.(1.): 353-65;
32. http://iavireport.org/specials/OngoingTrialsofPreventiveHIVVaccines.pdf, NIAID's report 2007 " Ongoing Trials of Preventive AIDS Vaccines" ;
33. Sala M. Greco, R. et al. "Polynucleotides allowing the expression and secretion of recombinant HbSag virus-like particles containing a foreign peptide, their production and use" Appl.: Institute Pasteur, WO Patent 2008/020331, C07K 14/43, 21.02.2008;
34. Sanders R.W. et al., "Mutational Analyses and Natural Variability of the gp4143. Ectodomain", The 2002 HIV Sequence Compendium;
35. Sidhu S.S. "Phage Display in Biotechnology and Drug Discovery", 2005, Taylor & Francis group;
36. Soerensen B. "HIV peptides, antigens, vaccine compositions, immunoassay kit and a method of detesting antibodies induced by HIV", Appl.: BIONOR AS (NO), Patent WO0052040, 2000.09.08;
37. Steinbrook R. "One Step Forward, Two Steps Back - Will There Ever Be an AIDS Vaccine?" New England Journal of Medicine, Dec. 27, 2007(26), 357:2653-2655;
38. Torchilin VP, Levchenko TS, Lukyanov AN, Khaw BA, Klibanov AL, Rammohan R, Samokhin GP, Whiteman KR. "p-Nitrophenylcarbonyl-PEG-PE-liposomes: fast and simple attachment of specific ligands, including monoclonal antibodies, to distal ends of PEG chains via p-nitrophenylcarbonyl groups", Biochim Biophys Acta. 2001 Apr 2;1511(2):397-411;
39. Torchilin V.P. "Liposomes: A Practical Approach" Edition: 2, Publisher: Oxford Univ. Press, publ. 08/01/2003;
40. Wyatt R. et al., Sodroski J. The antigenic structure of the human immunodeficiency virus gp120 envelope glycoprotein. Nature, (1998) 393:705-11;
41. Wyatt R. et al., Sodroski J.G. "Structure of the Core of the HIV-1 gp120 Exterior Envelope Glycoprotein" The Human Retroviruses and AIDS Compendium, 1998;
42. http://www.hiv.1anl.gov/cgi-bin/vaccine/public/ Los Alamos HIV vaccine trials database.

## Claims

1. A method for producing an HIV immunogenic composition, comprising the steps of:
a) creation of a human recombinant IgG phagemid library containing HIV-1 specific scFv antibody fragments created from RNA isolated from B-lymphocytes of infected individuals,
b) enrichment for HIV-specific scFv antibody fragments in the phagemid library by panning with native or recombinant HIV-1 peptides,
c) multiplying HIV-1 material comprising HIV-1 peptides, polypeptides or proteins by virus cultivation in PBMC,
d) collecting HIV-1 peptides by reverse panning of the multiplied HIV-1 material using the enriched HIV-1 phagemid library of step b) bound to a support.
e) identification and characterization of the HIV-peptides obtained in step d),
f) expressing glycosylated env HIV-1 peptides using the results of step e) in an expression system, wherein the expression system is a suitable host with eukaryotic glycosylation, such as a *Leishmania tarentolae* expression system,
g) purification of the glycosylated env HIV-1 peptides, and
h) production of an immunogenic composition.

2. The method according to claim 1, wherein the individuals from which the HIV material is obtained are infected by the same or a different HIV subtype.

3. The method according to any of the preceding claims, wherein the individuals from which the viral material is obtained are antiretroviral therapy naïve patients or patients that have been subjected to antiretroviral therapy.

4. The method according to claim 1, wherein the phagemid library is prepared by the steps of
a) preparing DNA-fragments derived from nucleic acids encoding the variable region of a light chain and a heavy chain, respectively, of immunoglobulins expressed in B-Lymphocytes obtained from a number of individuals infected with HIV,
b) linking the DNA-fragments encoding the immunoglobulin light and heavy chain, to allow expression of a polypeptide, comprising the variable regions of a light chain and heavy chain, respectively, of immunoglobulins, to create a multitude of different specificities,
c) cloning of linked fragments in phagemid vector and transforming bacterial strain for expression on the bacteriophage's surface,
- preferably wherein amplification is carried out with any of the primer combination listed in tables 1-7,
- more preferably wherein the obtained scFv phagemid recombinant antibodies are specific to resistant HIV variants in patients subjected to HAART- or any other antiretroviral therapy.

5. The method according to claim 4, wherein step a) further comprises an enrichment of the phagemid library presenting the antibody scFv fragments in a panning procedure by binding the HIV-specific antibodies with recombinant gp120-, gp41- and native HIV-polypeptides isolated from different donors.

6. The method according to claim 1, wherein
in step e) LC mass spectrometry is applied for a quantitative analysis, identification and sequencing of HIV-1Gp120 and its standard and variable fragments.

7. The method according to any of the preceding claims, which involves preparing an HIV immunogenic composition by addition/conjugation of optional immunogenic stimulants, adjuvants or carriers, such as sterically stabilized liposomes (SSL).

## Patentansprüche

1. Verfahren zum Herstellen einer HIV-immunogenen Zusammensetzung, umfassend die Schritte:
a) Erstellung einer humanen rekombinanten IgG-Phagemid-Bibliothek, enthaltend HIV-1-spezifische scFv-Antikörperfragmente, die aus RNA erstellt wurden, die von B-Lymphozyten infizierter Individuen isoliert wurde,
b) Anreicherung auf HIV-spezifische scFv-Antikörperfragmente in der Phagemid-Bibliothek durch Panning mit nativen oder rekombinanten HIV-1-Peptiden,
c) Vermehren des HIV-1-Materials umfassend HIV-1-Peptide, Polypeptide oder Proteine durch Viruskultivierung in PBMC,
d) Aufnehmen der HIV-1-Peptide durch umgekehrtes (reverse) Panning des vermehrten HIV-1-Materials unter Verwendung der angereicherten HIV-1-Phagemid-Bibliothek aus Schritt b), die an einen Träger gebunden ist,
e) Identifizierung und Charakterisierung der HIV-Peptide, die in Schritt d) erhalten wurden,
f) Exprimieren glykosylierter env HIV-1-Peptide unter Verwendung der Ergebnisse aus Schritt e) in einem Expressionssystem, wobei das Expressionssystem ein geeigneter Wirt mit eukaryotischer Glykosylierung ist, wie ein *Leishmania tarentolae* Expressionssystem,
g) Aufreinigung der glykosylierten env HIV-1-Peptide und
h) Herstellung einer immunogenen Zusammensetzung.

2. Verfahren nach Anspruch 1, wobei die Individuen, von denen das HIV-Material erhalten wird, mit demselben oder einem verschiedenen HIV-Subtyp infiziert sind.

3. Verfahren nach einem der vorhergehenden Ansprüche, wobei die Individuen, von denen das virale Material erhalten wird, Patienten sind, die naiv bezüglich antiretroviraler Therapie sind oder Patienten sind, die sich einer antiretroviralen Therapie unterzogen haben.

4. Verfahren nach Anspruch 1, wobei die Phagemid-Bibliothek erstellt wird durch die Schritte:
a) Erstellen von DNA-Fragmenten, die von Nukleinsäuren abgeleitet sind, die für die variable Region einer leichten Kette beziehungsweise einer schweren Kette von Immunglobulinen kodieren, die in B-Lymphozyten exprimiert werden, die aus einer Anzahl von Individuen erhalten wurden, die mit HIV infiziert sind,
b) Verbinden der DNA-Fragmente, die für die leichte und schwere Immunglobulin-Kette kodieren, um die Expression eines Polypeptids zu erlauben, umfassend die variablen Regionen einer leichten Kette beziehungsweise einer schweren Kette der Immunglobuline, um eine Vielzahl an unterschiedlichen Spezifitäten zu erzeugen,
c) Klonieren von verbundenen Fragmenten in einem Phagemid-Vektor und Transformieren eines bakteriellen Stamms zur Expression auf einer Bakteriophagenoberfläche,
- bevorzugt wobei die Amplifikation mit einer beliebigen derjenigen Primerkombinationen ausgeführt wird, die in den Tabellen 1-7 aufgeführt sind,
- stärker bevorzugt wobei die erhaltenen rekombinanten scFv Phagemid Antikörper spezifisch sind für resistente HIV-Varianten in Patienten, die sich einer HAART- oder irgendeiner anderen antiretroviralen Therapie unterzogen haben/sich unterziehen.

5. Verfahren nach Anspruch 4, wobei Schritt a) weiter umfasst eine Anreicherung der Phagemid-Bibliothek, welche die scFv-AntikörperFragmente präsentiert, in einem Panning-Verfahren durch Binden der HIVspezifischen Antikörper an rekombinante gp120-, gp41- und native HIV-Polypeptide, die von verschiedenen Spendern isoliert wurden.

6. Verfahren nach Anspruch 1, wobei
in Schritt e) LC-Massenspektrometrie für eine quantitative Analyse, Identifikation und Sequenzierung von HIV-1- gp120 und seiner Standard- und variablen Fragmente angewendet wird.

7. Verfahren nach einem beliebigen der vorhergehenden Ansprüche, umfassend das Herstellen einer HIV-immunogenen Zusammensetzung durch Addition/Konjugation optionaler immunogener Stimulanzien, Adjuvanzien oder Träger, wie sterisch stabilisierter Liposomen (SSL).

## Revendications

1. Procédé de production d'une composition immunogène du VIH, comprenant les étapes de :
a) création d'une banque de phagemides d'IgG recombinantes humaines contenant des fragments scFv d'anticorps spécifiques du VIH-1 créés à partir d'un ARN isolé des lymphocytes B d'individus infectés,
b) enrichissement des fragments d'anticorps scFv spécifiques du VIH dans la banque de phagemides par adhérence sur des peptides natifs ou recombinants de VIH-1,
c) multiplication du matériel de VIH-1 comprenant des peptides, polypeptides ou protéines du VIH-1 par la culture de virus dans des PBMC,
d) collecte des peptides du VIH-1 par adhérence inverse du matériel de VIH-1 multiplié en utilisant la banque de phagemides de VIH-1 enrichie de l'étape b) liée à un support,
e) identification et caractérisation des peptides de VIH obtenus dans l'étape d),
f) expression des peptides env glycosylés de VIH-1 en utilisant les résultats de l'étape e) dans un système d'expression, dans lequel le système d'expression est un hôte approprié à la glycosylation eucaryote, tel qu'un système d'expression de *Leishmania tarentolae,*
g) purification des peptides env glycosylés de VIH-1, et
h) production d'une composition immunogène.

2. Procédé selon la revendication **1**, dans lequel les individus à partir desquels le matériel de VIH est obtenu sont infectés par un sous-type de VIH identique ou différent.

3. Procédé selon l'une quelconque des revendications précédentes, dans lequel les individus à partir desquels le matériel viral est obtenu sont des patients naïfs à une thérapie antirétrovirale ou des patients qui ont été soumis à une thérapie antirétrovirale.

4. Procédé selon la revendication 1, dans lequel la banque de phagemides est préparée par les étapes de :
a) préparation de fragments d'ADN dérivés d'acides nucléiques codant la région variable d'une chaîne légère et d'une chaîne lourde, respectivement, d'immunoglobulines exprimées par des lymphocytes B obtenus auprès d'un nombre d'individus infectés par le VIH,
b) liaison des fragments d'ADN codant la chaîne légère et la chaîne lourde d'immunoglobuline, pour permettre l'expression d'un polypeptide, comprenant les régions variables d'une chaîne légère et d'une chaîne lourde, respectivement, d'immunoglobulines, pour créer une multitude de spécificités différentes,
c) clonage des fragments liés dans un vecteur phagemide et transformation d'une souche bactérienne pour une expression à la surface de bactériophages,
- de préférence, dans lequel l'amplification est réalisée avec l'une quelconque des combinaisons d'amorces listées dans les tableaux 1 à 7,
- de manière davantage préférée, dans lequel les anticorps recombinants de phagemides scFv sont spécifiques de variants résistants du VIH chez des patients soumis à un TAHA ou à n'importe quelle autre thérapie antirétrovirale.

5. Procédé selon la revendication **4**, dans lequel l'étape a) comprend en outre un enrichissement de la banque de phagemides présentant les fragments scFv d'anticorps dans une procédure d'adhérence par la liaison des anticorps spécifiques du VIH à des polypeptides recombinants gp120, gp41 et natifs du VIH isolés à partir de donneurs différents.

6. Procédé selon la revendication **1**, dans lequel
dans l'étape e), une spectrométrie de masse LC est appliquée pour une analyse quantitative, une identification et un séquençage de Gp120 de VIH-1 et de ses fragments standards et variables.

7. Procédé selon l'une quelconque des revendications précédentes, qui implique la préparation d'une composition immunogène du VIH par l'ajout/la conjugaison de stimulants immunogènes, d'adjuvant ou de supports facultatifs, tels que des liposomes stériquement stabilisés (SSL).
